# EUROPEAN PATENT APPLICATION

(11) **EP 2 375 252 A1**
(43) Date of publication of application: **12.10.2011**
(21) Application number: 11158716.8
(22) Date of filing: 11.06.2009
(51) Int. Cl.: G01N 33/564

(54) **Biomarkers for lupus**

(30) Priority: 11.06.2008 GB 0810709
(62) Divisional of application: 09761971.2
(71) Applicant: SENSE PROTEOMIC LIMITED, Yarnton Oxford Oxfordshire OX5 1PF (GB)
(72) Inventor: Ebner, Martin Johannes, Maidenhead, Berkshire SL6 7RJ (GB); Wheeler, Colin, Henry, Maiadenhead, Berkshire SL6 7RJ (GB); Fallon, Rachel, Alison, Maidenhead, Berkshire SL6 7RJ (GB); Joyce, Sarah, Paula, Maidenhead, Berkshire SL6 7RJ (GB); Koopman, Jens-Oliver, Maidenhead, Berkshire SL6 7RJ (GB); McAndrew, Michael, Bernard, Maidenhead, Berkshire SL6 7RJ (GB); Workman,, Nicholas, Ian, Maidenhead, Berkshire SL6 7RJ (GB)
(74) Representative: Marshall, Cameron John

(57) **Abstract**

The present invention provides biomarkers valuable in diagnosing or detecting a susceptibility to lupus. The present invention also provides panels of biomarkers valuable in diagnosing or detecting a susceptibility to lupus with good specificity and selectivity. Said biomarkers and panels of biomarkers are also useful in monitoring the progression or regression of lupus, or monitoring the progression to a flare of the disease or transition from a flare into remission or monitoring the efficacy of a therapeutic agent to lupus.

SEQUENCE LISTING

<110> SENSE PROTEOMIC LTD

<120> BIOMARKERS FOR LUPUS

<130> 27353-518001WO

<140>
<141>

<150> GB 0810709.6
<151> 2008-06-11

<160> 45

<170> PatentIn version 3.5

<210> 1
<211> 1662
<212> DNA
<213> Homo sapiens

<400> 1
atggcggccc cggcaggcgg cggaggctcc gcggtgtcgg tgctggcccc gaacggccgg 60

cgccacacgg tgaaggtgac gccgagcacc gtgctgcttc aggttctgga ggacacgtgc 120

cggcggcagg acttcaaccc ctgtgaatat gatctgaagt ttcagaggag cgtgctcgac 180

ctttctctcc agtggagatt tgccaacctg cccaacaatg ccaagctgga gatggtgccc 240

gcttcccgga gccgtgaggg gcctgagaac atggttcgca tcgctttgca gctggacgat 300

ggctcgaggt tgcaggactc tttctgttca ggccagaccc tctgggagct tctcagccat 360

tttccacaga tcagggagtg cctgcagcac cccggcgggg ccaccccagt ctgcgtgtac 420

acgagggatg aggtgacggg tgaagctgcc ctgcggggca cgacgctgca gtcgctgggc 480

ctgaccgggg gcagcgccac catcaggttt gtcatgaagt gctacgaccc cgtgggcaag 540

accccaggaa gcctgggctc gtcagcgtcg gctggccagg cagccgccag cgctccactt 600

cccttggaat ctggggagct cagccgcggc gacttgagcc gtccggagga cgcggacacc 660

tcagggccct gctgcgagca cactcaggag aagcagagca caagggcacc cgcagctgcc 720

ccctttgttc ctttctcggg tgggggacag agacaggggg gccctcctgg gcccacgagg 780

cctctgacat catcttcagc taagttgccg aagtccctct ccagccctgg aggcccctcc 840

aagccaaaga agtccaagtc gggccaggat ccccagcagg agcaggagca ggagcgggag 900

cgggatcccc agcaggagca ggagcgggag cggcccgtgg accgggagcc cgtggaccgg 960

gagccggtgg tgtgccaccc cgacctggag gagcggctgc aggcctggcc agcggagctg 1020

cctgatgagt tctttgagct gacggtggac gacgtgagaa gacgcttggc ccagctcaag 1080

agtgagcgga agcgcctgga agaagccccc ttggtgacca aggccttcag ggaggcgcag 1140

ataaaggaga agctggagcg ctacccaaag gtggctctga gggtcctgtt ccccgaccgc 1200

tacgtcctac agggcttctt ccgccccagc gagacagtgg gggacttgcg agacttcgtg 1260

aggagccacc tggggaaccc cgagctgtca ttttacctgt tcatcacccc tccaaaaaca 1320

gtcctggacg accacacgca gaccctcttt caggcgaacc tcttcccggc cgctctggtg 1380

cacttgggag ccgaggagcc ggcaggtgtc tacctggagc ctggcctgct ggagcatgcc 1440

atctccccat ctgcggccga cgtgctggtg gccaggtaca tgtccagggc cgccgggtcc 1500

ccttccccat tgccagcccc tgaccctgca cctaagtctg agccagctgc tgaggagggg 1560

gcgctggtcc cccctgagcc catcccaggg acggcccagc ccgtgaagag gagcctgggc 1620

aaggtgccca agtggctgaa gctgccggcc agcaagaggt ga 1662

<210> 2
<211> 2358
<212> DNA
<213> Homo sapiens

<400> 2
atgctgccag cagcgccagg caaggggctt gggagcccgg accccgcccc ctgcggccca 60

gcgcccccag gaaatacaaa agatataata atgatatatg aagaagatgc tgaggaatgg 120

gctctgtact tgacagaagt atttttacat gttgtgaaaa gggaagccat cctgttatat 180

cgcttggaga atttctcttt tcggcatttg gagttgctga acttaacgtc ttacaaatgt 240

aaacttttga tattatcaaa tagcctgctt agagacctaa ctccaaagaa atgtcagttt 300

ctggaaaaga tacttcattc accaaaaagt gtagttactt tgctttgtgg agtgaagagt 360

tcagatcagc tctatgaatt actaaatatc tctcaaagca gatgggagat ctcaactgaa 420

caggaacctg aagactacat ctctgtaatc cagagtatca tattcaaaga ttctgaagac 480

tactttgagg tcaacattcc aacagaccta cgagcaaaac attctgggga aataagtgag 540

agaaaggaaa ttgaagaact atcagaagct tcaagaaaca ccataccact agcagtggtg 600

cttcccactg aaattccatg tgagaatcct ggtgaaatat tcataatttt gagagatgaa 660

gtaattggtg atactgtaga ggttgaattt acatcaagta ataagcgcat tagaacacgg 720

ccagcccttt ggaataagaa agtctggtgc atgaaagctt tagagtttcc tgctggttca 780

gtccatgtca atgtctactg tgatggaatc gttaaagcta caaccaaaat taagtactac 840

ccaacagcaa aggcaaagga atgcctattc agaatggcag attcaggaga gagtttgtgc 900

cagaatagca ttgaagaact tgatggtgtc cttacatcca tattcaaaca tgagatacca 960

tattatgagt tccagtctct tcaaactgaa atttgttctc aaaacaaata tactcatttc 1020

aaagaacttc caactcttct ccactgtgca gcaaaatttg gcttaaagaa cctggctatt 1080

catttgcttc aatgttcagg agcaacctgg gcatctaaga tgaaaaatat ggagggttca 1140

gaccccgcac atattgctga aaggcatggt cacaaagaac tcaagaaaat cttcgaagac 1200

ttttcaatcc aagaaattga cataaataat gagcaagaaa atgattatga agaggatatt 1260

gcctcatttt ccacatatat tccttccaca cagaacccag catttcatca tgaaagcaga 1320

aagacatacg ggcagagtgc agatggagct gaggcaaatg aaatggaagg ggaaggaaaa 1380

cagaatggat caggcatgga gaccaaacac agcccactag aggttggcag tgagagttct 1440

gaagaccagt atgatgactt gtatgtgttc attcctggtg ctgatccaga aaataattca 1500

caagagccac tcatgagcag cagacctcct ctccccccgc cgcgacctgt agctaatgcc 1560

ttccaactgg aaagacctca cttcacctta ccagggacaa tggtggaagg ccaaatggaa 1620

agaagtcaaa actggggtca tcctggtgtt agacaagaaa caggagatga acccaaagga 1680

gaaaaagaga agaaagaaga ggaaaaagag caggaggagg aagaagaccc atatactttt 1740

gctgagattg atgacagtga atatgacatg atattggcca atctgagtat aaagaaaaaa 1800

actgggagtc ggtctttcat tataaataga cctcctgccc ccacaccccg acccacaagt 1860

atacctccaa aagaggaaac tacaccttac atagctcaag tgtttcaaca aaagacagcc 1920

agaagacaat ctgatgatga caagttccgt ggtcttccta agaaacaaga cagagctcgg 1980

atagagagtc cagccttttc tactctcagg ggctgtctaa ctgatggtca ggaagaactc 2040

atcctcctgc aggagaaagt aaagaatggg aaaatgtcta tggatgaagc tctggagaaa 2100

tttaaacact ggcagatggg aaaaagtggc ctggaaatga ttcagcagga gaaattacga 2160

caactacgag actgcattat tgggaaaagg ccagaagaag aaaatgtcta taataaactc 2220

accattgtgc accatccagg tggtaaggaa actgcccaca atgaaaataa gttttataat 2280

gtacacttca gcaataagct tcctgctcga ccccaagttg aaaaggaatt tggtttctgt 2340

tgcaagaaag atcattaa 2358

<210> 3
<211> 453
<212> DNA
<213> Homo sapiens

<400> 3
atgagcaaga tatcgcagca aaacagcact ccaggggtga acggaattag tgttatccat 60

acccaggcac atgccagcgg cttacagcag gttcctcagc tggtgcctgc tggccctggg 120

ggaggaggca aagctgtggc tcccagcaag cagagcaaaa agagttcgcc catggatcga 180

aacagtgacg agtatcggca acgccgagag aggaacaaca tggctgtgaa aaagagccgg 240

ttgaaaagca agcagaaagc acaagacaca ctgcagagag tcaatcagct caaagaagag 300

aatgaacggt tggaagcaaa aatcaaattg ctgaccaagg aattaagtgt actcaaagat 360

ttgtttcttg agcatgcaca caaccttgca gacaacgtac agtccattag cactgaaaat 420

acgacagcag atggcgacaa tgcaggacag tag 453

<210> 4
<211> 1803
<212> DNA
<213> Homo sapiens

<400> 4
atggagcatg tgacagaggg ctcctgggag tcgctgcctg tgccgctgca cccgcaggtg 60

ctgggcgcgc tgcgggagct gggcttcccg tacatgacgc cggtgcagtc cgcaaccatc 120

cctctgttca tgcgaaacaa agatgtcgct gcagaagcgg tcacaggtag tggcaaaaca 180

ctcgcttttg tcatccccat cctggaaatt cttctgagaa gagaagagaa gttaaaaaag 240

agtcaggttg gagccataat catcaccccc actcgagagc tggccattca aatagacgag 300

gtcctgtcgc atttcacgaa gcacttcccc gagttcagcc agattctttg gatcggaggc 360

aggaatcctg gagaagatgt tgagaggttt aagcaacaag gtgggaacat cattgtggcc 420

actccaggcc gcttggagga cttgttccgg aggaaggccg aaggcttgga tctggccagc 480

tgtgtgcgat ccctggatgt cctggtgttg gatgaggcag acagacttct ggacatgggg 540

tttgaggcaa gcatcaacac cattctggag tttttgccaa agcagaggag aacaggcctt 600

ttctctgcca ctcagacgca ggaagtggag aacctggtga gagcgggcct ccggaaccct 660

gtccgggtct cagtgaagga gaagggcgtg gcagccagca gtgcccagaa gaccccctcc 720

cgcctggaaa actactacat ggtatgcaag gcagatgaga aatttaatca gctggtccat 780

tttcttcgca atcataagca ggagaaacac ctggtcttct tcagcacctg tgcctgtgtg 840

gaatactatg ggaagactct ggaagtgctg gtgaagggcg tgaagattat gtgcattcac 900

ggaaagatga aatataaacg caataagatc ttcatggagt tccgcaaatt gcaaagtggg 960

attttagtgt gcactgatgt gatggcccgg ggaattgata ttcctgaagt caactgggtt 1020

ttgcagtatg accctcccag caatgcaagt gccttcgtgc atcgctgcgg tcgcacagct 1080

cgcattggcc acgggggcag cgctctggtg ttcctcctgc ccatggaaga gtcatacatc 1140

aatttccttg caattaacca aaaatgcccc ctgcaggaga tgaagcccca gagaaacaca 1200

gcggaccttc tgccaaaact caagtccatg gccctggctg acagagctgt gtttgaaaag 1260

ggcatgaaag cttttgtgtc atatgtccaa gcttatgcaa agcatgaatg caacctgatt 1320

ttcagattaa aggatcttga ttttgccagc cttgctcgag gttttgccct gctgaggatg 1380

cccaagatgc cagaattgag agggaagcag tttccagatt ttgtgcccgt ggacgttaat 1440

accgacacga ttccatttaa agataaaatc agagaaaagc agaggcagaa actcctggag 1500

caacaaagaa gagagaaaac agaaaatgaa gggagaagaa aattcataaa aaataaagct 1560

tggtcaaagc agaaggccaa aaaagaaaag aagaaaaaaa tgaatgagaa aaggaaaagg 1620

gaagagggtt ctgatattga agatgaggac atggaagaac ttcttaatga cacaagactc 1680

ttgaaaaaac ttaagaaagg caaaataact gaagaagaat ttgagaaggg cttgttgaca 1740

actggcaaaa gaacaatcaa gacagtggat ttagggatct cagatttgga agatggctgc 1800

taa 1803

<210> 5
<211> 1191
<212> DNA
<213> Homo sapiens

<400> 5
atggatccca gggggaccaa gagaggagct gagaagacag aggtagctga gcctcggaac 60

aaactacctc gtccagcacc ttctctgccc acagaccctg ccctctactc tgggcccttt 120

cctttctacc ggcgcccttc ggaactgggc tgcttctccc tggatgctca acgccagtac 180

catggagatg cccgagccct gcgctactat agcccacccc ccactaacgg tccaggcccc 240

aactttgacc tcagagacgg atacccggat cgataccagc cccgggacga ggaggtccag 300

gaaaggctgg accacctgct gtgctggctc ctggaacacc gaggccggtt ggaggggggt 360

ccaggctggc tggcagaggc catagtgacg tggcgggggc acctgacaaa actgctgacg 420

acaccgtatg agcggcagga gggctggcag ctggcagcct cccggttcca gggaacacta 480

tacctgagtg aagtggagac accgaacgct cgggcccaga ggcttgctcg gccaccgctc 540

ctccgggagc ttatgtacat gggatacaaa tttgagcagt acatgtgtgc agacaaacct 600

ggaagctccc cagacccctc tggggaggtt aacaccaacg tggccttctg ctctgtgcta 660

cgcagccgcc tgggaagcca ccctctgctc ttctcagggg aggtagactg cacagacccc 720

caagccccat ccacacagcc cccaacctgc tatgtggagc tcaagacctc caaggagatg 780

cacagccctg gccaatggag gagtttctac agacacaagc tcctgaaatg gtgggctcag 840

tcattcctcc caggggtccc gaatgttgtt gctggcttcc gtaacccaga cggttttgtc 900

tcttccctca agacctttcc taccatgaag atgtttgaat atgtcaggaa tgaccgtgac 960

ggctggaatc cctctgtgtg catgaacttc tgtgccgcct tccttagctt tgcccagagc 1020

acggttgtcc aggatgaccc caggctcgtt catctcttct cttgggagcc tggcggccca 1080

gtcaccgtgt ctgtacacca agatgcacct tacgccttcc tgcccatatg gtatgtggaa 1140

gctatgactc aggacctccc atcacccccc aagactccct ctcccaaata g 1191

<210> 6
<211> 2271
<212> DNA
<213> Homo sapiens

<400> 6
atggacaata ttaccaggca gaaccaattc tacgataccc aagtcatcaa acaagaaaac 60

gagtcaggct acgagaggag accactggaa atggagcagc agcaggccta tcgtccagaa 120

atgaagacag agatgaagca aggagcaccc accagcttcc tcccgcctga agcttctcaa 180

ctcaagccag acaggcagca attccagagt cgaaagaggc cttatgaaga aaaccgggga 240

cgggggtact ttgagcaccg agaggatagg aggggccgct ctcctcagcc tcctgctgaa 300

gaggatgaag atgactttga tgataccctt gttgctattg acacctataa ctgcgacctc 360

cacttcaagg tggcccgaga tcggagtagt ggctatccgc tcacaattga gggctttgca 420

tacctgtggt caggagcccg tgccagctat ggggtcagaa ggggccgtgt atgcttcgag 480

atgaagatca atgaggaaat ctccgtgaag caccttccgt ctacagagcc tgacccccac 540

gtggtccgta tcggctggtc cctggactcc tgcagcaccc agctaggcga agagcctttc 600

tcctatggct atggaggcac tgggaagaag tccaccaata gccggtttga aaactacgga 660

gacaagtttg cagagaacga tgtgattggc tgctttgcgg attttgaatg tggaaatgac 720

gtggaactgt cttttaccaa gaatggaaag tggatgggca ttgctttccg aatccagaag 780

gaagccttgg ggggtcaggc cctctatcct catgtcctgg tgaagaattg cgcagtggag 840

ttcaacttcg gacagagagc agagccctac tgttctgtcc tcccggggtt taccttcatc 900
cagcaccttc cccttagtga gcgtatccgg ggcaccgttg gaccaaagag caaggcagaa 960
tgtgagattc tgatgatggt gggcctgcct gctgctggca agaccacatg ggccatcaaa 1020
catgcagcct ccaacccttc caagaagtac aacatcctgg gtaccaatgc catcatggat 1080
aagatgcggg tgatgggcct acgccggcag cggaactatg ctggccgctg ggatgtcctg 1140
atccagcagg ccacccagtg cctcaaccgc ctcatccaga ttgctgcccg caagaaacgc 1200
aactatatcc tagatcagac aaatgtttat gggtcagccc agagacgaaa aatgagacca 1260
tttgaaggct tccagcgcaa agctattgta atttgtccca ctgacgagga cctaaaagac 1320
cgaacaataa agcgaaccga cgaggaaggg aaggatgtcc cagatcatgc ggtcttagaa 1380
atgaaagcca acttcacgtt gccagatgtt ggggacttcc tggatgaggt tctgttcatt 1440
gagctgcagc gggaggaagc ggacaagcta gtgaggcagt acaacgagga aggccgcaag 1500
gctgggccac cccctgaaaa gcgctttgac aaccgaggtg gtggtggctt ccggggccgc 1560
gggggtggtg gtggcttcca gcgctatgaa aaccgaggac cccctggagg caaccgtggc 1620
ggcttccaga accgaggggg aggcagcggt ggaggaggca actaccgagg aggtttcaac 1680
cgcagcggag gtggtggcta tagccagaac cgctggggta acaacaaccg ggataacaac 1740
aactccaaca acagaggcag ctacaaccgg gctccccagc aacagccgcc accacagcag 1800
cctccgccac cacagccacc accccagcag ccaccgccac cacccagcta cagccctgct 1860
cggaaccccc caggggccag cacctacaat aagaacagca acatccctgg ctcaagcgcc 1920
aataccagca cccccaccgt cagcagctac agccctccac agccgagtta cagccagcca 1980
ccctacaacc agggaggtta cagccagggc tacacagccc caccgcctcc acctccacca 2040
ccacctgcct acaactatgg gagctacggc ggttacaacc cggcccccta taccccaccg 2100
ccacccccca ccgcacagac ctaccctcag cccagctata accagtatca gcagtatgcc 2160
cagcagtgga accagtacta tcagaaccag ggccagtggc cgccatacta cgggaactac 2220
gactacggga gctactccgg gaacacacag ggtggcacaa gtacacagta g 2271

<210> 7
<211> 1581
<212> DNA
<213> Homo sapiens

<400> 7
atggcctcaa acgattatac ccaacaagca acccaaagct atggggccta ccccacccag 60
cccgggcagg gctattccca gcagagcagt cagccctacg gacagcagag ttacagtggt 120
tatagccagt ccacggacac ttcaggctat ggccagagca gctattcttc ttatggccag 180
agccagaaca caggctatgg aactcagtca actccccagg gatatggctc gactggcggc 240
tatggcagta gccagagctc ccaatcgtct tacgggcagc agtcctccta tcctggctat 300
ggccagcagc cagctcccag cagcacctcg ggaagttacg gtagcagttc tcagagcagc 360

agctatgggc agccccagag tgggagctac agccagcagc ctagctatgg tggacagcag 420

caaagctatg gacagcagca aagctataat ccccctcagg gctatggaca gcagaaccag 480

tacaacagca gcagtggtgg tggaggtgga ggtggaggtg gaggtaacta tggccaagat 540

caatcctcca tgagtagtgg tggtggcagt ggtggcggtt atggcaatca agaccagagt 600

ggtggaggtg gcagcggtgg ctatggacag caggaccgtg gaggccgcgg caggggtggc 660

agtggtggcg gcggcggcgg cggcggtggt ggttacaacc gcagcagtgg tggctatgaa 720

cccagaggtc gtggaggtgg ccgtggaggc agaggtggca tgggcggaag tgaccgtggt 780

ggcttcaata aatttggtgg ccctcgggac caaggatcac gtcatgactc cgaacaggat 840

aattcagaca acaacaccat ctttgtgcaa ggcctgggtg agaatgttac aattgagtct 900

gtggctgatt acttcaagca gattggtatt attaagacaa acaagaaaac gggacagccc 960

atgattaatt tgtacacaga cagggaaact ggcaagctga agggagaggc aacggtctct 1020

tttgatgacc caccttcagc taaagcagct attgactggt ttgatggtaa agaattctcc 1080

ggaaatccta tcaaggtctc atttgctact cgccgggcag actttaatcg gggtggtggc 1140

aatggtcgtg gaggccgagg gcgaggagga cccatgggcc gtggaggcta tggaggtggt 1200

ggcagtggtg gtggtggccg aggaggattt cccagtggag gtggtggcgg tggaggacag 1260

cagcgagctg gtgactggaa gtgtcctaat cccacctgtg agaatatgaa cttctcttgg 1320

aggaatgaat gcaaccagtg taaggcccct aaaccagatg gcccaggagg gggaccaggt 1380

ggctctcaca tggggggtaa ctacggggat gatcgtcgtg gtggcagagg aggctatgat 1440

cgaggcggct accggggccg cggcggggac cgtggaggct tccgaggggg ccggggtggt 1500

ggggacagag gtggctttgg ccctggcaag atggattcca ggggtgagca cagacaggat 1560

cgcagggaga ggccgtatta a 1581

<210> 8
<211> 783
<212> DNA
<213> Homo sapiens

<400> 8
atgaaggtag aggtgctgcc tgccctgacc gacaactaca tgtacctggt cattgatgat 60

gagaccaagg aggctgccat tgtggatccg gtgcagcccc agaaggtcgt ggacgcggcg 120

agaaagcacg gggtgaaact gaccacagtg ctcaccaccc accaccactg ggaccatgct 180

ggcgggaatg agaaactggt caagctggag tcgggactga aggtgtacgg gggtgacgac 240

cgtatcgggg ccctgactca caagatcact cacctgtcta cactgcaggt ggggtctctg 300

aacgtcaagt gcctggcgac cccgtgccac acttcaggac acatttgtta cttcgtgagc 360

aagcccggag gctcggagcc ccctgccgtg ttcacaggtg acaccttgtt tgtggctggc 420

tgcgggaagt tctatgaagg gactgcggat gagatgtgta aagctctgct ggaggtcttg 480

ggccggctcc ccccggacac aagagtctac tgtggccacg agtacaccat caacaacctc 540

aagtttgcac gccacgtgga gcccggcaat gccgccatcc gggagaagct ggcctgggcc 600

aaggagaagt acagcatcgg ggagcccaca gtgccatcca ccctggcaga ggagtttacc 660

tacaacccct tcatgagagt gagggagaag acggtgcagc agcacgcagg tgagacggac 720

ccggtgacca ccatgcgggc cgtgcgcagg gagaaggacc agttcaagat gccccgggac 780

tga 783

<210> 9
<211> 954
<212> DNA
<213> Homo sapiens

<400> 9
atgtcccacg gccccaagca gcccggcgcg gccgccgcgc cggcgggcgg caaggctccg 60

ggccagcatg ggggcttcgt ggtgactgtc aagcaagagc gcggcgaggg tccacgcgcg 120

ggcgagaagg ggtcccacga ggaggagccg gtgaagaaac gcggctggcc caagggcaag 180

aagcggaaga agattctgcc gaatgggccc aaggcaccgg tcacgggcta cgtgcgcttc 240

ctgaacgagc ggcgcgagca gatccgcacg cgccacccgg atctgccctt tcccgagatc 300

accaagatgc tgggcgccga gtggagcaag ctgcagccaa cggaaaagca gcggtacctg 360

gatgaggccg agagagagaa gcagcagtac atgaaggagc tgcgggcgta ccagcagtct 420

gaagcctata agatgtgcac ggagaagatc caggagaaga agatcaagaa agaagactcg 480

agctctgggc tcatgaacac tctcctgaat ggacacaagg gtggggactg cgatggcttc 540

tccaccttcg atgttcccat cttcactgaa gagttcttgg accaaaacaa agcgcgtgag 600

gcggagcttc ggcgcttgcg gaagatgaat gtggccttcg aggagcagaa cgcggtactg 660

cagaggcaca cgcagagcat gagcagcgcg cgcgagcgtc tggagcagga gctggcgctg 720

gaggagcgga ggacgctggc gctgcagcag cagctccagg ccgtgcgcca ggcgctcacc 780

gccagcttcg cctcactgcc ggtgccgggc acgggcgaaa cgcccacgct gggcactctg 840

gacttctaca tggcccggct tcacggagcc atcgagcgcg accccgccca gcacgagaag 900

ctcatcgtcc gcatcaagga aatcctggcc caggtcgcca gcgagcacct gtga 954

<210> 10
<211> 675
<212> DNA
<213> Homo sapiens

<400> 10
atgagttcct atttcgtgaa ctccaccttc cccgtcactc tggccagcgg gcaggagtcc 60

ttcctgggcc agctaccgct ctattcgtcg ggctatgcgg acccgctgag acattacccc 120

gcgccctacg ggccagggcc gggccaggac aagggctttg ccacttcctc ctattacccg 180

ccggcgggcg gtggctacgg ccgagcggcg ccctgcgact acgggccggc gccggccttc 240

taccgcgaga aagagtcggc ctgcgcactc tccggcgccg acgagcagcc cccgttccac 300

cccgagccgc ggaagtcgga ctgcgcgcag gacaagagcg tgttcggcga gacagaagag 360

cagaagtgct ccactccggt ctacccgtgg atgcagcgga tgaattcgtg caacagttcc 420

tcctttgggc ccagcggccg gcgaggccgc cagacataca cacgttacca gacgctggag 480
ctggagaagg agtttcacta caatcgctac ctgacgcggc ggcggcgcat cgagatcgcg 540
cacgccctgt gcctgacgga gaggcagatc aagatatggt tccagaaccg acgcatgaag 600
tggaaaaagg agagcaaact gctcagcgcg tctcagctca gtgccgagga ggaggaagaa 660
aaacaggccg agtga 675

<210> 11
<211> 2190
<212> DNA
<213> Homo sapiens

<400> 11
atgggaaaaa aatacaagaa cattgttcta ctaaaaggat tagaggtcat caatgattat 60
cattttagaa tggttaagtc cttactgagc aacgatttaa aacttaattt aaaaatgaga 120
gaagagtatg acaaaattca gattgctgac ttgatggaag aaaagttccg aggtgatgct 180
ggtttgggca aactaataaa aattttcgaa gatataccaa cgcttgaaga cctggctgaa 240
actcttaaaa aagaaaagtt aaaagtaaaa ggaccagccc tatcaagaaa gaggaagaag 300
gaagtggatg ctacttcacc tgcaccctcc acaagcagca ctgtcaaaac tgaaggagca 360
gaggcaactc ctggagctca gaaaagaaaa aaatcaacca aagaaaaggc tggacccaaa 420
gggagtaagg tgtccgagga acagactcag cctccctctc ctgcaggagc cggcatgtcc 480
acagccatgg gccgttcccc atctcccaag acctcattgt cagctccacc caacacttct 540
tcaactgaga acccgaaaac agtggccaaa tgtcaggtaa ctcccagaag aaatgttctc 600
caaaaacgcc cagtgatagt gaaggtactg agtacaacaa agccatttga atatgagacc 660
ccagaaatgg agaaaaaaat aatgtttcat gctacagtgg ctacacagac acagttcttc 720
catgtgaagg ttttaaacac cagcttgaag gagaaattca atggaaagaa aatcatcatc 780
atatcagatt atttggaata tgatagtctc ctagaggtca atgaagaatc tactgtatct 840
gaagctggtc ctaaccaaac gtttgaggtt ccaaataaaa tcatcaacag agcaaaggaa 900
actctgaaga ttgatattct tcacaaacaa gcttcaggaa atattgtata tggggtattt 960
atgctacata agaaaacagt aaatcagaag accacaatct acgaaattca ggatgataga 1020
ggaaaaatgg atgtagtggg gacaggacaa tgtcacaata tcccctgtga agaaggagat 1080
aagctccaac ttttctgctt tcgacttaga aaaaagaacc agatgtcaaa actgatttca 1140
gaaatgcata gttttatcca gataaagaaa aaaacaaacc cgagaaacaa tgaccccaag 1200
agcatgaagc taccccagga acagagtcag cttccaaatc cttcagaggc cagcacaacc 1260
ttccctgaga gccatcttcg gactcctcag atgccaccaa caactccatc cagcagtttc 1320
ttcaccaaga aaagtgaaga cacaatctcc aaaatgaatg acttcatgag gatgcagata 1380
ctgaaggaag ggagtcattt tccaggaccg ttcatgacca gcataggccc agctgagagc 1440
catccccaca ctcctcagat gcctccatca acaccaagca gcagtttctt aaccacgttg 1500

aaaccaagac tgaagactga acctgaagaa gtttccatag aagacagtgc ccagagtgac 1560
ctcaaagaag tgatggtgct gaacgcaaca gaatcatttg tatatgagcc caaagagcag 1620
aagaaaatgt ttcatgccac agtggcaact gagaatgaag tcttccgagt gaaggttttt 1680
aatattgacc taaaggagaa gttcacccca aagaagatca ttgccatagc aaattatgtt 1740
tgccgcaatg ggttcctgga ggtatatcct ttcacacttg tggctgatgt gaatgctgac 1800
cgaaacatgg agatcccaaa aggattgatt agaagtgcca gcgtaactcc taaaatcaat 1860
cagctttgct cacaaactaa aggaagtttt gtgaatgggg tgtttgaggt acataagaaa 1920
aatgtaaggg gtgaattcac ttattatgaa atacaagata atacagggaa gatggaagtg 1980
gtggtgcatg gacgactgac cacaatcaac tgtgaggaag gagataaact gaaactcacc 2040
tgctttgaat tggcaccgaa aagtgggaat accggggagt tgagatctgt aattcatagt 2100
cacatcaagg tcatcaagac caggaaaaac aagaaagaca tactcaatcc tgattcaagt 2160
atggaaactt caccagactt tttcttctaa 2190

<210> 12
<211> 840
<212> DNA
<213> Homo sapiens

<400> 12
atgaacaagg tagagctgga gtctcgcctg gaagggctga ccgacgagat caacttcctc 60
aggcagctat atgaagagga gatccgggag ctgcagtccc agatctcgga cacatctgtg 120
gtgctgtcca tggacaacag ccgctccctg gacatggaca gcatcattgc tgaggtcaag 180
gcacagtacg aggatattgc caaccgcagc cgggctgagg ctgagagcat gtaccagatc 240
aagtatgagg agctgcagag cctggctggg aagcacgggg atgacctgcg gcgcacaaag 300
actgagatct ctgagatgaa ccggaacatc agccggctcc aggctgagat tgagggcctc 360
aaaggccaga gggcttccct ggaggccgcc attgcagatg ccgagcagcg tggagagctg 420
gccattaagg atgccaacgc caagttgtcc gagctggagg ccgccctgca gcgggccaag 480
caggacatgg cgcggcagct gcgtgagtac caggagctga tgaacgtcaa gctggccctg 540
gacatcgaga tcgccaccta caggaagctg ctggagggcg aggagagccg gctggagtct 600
gggatgcaga acatgagtat tcatacgaag accaccagcg gctatgcagg tggtctgagc 660
tcggcctatg ggggcctcac aagccccggc ctcagctaca gcctgggctc cagctttggc 720
tctggcgcgg gctccagctc cttcagccgc accagctcct ccagggccgt ggttgtgaag 780
aagatcgaga cacgtgatgg gaagctggtg tctgagtcct ctgacgtcct gcccaagtga 840

<210> 13
<211> 630
<212> DNA
<213> Homo sapiens

<400> 13
atgggctccg tgtccaacca gcagtttgca ggtggctgcg ccaaggcggc agaagaggcg 60

cccgaggagg cgccggagga cgcggcccgg gcggcggacg agcctcagct gctgcacggt 120

gcgggcatct gtaagtggtt caacgtgcgc atggggttcg gcttcctgtc catgaccgcc 180

cgcgccgggg tcgcgctcga ccccccagtg gatgtctttg tgcaccagag taagctgcac 240

atggaagggt tccggagctt gaaggagggt gaggcagtgg agttcacctt taagaagtca 300

gccaagggtc tggaatccat ccgtgtcacc ggacctggtg gagtattctg tattgggagt 360

gagaggcggc caaaaggaaa gagcatgcag aagcgcagat caaaaggaga caggtgctac 420

aactgtggag gtctagatca tcatgccaag gaatgcaagc tgccacccca gcccaagaag 480

tgccacttct gccagagcat cagccatatg gtagcctcat gtccgctgaa ggcccagcag 540

ggccctagtg cacagggaaa gccaacctac tttcgagagg aagaagaaga aatccacagc 600

cctaccctgc tcccggaggc acagaattga 630

<210> 14
<211> 2187
<212> DNA
<213> Homo sapiens

<400> 14
atgaaccagc cagagtctgc caacgatcct gaacccctgt gtgcagtgtg tggccaagcc 60

cactccttgg aggaaaacca cttctacagc tatccagagg aagtggatga tgacctcatc 120

tgccacatct gcctgcaggc tttgctggac cccctggaca ctccgtgtgg acacacctac 180

tgcaccctct gcctcaccaa cttcctggtg gagaaggact tctgtcccat ggaccgcaag 240

cctctggttc tgcagcactg caagaagtcc agcatcctgg tcaacaaact cctcaacaag 300

ctactggtga cctgcccatt cagggagcac tgcacccagg tgttgcagcg ctgtgacctc 360

gagcatcact ttcaaaccag ctgtaaaggt gcctcccact acggcctgac caaagatagg 420

aagaggcgct cacaagatgg ctgtccagac ggctgtgcga gcctcacagc cacggctccc 480

tccccagagg tttctgcagc tgccaccatc tccttaatga cagacgagcc tggcctagac 540

aaccctgcct acgtgtcctc ggcagaggac gggcagccag caatcagccc agtggactct 600

ggccggagca accgaactag ggcacggccc tttgagagat ccactattag aagcagatca 660

tttaaaaaaa taaatcgagc tttgagtgtt cttcgaagga caaagagcgg gagtgcagtt 720

gccaaccatg ccgaccaggg cagggaaaat tctgaaaaca ccactgcccc tgaagtcttt 780

ccaaggttgt accacctgat tccagatggt gaaattacca gcatcaagat caatcgagta 840

gatcccagtg aaagcctctc tattaggctg gtgggaggta gcgaaacccc actggtccat 900

atcattatcc aacacattta tcgtgatggg gtgatcgcca gagacggccg gctactgcca 960

ggagacatca ttctaaaggt caacgggatg gacatcagca atgtccctca caactacgct 1020

gtgcgtctcc tgcggcagcc ctgccaggtg ctgtggctga ctgtgatgcg tgaacagaag 1080

ttccgcagca ggaacaatgg acaggccccg gatgcctaca gaccccgaga tgacagcttt 1140

catgtgattc tcaacaaaag tagccccgag gagcagcttg gaataaaact ggtgcgcaag 1200

gtggatgagc ctggggtttt catcttcaat gtgctggatg gcggtgtggc atatcgacat 1260

ggtcagcttg aggagaatga ccgtgtgtta gccatcaatg gacatgatct tcgatatggc 1320

agcccagaaa gtgcggctca tctgattcag gccagtgaaa gacgtgttca cctcgtcgtg 1380

tcccgccagg ttcggcagcg gagccctgac atctttcagg aagccggctg gaacagcaat 1440

ggcagctggt ccccagggcc aggggagagg agcaacactc ccaagcccct ccatcctaca 1500

attacttgtc atgagaaggt ggtaaatatc caaaaagacc ccggtgaatc tctcggcatg 1560

accgtcgcag ggggagcatc acatagagaa tgggatttgc ctatctatgt catcagtgtt 1620

gagcccggag gagtcataag cagagatgga agaataaaaa caggtgacat tttgttgaat 1680

gtggatgggg tcgaactgac agaggtcagc cggagtgagg cagtggcatt attgaaaaga 1740

acatcatcct cgatagtact caaagctttg gaagtcaaag agtatgagcc ccaggaagac 1800

tgcagcagcc cagcagccct ggactccaac cacaacatgg ccccacccag tgactggtcc 1860

ccatcctggg tcatgtggct ggaattacca cggtgcttgt ataactgtaa agatattgta 1920

ttacgaagaa acacagctgg aagtctgggc ttctgcattg taggaggtta tgaagaatac 1980

aatggaaaca aacctttttt catcaaatcc attgttgaag gaacaccagc atacaatgat 2040

ggaagaatta gatgtggtga tattcttctt gctgtcaatg gtagaagtac atcaggaatg 2100

atacatgctt gcttggcaag actgctgaaa gaacttaaag gaagaattac tctaactatt 2160

gtttcttggc ctggcacttt tttatag 2187

<210> 15
<211> 2019
<212> DNA
<213> Homo sapiens

<400> 15
atgggcgtgg gccggctgga catgtatgtg ctgcacccgc cctccgccgg cgccgagcgc 60

acgctggcct gtgtgtgcgc cctgctggtg tggcaccccg ccggccccgg cgagaaggtg 120

gtgcgcgtgc tgttccccgg ttgcaccccg cccgcctgcc tcctggacgg cctggtccgc 180

ctgcagcact tgaggttcct gcgagagccc gtggtgacgc cccaggacct ggaggggccg 240

gggcgagccg agagcaaaga gagcgtgggc tcccgggaca gctcgaagag agagggcctc 300

ctggccaccc accctagacc tggccaggag cgccctgggg tggcccgcaa ggagccagca 360

cgggctgagg ccccacgcaa gactgagaaa gaagccaaga cccccccgga gttgaagaaa 420

gaccccaaac cgagtgtctc ccggacccag ccgcgggagg tgcgccgggc agcctcttct 480

gtgcccaacc tcaagaagac gaatgcccag gcggcaccca agccccgcaa agcgcccagc 540

acgtcccact ctggcttccc gccggtggca aatggacccc gcagcccgcc cagcctccga 600

tgtggagaag ccagcccccc cagtgcggcc tgcggctctc cggcctccca gctggtggcc 660

acgcccagcc tggagctggg gccgatccca gccggggagg agaaggcact ggagctgcct 720

ttggccgcca gctcaatccc aaggccacgc acaccctccc ctgagtccca ccggagcccc 780

gcagagggca gcgagcggct gtcgctgagc ccactgcggg gcggggaggc cgggccagac 840

gcctcaccca cagtgaccac acccacggtg accacgccct cactacccgc agaggtgggc 900
tccccgcact cgaccgaggt ggacgagtcc ctgtcggtgt cctttgagca ggtgctgccg 960
ccatccgccc ccaccagtga ggctgggctg agcctcccgc tgcgtggccc ccgggcgcgg 1020
cgctcggctt ccccacacga tgtggacctg tgcctggtgt caccctgtga atttgagcat 1080
cgcaaggcgg tgccaatggc accggcacct gcgtcccccg gcagctcgaa tgacagcagt 1140
gcccggtcac aggaacgggc aggtgggctg ggggccgagg agacgccacc cacatcggtc 1200
agcgagtccc tgcccaccct gtctgactcg gatcccgtgc ccctggcccc cggtgcggca 1260
gactcagacg aagacacaga gggctttgga gtccctcgcc acgacccttt gcctgacccc 1320
ctcaaggtcc ccccaccact gcctgaccca tccagcatct gcatggtgga ccccgagatg 1380
ctgcccccca agacagcacg gcaaacggag aacgtcagcc gcacccggaa gcccctggcc 1440
cgccccaact cacgcgctgc cgcccccaaa gccactccag tggctgctgc caaaaccaag 1500
gggcttgctg gtggggaccg tgccagccga ccactcagtg cccggagtga gcccagtgag 1560
aagggaggcc gggcacccct gtccagaaag tcctcaaccc ccaagactgc cactcgaggc 1620
ccgtcggggt cagccagcag ccggcccggg gtgtcagcca ccccacccaa gtccccggtc 1680
tacctggacc tggcctacct gcccagcggg agcagcgccc acctggtgga tgaggagttc 1740
ttccagcgcg tgcgcgcgct ctgctacgtc atcagtggcc aggaccagcg caaggaggaa 1800
ggcatgcggg ccgtcctgga cgcgctactg gccagcaagc agcattggga ccgtgacctg 1860
caggtgaccc tgatccccac tttcgactcg gtggccatgc atacgtggta cgcagagacg 1920
cacgcccggc accaggcgct gggcatcacg gtgttgggca gcaacagcat ggtgtccatg 1980
caggatgacg ccttcccggc ctgcaaggtg gagttctag 2019

<210> 16
<211> 1422
<212> DNA
<213> Homo sapiens

<400> 16
atggctgccc cgtgtttgct gcggcaagga cgagccgggg cgctgaagac tatgctccag 60
gaagcccagg tgtttcgagg acttgcttct acggtttctt tgtctgcgga atcagggaag 120
agtgaaaagg gtcagccaca gaattccaag aagcaaagtc caccaaaaaa tgtagtggaa 180
ccaaaggaga ggggcaagct cctagccacc cagacagcag ctgaattgtc taaaaactta 240
tcttcaccca gttcttaccc gccagctgtg aataagggca ggaaggtagc tagtcccagt 300
cccagtggca gcgtgctatt cacagatgaa ggggttccga aatttttgtc aagaaagact 360
ttggtagagt ttccacagaa agttctgtct ccattcagaa aacagggctc tgattcagaa 420
gctcgtcagg tgggtcggaa agtgacgtcg ccttcgtctt catcctcgtc cagctcctct 480
gattctgaat ctgatgatga ggctgacgtt tcagaggtca ctcctcgagt ggtgagcaaa 540
ggcagagggg ggcttcgaaa accagaggcc tctcattcct ttgaaaacag agccccccga 600
gttacagtat cagcaaaaga gaaaaccttg ctgcagaagc cgcatgtgga cattactgat 660

ccagagaagc cccaccagcc aaagaagaaa gggtcccctg ctaagccatc agaaggcagg 720
gaaaatgcga gaccaaaaac cacaatgccc agatctcaag tagatgaaga gtttttgaag 780
caaagtttaa aggaaaaaca attgcagaaa acatttagat taaatgaaat agataaagaa 840
agccaaaagc catttgaagt taaaggaccc ttacctgtcc acacaaaatc agggttgtct 900
gcgccaccga agggcagccc agcgcctgct gtgttggcag aagaggccag agcagagggg 960
cagctgcaag ccagtcctcc tggggcggca gaggggcatc tggaaaaacc cgtgccagag 1020
ccccagcgca aggcggcccc tcccctgccc agaaaggaaa cctcagggac gcagggaata 1080
gaaggccacc tgaagggtgg acaggcaatc gtggaagatc agataccacc aagcaatttg 1140
gagacagttc ctgttgagaa taaccacggt ttccatgaaa agacagcagc gctgaagctt 1200
gaggccgagg gcgaggccat ggaagatgca gccgcgccag ggaacgaccg aggcggcaca 1260
caggagccag ccccagtgcc tgctgagccg tttgacaaca ctacctacaa gaacctgcag 1320
catcatgact acagcacgta caccttctta gacctcaacc tcgaactctc aaaattcagg 1380
atgcctcagc cctcctcagg ccgggagtca cctcgacact ga 1422

<210> 17
<211> 1911
<212> DNA
<213> Homo sapiens

<400> 17
atgaacccca gtgcccccag ctaccccatg gcctcgctct acgtggggga cctccacccc 60
gacgtgaccg aggcgatgct ctacgagaag ttcagcccgg ccgggcccat cctctccatc 120
cgggtctgca gggacatgat cacccgccgc tccttgggct acgcgtatgt gaacttccag 180
cagccggcgg acgcggagcg tgctttggac accatgaatt ttgatgttat aaagggcaag 240
ccagtacgca tcatgtggtc tcagcgtgat ccatcacttc gcaaaagtgg agtaggcaac 300
atattcatta aaaatctgga caaatccatt gataataaag cactgtatga tacattttct 360
gcttttggta acatcctttc atgtaaggtg gtttgtgatg aaaatggttc caagggctat 420
ggatttgtac actttgagac gcaggaagca gctgaaagag ctattgaaaa aatgaatgga 480
atgctcctaa atgatcgcaa agtatttgtt ggacgattta agtctcgtaa agaacgagaa 540
gctgaacttg gagctagggc aaaagaattc accaatgttt acatcaagaa ttttggagaa 600
gacatggatg atgagcgcct taaggatctc tttggcaagt ttgggcctgc cttaagtgtg 660
aaagtaatga ctgatgaaag tggaaaatcc aaaggatttg gatttgtaag ctttgaaagg 720
catgaagatg cacagaaagc tgtggatgag atgaacggaa aggagctcaa tggaaaacaa 780
atttatgttg gtcgagctca gaaaaaggtg gaacggcaga cggaacttaa gcgcaaattt 840
gaacagatga aacaagatag gatcaccaga taccagggtg ttaatcttta tgtgaaaaat 900
cttgatgatg gtattgatga tgaacgtctc cggaaagagt tttctccatt tggtacaatc 960
actagtgcaa aggttatgat ggagggtggt cgcagcaaag ggtttggttt tgtatgtttc 1020

tcctccccag aagaagccac taaagcagtt acagaaatga acggtagaat tgtggccaca 1080
aagccattgt atgtagcttt agctcagcgc aaagaagagc gccaggctca cctcactaac 1140
cagtatatgc agagaatggc aagtgtacga gctgttccca accctgtaat caacccctac 1200
cagccagcac ctccttcagg ttacttcatg gcagctatcc cacagactca gaaccgtgct 1260
gcatactatc ctcctagcca aattgctcaa ctaagaccaa gtcctcgctg gactgctcag 1320
ggtgccagac ctcatccatt ccaaaatatg cccggtgcta tccgcccagc tgctcctaga 1380
ccaccattta gtactatgag accagcttct tcacaggttc cacgagtcat gtcaacacag 1440
cgtgttgcta acacatcaac acagacaatg ggtccacgtc ctgcagctgc agccgctgca 1500
gctactcctg ctgtccgcac cgttccacag tataaatatg ctgcaggagt tcgcaatcct 1560
cagcaacatc ttaatgcaca gccacaagtt acaatgcaac agcctgctgt tcatgtacaa 1620
ggtcaggaac ctttgactgc ttccatgttg gcatctgccc ctcctcaaga gcaaaagcaa 1680
atgttgggtg aacggctgtt tcctcttatt caagccatgc accctactct tgctggtaaa 1740
atcactggca tgttgttgga gattgataat tcagaacttc ttcatatgct cgagtctcca 1800
gagtcactcc gttctaaggt tgatgaagct gtagctgtac tacaagccca ccaagctaaa 1860
gaggctgccc agaaagcagt taacagtgcc accggtgttc caactgttta a 1911

<210> 18
<211> 780
<212> DNA
<213> Homo sapiens

<400> 18
atgctggaga gtagcggctg caaagcgctg aaggagggcg tgctggagaa gcgcagcgac 60
gggttgttgc agctctggaa gaaaaagtgt tgcatcctca ccgaggaagg gctgctgctt 120
atcccgccca agcagctgca acaccagcag cagcagcaac agcagcagca gcagcagcaa 180
caacagcccg ggcaggggcc ggccgagccg tcccaaccca gtggccccgc tgtcgccagc 240
ctcgagccgc cggtcaagct caaggaactg cacttctcca acatgaagac cgtggactgt 300
gtggagcgca agggcaagta catgtacttc actgtggtga tggcagaggg caaggagatc 360
gactttcggt gcccgcaaga ccagggctgg aacgccgaga tcacgctgca gatggtgcag 420
tacaagaatc gtcaggccat cctggcggtc aaatccacgc ggcagaagca gcagcacctg 480
gtccagcagc agcccccctc gcagccgcag ccgcagccgc agctccagcc ccaaccccag 540
cctcagcctc agccgcaacc ccagccccaa tcacaacccc agcctcagcc ccaacccaag 600
cctcagcccc agcagctcca cccgtatccg catccacatc cacatccaca ctctcatcct 660
cactcgcacc cacaccctca cccgcacccg catccgcacc aaataccgca cccacaccca 720
cagccgcact cgcagccgca cgggcaccgg cttctccgca gcacctccaa ctctgcctga 780

<210> 19
<211> 1719
<212> DNA
<213> Homo sapiens

<400> 19
atggcggatt tcgaagagtt gaggaatatg gtttctagtt ttagggtttc tgaactacaa 60
gtattactag gctttgctgg acggaataaa agtggacgca agcatgacct cctgatgagg 120
gcgctgcatt tattgaagag cggctgcagc cctgcggttc agattaaaat ccgagaattg 180
tatagacgcc gatatccacg aactcttgaa ggactttctg atttatccac aatcaaatca 240
tcggttttca gtttggatgg tggctcatca cctgtagaac ctgacttggc cgtggctgga 300
atccactcgt tgccttccac ttcagttaca cctcactcac catcctctcc tgttggttct 360
gtgctgcttc aagatactaa gcccacattt gagatgcagc agccatctcc cccaattcct 420
cctgtccatc ctgatgtgca gttaaaaaat ctgccctttt atgatgtcct tgatgttctc 480
atcaagccca cgagtttagt tcaaagcagt attcagcgat ttcaagagaa gttttttatt 540
tttgctttga cacctcaaca agttagagag atatgcatat ccagggattt tttgccaggt 600
ggtaggagag attatacagt ccaagttcag ttgagacttt gcctggcaga gacaagttgc 660
cctcaagaag ataactatcc aaatagtcta tgtataaaag taaatgggaa gctatttcct 720
ttgcctggct atgcaccacc gcctaaaaat gggattgaac agaagcgccc tggacgcccc 780
ttgaatatta catctttagt taggttatct tcagctgtgc caaaccaaat ttccatttct 840
tgggcatcag aaattgggaa gaattactct atgtctgtat atcttgtacg gcagcttaca 900
tcagccatgt tattacagag attaaaaatg aaaggtatta gaaaccctga tcattccaga 960
gcactaatta aagaaaaact tactgcagat cctgatagtg aaattgctac aactagcctt 1020
cgggtatcct tgatgtgccc tttaggaaaa atgaggctga caatcccatg ccgtgcagtg 1080
acttgtacac atctgcagtg ttttgatgct gccctctatc tacaaatgaa tgagaaaaag 1140
cccacctgga tttgtcctgt gtgtgacaaa aaagctgcct atgaaagtct aatattagat 1200
gggcttttta tggaaattct caatgactgt tctgatgtag atgagatcaa attccaagaa 1260
gatggttctt ggtgtccaat gagaccgaag aaagaagcta tgaaagtatc cagccaaccg 1320
tgtacaaaaa tagaaagttc aagcgtcctc agtaagcctt gttcagtgac tgtagccagt 1380
gaggcaagca agaagaaagt agatgttatt gatcttacaa tagaaagctc ttctgacgaa 1440
gaggaagacc ctcctgccaa aaggaaatgc atctttatgt cagaaacaca aagcagccca 1500
accaaagggg ttctcatgta tcagccatct tctgtaaggg tgcccagtgt gacttcggtt 1560
gatcctgctg ctattccgcc ttcattaaca gactactcag taccattcca ccatacgcca 1620
atatcaagca tgtcatcaga tttgccagga gaacaaagaa gaaatgatat taataatgaa 1680
ctgaagcttg gaacatcttc tgatactgtg caacagtga 1719

<210> 20
<211> 3072
<212> DNA
<213> Homo sapiens

<400> 20
atgacaatgc tcaccattga acagttatcc tacctgctca agtttgccat tcagaaaatg 60

aaacagccag ggacagatgc attccagaag cccgttccat tggaacagca ccctgactat 120
gcggaataca tcttccatcc aatggacctt tgtacattgg aaaagaatgc gaaaaagaaa 180
atgtatggct gcacagaagc cttcctggct gatgcaaagt ggattttgca caactgcatc 240
atttataatg ggggaaatca caaattgacg caaatagcga aagtagtcat caaaatctgt 300
gaacatgaga tgaatgaaat cgaagtatgt ccagaatgtt atctagctgc ttgccaaaaa 360
cgagataact ggttttgtga gccttgtagc aatccacatc ctttggtctg ggccaaactg 420
aaggggtttc cattctggcc tgcaaaagct ctaagggata aagacgggca ggtcgatgcc 480
cgattctttg gacaacatga cagggcctgg gttccaataa ataattgcta cctcatgtct 540
aaagaaattc ctttttctgt gaaaaagact aagagcatct tcaacagtgc catgcaagag 600
atggaggttt acgtggagaa catccgcagg aagtttgggg tttttaatta ctctccattt 660
aggacaccct acacacccaa cagccagtat caaatgctgc tcgatcccac caaccccagc 720
gccggcactg ccaagataga caagcaggag aaggtcaagc tcaactttga catgacggca 780
tcccccaaga tcctgatgag caagcctgtg ctgagtgggg gcacaggccg ccggatttcc 840
ttgtcggata tgccgcgctc ccccatgagc acaaactctt ctgtgcacac gggctccgac 900
gtggagcagg atgctgagaa gaaggccacg tcgagccact tcagtgcgag cgaggagtcc 960
atggacttcc tggataagag cacagcttca ccagcctcca ccaagacggg acaagcaggg 1020
agtttatccg gcagcccaaa gcccttctct cctcaactgt cagctcctat cacgacgaaa 1080
acggacaaaa cctccaccac cggcagcatc ctgaatctta acctggatcg aagcaaagct 1140
gagatggatt tgaaggagct gagcgagtcg gtccagcaac agtccacccc tgttcctctc 1200
atctctccca agcgccagat tcgtagcagg ttccagctga atcttgacaa gaccatagag 1260
agttgcaaag cacaattagg cataaatgaa atctcggaag atgtctatac ggccgtagag 1320
cacagcgatt cggaggattc tgagaagtca gatagtagcg atagtgagta tatcagtgat 1380
gatgagcaga agtctaagaa cgagccagaa gacacagagg acaaagaagg ttgtcagatg 1440
gacaaagagc catctgctgt taaaaaaaag cccaagccta caaacccagt ggagattaaa 1500
gaggagctga aaagcacgtc accagccagc gagaaggcag accctggagc agtcaaggac 1560
aaggccagcc ctgagcctga gaaggacttt tccgaaaagg caaaaccttc acctcacccc 1620
ataaaggata aactgaaggg aaaagatgag acggattccc caacagtcca tttgggcctg 1680
gactctgatt cagagagcga acttgtcata gatttaggag aagaccattc tgggcgggag 1740
ggtcgaaaaa ataagaagga acccaaagaa ccatctccca aacaggatgt tgtaggtaaa 1800
actccaccat ccacgacggt gggcagccat tctcccccgg aaacaccggt gctcacccgc 1860
tcttccgccc aaacttccgc ggctggcgcc acagccacca ccagcacgtc ctccacggtc 1920
accgtcacgg ccccggcccc cgccgccaca ggaagcccag tgaaaaagca gaggccgctt 1980
ttaccgaagg agactgcccc ggccgtgcag cgggtcgtgt ggaactcatc aactgtccag 2040
cagaaggaga tcacacagag cccatccacg tccaccatca ccctggtgac cagcacacag 2100

tcatcgcccc tggtcaccag ctcggggtcc atgagcaccc ttgtgtcctc agtcaacgct 2160
gacctgccca tcgccactgc ctcagctgat gtcgccgctg atattgccaa gtacactagc 2220
aaaatgatgg atgcaataaa aggaacaatg acagaaatat acaacgatct ttctaaaaac 2280
actactggaa gcacaatagc tgagattcgc aggctgagga tcgagataga gaagctccag 2340
tggctgcacc agcaagagct ctccgaaatg aaacacaact tagagctgac catggcggag 2400
atgcggcaga gcctggagca ggagcgggac cggctcatcg ccgaggtgaa gaagcagctg 2460
gagttggaga agcagcaggc ggtggatgag accaagaaga agcagtggtg cgccaactgc 2520
aagaaggagg ccatctttta ctgctgttgg aacaccagct actgtgacta cccctgccag 2580
caagcccact ggcctgagca catgaagtcc tgcacccagt cagctactgc tcctcagcag 2640
gaagcggatg ctgaggtgaa cacagaaaca ctaaataagt cctcccaggg gagctcctcg 2700
agcacacaat cagcaccttc agaaacggcc agcgcctcca aagagaagga gacgtcagct 2760
gagaaaagca aggagagtgg ctcgaccctt gacctttctg gctccagaga gacgccctcc 2820
tccattctct taggctccaa ccaaggctct gttagcaaaa ggtgtgacaa gcaacctgcc 2880
tatgccccaa ccaccacaga ccaccagccg caccccaact accccgccca gaagtaccat 2940
tcccggagta ataaatccag ttggagcagc agtgatgaga agaggggatc gacacgttcc 3000
gatcacaaca ccagtaccag cacgaagagc ctcctcccga aagagtctcg gctggacacc 3060
ttctgggact ag 3072

<210> 21
<211> 942
<212> DNA
<213> Homo sapiens

<400> 21
atgtcccaga gcaggcaccg cgccgaggcc ccgccgctgg agcgcgagga cagtgggacc 60
ttcagtttgg ggaagatgat aacagctaag ccagggaaaa caccgattca ggtattacac 120
gaatacggca tgaagaccaa gaacatccca gtttatgaat gtgaaagatc tgatgtgcaa 180
atacacgtgc ccactttcac cttcagagta accgttggtg acataacctg cacaggtgaa 240
ggtacaagta agaagctggc gaaacataga gctgcagagg ctgccataaa cattttgaaa 300
gccaatgcaa gtatttgctt tgcagttcct gaccccttaa tgcctgaccc ttccaagcaa 360
ccaaagaacc agcttaatcc tattggttca ttacaggaat tggctattca tcatggctgg 420
agacttcctg aatataccct ttcccaggag ggaggacctg ctcataagag agaatatact 480
acaatttgca ggctagagtc atttatggaa actggaaagg gggcatcaaa aaagcaagcc 540
aaaaggaatg ctgctgagaa atttcttgcc aaatttagta atatttctcc agagaaccac 600
atttctttaa caaatgtagt aggacattct ttaggatgta cttggcattc cttgaggaat 660
tctcctggtg aaaagatcaa cttactgaaa agaagcctcc ttagtattcc aaatacagat 720
tacatccagc tgcttagtga aattgccaag gaacaaggtt ttaatataac atatttggat 780

atagatgaac tgagcgccaa tggacaatat caatgtcttg ctgaactgtc caccagcccc 840

atcacagtct gtcatggctc cggtatctcc tgtggcaatg cacaaagtga tgcagctcac 900

aatgctttgc agtatttaaa gataatagca gaaagaaagt aa 942

<210> 22
<211> 765
<212> DNA
<213> Homo sapiens

<400> 22
atggcctcgt tgctgaaggt ggatcaggaa gtgaagctca aggttgattc tttcagggag 60

cggatcacaa gtgaggcaga agacttggtg gcaaattttt tcccaaagaa gttattagaa 120

cttgatagtt ttctgaagga accaatctta aacatccatg acctaactca gatccactct 180

gacatgaatc tcccagtccc tgaccccatt cttctcacca atagccatga tggactggat 240

ggtcccactt ataagaagcg aaggttggat gagtgtgaag aagccttcca aggaaccaag 300

gtgtttgtga tgcccaatgg gatgctgaaa agcaaccagc agctggtgga cattattgag 360

aaagtgaaac ctgagatccg gctgttgatt gagaaatgta acacggtcaa aatgtgggta 420

cagctcctga ttcccaggat agaagatgga aacaactttg gggtgtccat tcaggaggaa 480

acagttgcag agctaagaac tgttgagagt gaagctgcat cttatctgga ccagatttct 540

agatattata ttacaagagc caaattggtt tctaaaatag ctaaatatcc ccatgtggag 600

gactatcgcc gcaccgtgac agagattgat gagaaagaat atatcagcct tcggctcatc 660

atatcagagc tgaggaatca atatgtcact ctacatgaca tgatcctgaa aaatatcgag 720

aagatcaaac ggccccggag cagcaatgca gagactctgt actga 765

<210> 23
<211> 2271
<212> DNA
<213> Homo sapiens

<400> 23
atggcgtcgg ccccgccggc ctcgcccccg ggctcggagc cgccggggcc cgacccggag 60

ccgggcgggc cggacgggcc gggggcggcg caactggctc cgggccctgc ggagctacgc 120

ctcggagcgc ccgtcggcgg ccccgacccg cagtccccgg gcctggatga gcctgcgccc 180

ggggccgctg cagatggcgg ggcgcgttgg agcgccgggc cggccccggg gctggaggga 240

ggcccgcgag accccgggcc gtccgccccg ccgccgcgct ccggcccgcg ggggcagctt 300

gcgagccccg acgccccggg cccagggccg cgctccgaag cgccgcttcc agaactcgac 360

ccgttgttct cctggactga ggagcccgag gagtgtggcc ccgcgagctg cccggagagc 420

gcgcctttcc gcttgcaggg gtccagcagc agccaccgag cgcggggcga ggtcgacgtc 480

ttctctccct tccccgcgcc cacggcgggc gagctggcgc tggagcaagg tcccgggtcc 540

ccgccgcagc cctcggacct cagccagacc cacccccttc cgagcgagcc cgtggggagt 600

caggaggacg gcccccgcct ccgagccgtg ttcgatgccc tggacgggga tggggacggt 660

ttcgtccgca tcgaggactt catccagttt gctacggtct acggggcaga gcaggtgaag 720

gacttaacta agtacttgga tcccagtggg ctcggcgtga tcagctttga agacttctac 780

caagggatca cagccatcag aaacggagat cctgatggcc agtgctacgg tggtgtcgct 840

tctgcccaag atgaggagcc cctggcctgc ccggacgagt tcgatgactt cgtcacctat 900

gaggccaacg aggtgacgga cagcgcgtac atgggctccg agagcaccta cagtgagtgt 960

gagaccttca cggacgagga caccagcacc ctggtgcacc ctgagctgca acctgaaggg 1020

gacgcagaca gtgccggcgg ctcggccgtg ccctctgagt gcctggacgc catggaggag 1080

cccgaccatg gtgccctgct gctgctccca ggcaggcctc acccccatgg ccagtctgtc 1140

atcacggtga tcgggggcga ggagcacttt gaggactacg gtgaaggcag tgaggcggag 1200

ctgtccccag agaccctatg caacgggcag ctgggctgca gtgaccccgc tttcctcacg 1260

cccagtccga caaagcggct ctccagcaag aaggtggcaa ggtacctgca ccagtcaggg 1320

gccctgacca tggaggccct ggaggaccct tcccccgagc tcatggaggg cccagaggag 1380

gacattgctg acaaggttgt cttcctggaa aggcgtgtgc tggagctgga aaaggacacg 1440

gcagccaccg gtgagcaaca cagccgcctg aggcaggaga acctgcagct ggtgcacaga 1500

gcaaacgccc tggaggagca gctgaaggag caggagctga gagcctgcga gatggtcctg 1560

gaagagaccc ggcgtcagaa ggagctcctg tgcaagatgg agagggagaa gagcattgag 1620

atcgagaacc tgcagaccag gctacagcaa ctggacgagg agaacagtga actccggtcc 1680

tgcacgccct gtctgaaggc caacattgag cgtctggagg aggagaagca gaagctgttg 1740

gatgagatag agtcgctgac gctgcggctc agtgaagagc aggagaacaa gaggagaatg 1800

ggggacaggc tgagtcacga gaggcaccag ttccagaggg acaaggaggc cacccaggag 1860

ctgatcgagg acctccgaaa gcagctggag cacctgcagc tcctcaagct ggaggccgag 1920

cagcggcggg gccgcagcag cagcatgggc ctgcaggagt accacagccg cgcccgggag 1980

agcgagctgg agcaggaggt ccgcaggctg aagcaggaca accgcaacct gaaggagcag 2040

aacgaggagc tgaacgggca gatcattacc ctcagcatcc agggcgccaa gagcctcttc 2100

tccacagcct tctctgagtc cctggctgca gagatcagct ccgtctcccg agatgagctc 2160

atggaggcga ttcagaagca ggaggagatc aacttccgcc tgcaggacta catcgacagg 2220

atcatcgtgg ccatcatgga gaccaacccg tccatcctgg aggtcaagta g 2271

<210> 24
<211> 1968
<212> DNA
<213> Homo sapiens

<400> 24
atgactgagt gcttcctgcc ccccaccagc agccccagtg aacaccgcag ggtggagcat 60

ggcagcgggc ttacccggac ccccagctct gaagagatca gccctactaa gtttcctgga 120

ttgtaccgca ctggcgagcc ctcacctccc catgacatcc tccatgagcc tcctgatgta 180

gtgtctgatg atgagaaaga tcatgggaag aaaaaaggga aatttaagaa aaaggaaaag 240

aggactgaag gctatgcagc ctttcaggaa gatagctctg gagatgaggc agaaagtcct 300
tctaaaatga agaggtccaa gggaatccat gttttcaaga agcccagctt ttctaaaaag 360
aaggaaaagg attttaaaat aaaagagaaa cccaaagaag aaaagcataa agaagaaaag 420
cacaaagaag aaaaacataa agagaagaag tcaaaagact tgacagcagc tgatgttgtt 480
aaacagtgga aggaaaagaa gaaaaagaaa aagccaattc aggagccaga ggtgcctcag 540
attgatgttc caaatctcaa acccattttt ggaattcctt tggctgatgc agtagagagg 600
accatgatgt atgatggcat tcggctgcca gccgttttcc gtgaatgtat agattacgta 660
gagaagtatg gcatgaagtg tgaaggcatc tacagagtat caggaattaa atcaaaggtg 720
gatgagctaa aagcagccta tgaccgggag gagtctacaa acttggaaga ctatgagcct 780
aacactgtag ccagtttgct gaagcagtat ttgcgagacc ttccagagaa tttgcttacc 840
aaagagctta tgcccagatt tgaagaggct tgtgggagga ccacggagac tgagaaagtg 900
caggaattcc agcgtttact caaagaactg ccagaatgta actatcttct gatttcttgg 960
ctcattgtgc acatggacca tgtcattgca aaggaactgg aaacaaaaat gaatatacag 1020
aacatttcta tagtgctcag cccaactgtg cagatcagca atcgagtcct gtatgtgttt 1080
ttcacacatg tgcaagaact ctttggaaat gtggtactaa agcaagtgat gaaacctctg 1140
cgatggtcta acatggccac gatgcccacg ctgccagaga cccaggcggg catcaaggag 1200
gagatcagga gacaggagtt tcttttgaat tgtttacatc gagatctgca gggtgggata 1260
aaggatttgt ctaaagaaga aagattatgg gaagtacaaa gaattttgac agccctcaaa 1320
agaaaactga gagaagctaa aagacaggag tgtgaaacca agattgcaca agagatagcc 1380
agtctttcaa aagaggatgt ttccaaagaa gagatgaatg aaaatgaaga agttataaat 1440
attctccttg ctcaggagaa tgagatcctg actgaacagg aggagctcct ggccatggag 1500
cagtttctgc gccggcagat tgcctcagaa aaagaagaga ttgaacgcct cagagctgag 1560
attgctgaaa ttcagagtcg ccagcagcac ggccgaagtg agactgagga gtactcctcc 1620
gagagcgaga gcgagagtga ggatgaggag gagctgcaga tcattctgga agacttacag 1680
agacagaacg aagagctgga aataaagaac aatcatttga atcaagcaat tcatgaggag 1740
cgcgaggcca tcatcgagct gcgcgtgcag ctgcggctgc tccagatgca gcgagccaag 1800
gccgagcagc aggcgcagga ggacgaggag cctgagtggc gcgggggtgc cgtccagccg 1860
cccagagacg gcgtccttga gccaaaagca gctaaagagc agccaaaggc aggcaaggag 1920
ccggcaaagc catcgcccag cagggatagg aaggagacgt ccatctga 1968

<210> 25
<211> 651
<212> DNA
<213> Homo sapiens

<400> 25
atggctgcgc agggagagcc ccaggtccag ttcaaacttg tattggttgg tgatggtggt 60

actggaaaaa cgaccttcgt gaaacgtcat ttgactggtg aatttgagaa gaagtatgta 120

gccaccttgg gtgttgaggt tcatccccta gtgttccaca ccaacagagg acctattaag 180

ttcaatgtat gggacacagc cggccaggag aaattcggtg gactgagaga tggctattat 240

atccaagccc agtgtgccat cataatgttt gatgtaacat cgagagttac ttacaagaat 300

gtgcctaact ggcatagaga tctggtacga gtgtgtgaaa acatccccat tgtgttgtgt 360

ggcaacaaag tggatattaa ggacaggaaa gtgaaggcga aatccattgt cttccaccga 420

aagaagaatc ttcagtacta cgacatttct gccaaaagta actacaactt tgaaaagccc 480

ttcctctggc ttgctaggaa gctcattgga gaccctaact tggaatttgt tgccatgcct 540

gctctcgccc caccagaagt tgtcatggac ccagctttgg cagcacagta tgagcacgac 600

ttagaggttg ctcagacaac tgctctcccg gatgaggatg atgacctgtg a 651

<210> 26
<211> 1389
<212> DNA
<213> Homo sapiens

<400> 26
atggccagca acagcagctc ctgcccgaca cctgggggcg ggcacctcaa tgggtacccg 60

gtgcctccct acgccttctt cttcccccct atgctgggtg gactctcccc gccaggcgct 120

ctgaccactc tccagcacca gcttccagtt agtggatata gcacaccatc cccagccacc 180

attgagaccc agagcagcag ttctgaagag atagtgccca gccctccctc gccaccccct 240

ctaccccgca tctacaagcc ttgctttgtc tgtcaggaca agtcctcagg ctaccactat 300

ggggtcagcg cctgtgaggg ctgcaagggc ttcttccgcc gcagcatcca gaagaacatg 360

gtgtacacgt gtcaccggga caagaactgc atcatcaaca aggtgacccg gaaccgctgc 420

cagtactgcc gactgcagaa gtgctttgaa gtgggcatgt ccaaggagtc tgtgagaaac 480

gaccgaaaca agaagaagaa ggaggtgccc aagcccgagt gctctgagag ctacacgctg 540

acgccggagg tgggggagct cattgagaag gtgcgcaaag cgcaccagga aaccttccct 600

gccctctgcc agctgggcaa atacactacg aacaacagct cagaacaacg tgtctctctg 660

gacattgacc tctgggacaa gttcagtgaa ctctccacca agtgcatcat taagactgtg 720

gagttcgcca agcagctgcc cggcttcacc accctcacca tcgccgacca gatcaccctc 780

ctcaaggctg cctgcctgga catcctgatc ctgcggatct gcacgcggta cacgcccgag 840

caggacacca tgaccttctc ggacgggctg accctgaacc ggacccagat gcacaacgct 900

ggcttcggcc ccctcaccga cctggtcttt gccttcgcca accagctgct gcccctggag 960

atggatgatg cggagacggg gctgctcagc gccatctgcc tcatctgcgg agaccgccag 1020

gacctggagc agccggaccg ggtggacatg ctgcaggagc cgctgctgga ggcgctaaag 1080

gtctacgtgc ggaagcggag gcccagccgc ccccacatgt tccccaagat gctaatgaag 1140

attactgacc tgcgaagcat cagcgccaag ggggctgagc gggtgatcac gctgaagatg 1200

gagatcccgg gctccatgcc gcctctcatc caggaaatgt tggagaactc agagggcctg 1260

gacactctga gcggacagcc ggggggtggg gggcgggacg ggggtggcct ggcccccccg 1320

ccaggcagct gtagccccag cctcagcccc agctccaaca gaagcagccc ggccacccac 1380

tccccgtga 1389

<210> 27
<211> 1212
<212> DNA
<213> Homo sapiens

<400> 27
atgggcaaag tgtggaaaca gcagatgtac cctcagtacg ccacctacta ttacccccag 60

tatctgcaag ccaagcagtc tctggtccca gcccacccca tggcccctcc cagtcccagc 120

accaccagca gtaataacaa cagtagcagc agtagcaact caggatggga tcagctcagc 180

aaaacgaacc tctatatccg aggactgcct ccccacacca ccgaccagga cctggtgaag 240

ctctgtcaac catatgggaa aatagtctcc acaaaggcaa ttttggataa gacaacaaac 300

aaatgcaaag gttatggttt tgtcgacttt gacagccctg cagcagctca aaaagctgtg 360

tctgccctga aggccagtgg ggttcaagct caaatggcaa agcaacagga acaagatcct 420

accaacctct acatttctaa tttgccactc tccatggatg agcaagaact agaaaatatg 480

ctcaaaccat ttggacaagt tatttctaca aggatactac gtgattccag tggtacaagt 540

cgtggtgttg gctttgctag gatggaatca acagaaaaat gtgaagctgt tattggtcat 600

tttaatggaa aatttattaa gacaccacca ggagtttctg cccccacaga acctttattg 660

tgtaagtttg ctgatggagg acagaaaaag agacagaacc caaacaaata catccctaat 720

ggaagaccat ggcatagaga aggagaggct ggaatgacac ttacttacga cccaactaca 780

gctgctatac agaacggatt ttatccttca ccatacagta ttgctacaaa ccgaatgatc 840

actcaaactt ctattacacc ctatattgca tctcctgtat ctgcctacca ggtgcaaagt 900

ccttcgtgga tgcaacctca accatatatt ctacagcacc ctggtgccgt gttaactccc 960

tcaatggagc acaccatgtc actacagccc gcatcaatga tcagccctct ggcccagcag 1020

atgagtcatc tgtcactagg cagcaccgga acatacatgc ctgcaacgtc agctatgcaa 1080

ggagcctact tgccacagta tgcacatatg cagacgacag cggttcctgt tgaggaggca 1140

agtggtcaac agcaggtggc tgtcgagacg tctaatgacc attctccata tacctttcaa 1200

cctaataagt aa 1212

<210> 28
<211> 1143
<212> DNA
<213> Homo sapiens

<400> 28
atgttggtga taccccccgg actgagcgag gaagaggagg ctctgcagaa gaaattcaac 60

aagctcaaga aaaagaaaaa ggcattgctg gctctgaaga agcaaagtag cagcagcaca 120

accagccaag gtggtgtcaa acgctcacta tcagagcagc ctgtcatgga cacagccaca 180

gcaacagagc aggcaaagca gctggtgaag tcaggagcca tcagtgccat caaggctgag 240

accaagaact caggcttcaa gcgttctcga acccttgagg ggaagttaaa ggaccccgag 300

aagggaccag tccccacttt ccagccgttc cagaggagca tatctgctga tgatgacctg 360

caagagtcat ccagacgtcc ccagaggaaa tctctgtatg agagctttgt gtcttctagt 420

gatcgacttc gagaactagg accagatgga gaagaggcag agggcccagg ggctggtgat 480

ggtccccctc gaagctttga ctggggctat gaagaacgca gtggtgccca ctcctcagcc 540

tcccctcccc gaagccgcag ccgggaccgc agccatgaga ggaaccggga cagagaccga 600

gatcgggagc gggatcgaga ccgggatcga gacagagaca gagagcggga cagggatcgg 660

gatcgggatc gagatcgaga ccgggaacgg gacagggatc gggagcggga tcgagaccga 720

gaccgagagg gtcctttccg caggtcggat tcattccctg aacggcgagc ccctaggaaa 780

gggaatactc tctatgtata tggagaagac atgacaccca cccttctccg tggggccttc 840

tctccttttg gaaacatcat tgacctctcc atggacccac ccagaaactg tgccttcgtc 900

acctatgaaa agatggagtc agcagatcag gccgttgctg agctcaacgg gacccaggtg 960

gagtctgtac agctcaaagt caacatagcc cgaaaacagc ccatgctgga tgccgctact 1020

ggcaagtctg tctggggctc cctcgctgtc cagaacagcc ctaagggttg ccaccgggac 1080

aagaggaccc agattgtcta cagtgatgac gtctacaagg aaaaccttgt ggatggcttc 1140

tag 1143

<210> 29
<211> 1875
<212> DNA
<213> Homo sapiens

<400> 29
atggaaaact cagattccaa tgacaaagga agtggtgatc agtctgcagc acagcgcaga 60

agtcagatgg accgattgga tcgagaagaa gctttctatc aatttgtaaa taacctgagt 120

gaagaagatt ataggcttat gagagataac aatttgctag gcaccccagg tgaaagtact 180

gaggaagagt tgctgagacg actacagcaa attaaagaag gcccaccacc gcaaaactca 240

gatgaaaata gaggaggaga ctcttcagat gatgtgtcta atggtgactc tataatagac 300

tggcttaact ctgtcagaca aactggaaat acaacaagaa gtgggcaaag aggaaaccaa 360

tcttggagag cagtgagtcg gactaatcca aacagtggtg atttcagatt cagtttagag 420

ataaatgtta accgtaataa tgggagccaa aattcagaga atgaaaatga gccatctgca 480

agacgttcta gtggagaaaa tgtggaaaac aacagccaaa ggcaagtgga aaacccacga 540

tctgaatcaa catctgcaag gccatctaga tcagaacgaa attcaactga agcgttaaca 600

gaggtcccac ctaccagagg tcagaggagg gcaagaagca ggagcccaga ccatcggaga 660

accagagcaa gagctgaaag aagtaggtca cctctgcatc caatgagtga aattccacga 720

agatctcatc atagtatctc atctcagact tttgaacatc ctttggtaaa tgagacggag 780

ggaagttcta gaacccggca ccatgtgaca ttgaggcagc aaatatctgg gcctgagttg 840

ctaagtagag gtctttttgc agcttctgga acaagaaatg cttctcaagg agcaggttct 900

tcagacacag ctgccagtgg tgaatctaca ggatcaggac agagacctcc aaccatagtc 960

cttgatcttc aagtaagaag agttcgtcct ggagaatatc ggcagagaga tagcatagcc 1020

agcagaactc ggtctaggtc tcagacacca aacaacactg tcacctatga aagtgaacga 1080

ggaggtttta ggcgtacatt ttcacgttct gagcgggcag gtgtgagaac ctatgtcagt 1140

accatcagaa ttcccattcg tagaatctta aatactggtt taagtgagac tacatctgtt 1200

gcaattcaga ccatgttaag gcagataatg acaggttttg gtgagttaag ctattttatg 1260

tacagtgata gcgactcaga gcctactggc tcagtctcaa atcgaaatat ggaaagggca 1320

gagtcacgga gtggaagagg aggttctggt ggtggtagta gttctggttc cagttcgagt 1380

tccagttcca gttcgagttc cagttccagt tcaagttcca gttccagtcc tagttccagt 1440

tccggtggtg aaagttcaga aactagctca gatttatttg aaggcagtaa tgaaggaagc 1500

tcatcatcag gctcatcagg tgccaggcga gagggtcgac atagggcccc agtcacattt 1560

gatgaaagtg gctctttgcc cttccttagc ctggctcagt ttttcctctt aaatgaggat 1620

gatgatgacc aacctagagg actcaccaaa gaacagattg acaacttggc aatgagaagt 1680

tttggtgaaa atgatgcatt aaaaacctgt agtgtttgca ttacagaata tacagaaggc 1740

aacaaacttc gtaaactacc ttgttcccat gagtaccatg tccactgcat cgatcgctgg 1800

ttatctgaga attctacctg tcctatttgt cgcagagcag tcttagcttc tggtaacaga 1860

gaaagtgttg tgtaa 1875

<210> 30
<211> 348
<212> DNA
<213> Homo sapiens

<400> 30
atggtggccg caaagaagac gaaaaagtcg ctggagtcga tcaactctag gctccaactc 60

gttatgaaaa gtgggaagta cgtcctgggg tacaagcaga ctctgaagat gatcagacaa 120

ggcaaagcga aattggtcat tctcgctaac aactgcccag ctttgaggaa atctgaaata 180

gagtactatg ctatgttggc taaaactggt gtccatcact acagtggcaa taatattgaa 240

ctgggcacag catgcggaaa atactacaga gtgtgcacac tggctatcat tgatccaggt 300

gactctgaca tcattagaag catgccagaa cagactggtg aaaagtaa 348

<210> 31
<211> 378
<212> DNA
<213> Homo sapiens

<400> 31
atggctcccg caaagaaggg tggcgagaag aaaaagggcc gttctgccat caacgaagtg 60

gtaacccgag aatacaccat caacattcac aagcgcatcc atggagtggg cttcaagaag 120

cgtgcacctc gggcactcaa agagattcgg aaatttgcca tgaaggagat gggaactcca 180

gatgtgcgca ttgacaccag gctcaacaaa gctgtctggg ccaaaggaat aaggaatgtg 240

ccataccgaa tccgtgtgcg gctgtccaga aaacgtaatg aggatgaaga ttcaccaaat 300

aagctatata ctttggttac ctatgtacct gttaccactt tcaaaaatct acagacagtc 360

aatgtggatg agaactaa 378

<210> 32
<211> 1803
<212> DNA
<213> Homo sapiens

<400> 32
atgatggagg agcgtgccaa cctgatgcac atgatgaaac tcagcatcaa ggtgttgctc 60

cagtcggctc tgagcctggg ccgcagcctg gatgcggacc atgccccctt gcagcagttc 120

tttgtagtga tggagcactg cctcaaacat gggctgaaag ttaagaagag ttttattggc 180

caaaataaat cattctttgg tcctttggag ctggtggaga aactttgtcc agaagcatca 240

gatatagcga ctagtgtcag aaatcttcca gaattaaaga cagctgtggg aagaggccga 300

gcgtggcttt atcttgcact catgcaaaag aaactggcag attatctgaa agtgcttata 360

gacaataaac atctcttaag cgagttctat gagcctgagg ctttaatgat ggaggaagaa 420

gggatggtga ttgttggtct gctggtggga ctcaatgttc tcgatgccaa tctctgcttg 480

aaaggagaag acttggattc tcaggttgga gtaatagatt tttccctcta ccttaaggat 540

gtgcaggatc ttgatggtgg caaggagcat gaaagaatta ctgatgtcct tgatcaaaaa 600

aattatgtgg aagaacttaa ccggcacttg agctgcacag ttggggatct tcaaaccaag 660

atagatggct tggaaaagac taactcaaag cttcaagaag agctttcagc tgcaacagac 720

cgaatttgct cacttcaaga agaacagcag cagttaagag aacaaaatga attaattcga 780

gaaagaagtg aaaagagtgt agagataaca aaacaggata ccaaagttga gctggagact 840

tacaagcaaa ctcggcaagg tctggatgaa atgtacagtg atgtgtggaa gcagctaaaa 900

gaggagaaga aagtccggtt ggaactggaa aaagaactgg agttacaaat tggaatgaaa 960

accgaaatgg aaattgcaat gaagttactg gaaaaggaca cccacgagaa gcaggacaca 1020

ctagttgccc tccgccagca gctggaagaa gtcaaagcga ttaatttaca gatgtttcac 1080

aaagctcaga atgcagagag cagtttgcag cagaagaatg aagccatcac atcctttgaa 1140

ggaaaaacca accaagttat gtccagcatg aaacaaatgg aagaaaggtt gcagcactcg 1200

gagcgggcga ggcagggagc tgaggagcgg agccacaagc tgcagcagga gctgggcggg 1260

aggatcggcg ccctgcagct gcagctctcc cagctgcacg agcaatgctc aagcctggag 1320

aaagaattga aatcagaaaa agagcaaaga caggctcttc agcgcgaatt acagcacgag 1380

aaagacactt cctctctact caggatggag ctgcaacaag tggaaggact gaaaaaggag 1440

ttgcgggagc ttcaggacga gaaggcagag ctgcagaaga tctgcgagga gcaggaacaa 1500

gccctccagg aaatgggcct gcacctcagc cagtccaagc tgaagatgga agatataaaa 1560

gaagtgaacc aggcactgaa gggccacgcc tggctgaaag atgacgaagc gacacactgt 1620

aggcagtgtg agaaggagtt ctccatttcc cggagaaagc accactgccg gaactgtggc 1680

cacatcttct gcaacacctg ctccagcaac gagctggccc tgccctccta ccccaagccg 1740

gtgcgagtgt gcgacagctg ccacaccctg ctcctgcagc gctgctcctc cacggcctcc 1800

tga 1803

<210> 33
<211> 849
<212> DNA
<213> Homo sapiens

<400> 33
atggcgatga gcagcggcgg cagtggtggc ggcgtcccgg agcaggagga ttccgtgctg 60

ttccggcgcg gcacaggcca gagcgatgat tctgacattt gggatgatac agcactgata 120

aaagcatatg ataaagctgt ggcttcattt aagcatgctc taaagaatgg tgacatttgt 180

gaaacttcgg gtaaaccaaa aaccacacct aaaagaaaac ctgctaagaa gaataaaagc 240

caaaagaaga atactgcagc ttccttacaa cagtggaaag ttggggacaa atgttctgcc 300

atttggtcag aagacggttg catttaccca gctaccattg cttcaattga ttttaagaga 360

gaaacctgtg ttgtggttta cactggatat ggaaatagag aggagcaaaa tctgtccgat 420

ctactttccc caatctgtga agtagctaat aatatagaac agaatgctca agagaatgaa 480

aatgaaagcc aagtttcaac agatgaaagt gagaactcca ggtctcctgg aaataaatca 540

gataacatca agcccaaatc tgctccatgg aactcttttc tccctccacc accccccatg 600

ccagggccaa gactgggacc aggaaagcca ggtctaaaat tcaatggccc accaccgcca 660

ccgccaccac caccacccca cttactatca tgctggctgc ctccatttcc ttctggacca 720

ccaataattc ccccaccacc tcccatatgt ccagattctc ttgatgatgc tgatgctttg 780

ggaagtatgt taatttcatg gtacatgagt ggctatcata ctggctatta tatggaaatg 840

ctggcatag 849

<210> 34
<211> 2058
<212> DNA
<213> Homo sapiens

<400> 34
atggagcttt tgcggactat cacctaccag ccagccgcca gcaccaaaat gtgcgagcag 60

gcgctgggca agggttgcgg agcagactcg aagaagaagc ggccgccgca gccccccgag 120

gaatcgcagc cacctcagtc ccaggcgcaa gtgcccccgg cggcccctca ccaccatcac 180

caccattcgc actcggggcc ggagatctcg cggattatcg tcgaccccac gactgggaag 240

cgctactgcc ggggcaaagt gctgggaaag ggtggctttg caaaatgtta cgagatgaca 300

gatttgacaa ataacaaagt ctacgccgca aaaattattc ctcacagcag agtagctaaa 360

cctcatcaaa gggaaaagat tgacaaagaa atagagcttc acagaattct tcatcataag 420

catgtagtgc agttttacca ctacttcgag gacaaagaaa acatttacat tctcttggaa 480

tactgcagta gaaggtcaat ggctcatatt ttgaaagcaa gaaaggtgtt gacagagcca 540

gaagttcgat actacctcag gcagattgtg tctggactga aataccttca tgaacaagaa 600

atcttgcaca gagatctcaa actagggaac ttttttatta atgaagccat ggaactaaaa 660

gttggggact tcggtctggc agccaggcta gaacccttgg aacacagaag gagaacgata 720

tgtggtaccc caaattatct ctctcctgaa gtcctcaaca aacaaggaca tggctgtgaa 780

tcagacattt gggccctggg ctgtgtaatg tatacaatgt tactagggag gcccccattt 840

gaaactacaa atctcaaaga aacttatagg tgcataaggg aagcaaggta tacaatgccg 900

tcctcattgc tggctcctgc caagcactta attgctagta tgttgtccaa aaacccagag 960

gatcgtccca gtttggatga catcattcga catgactttt ttttgcaggg cttcactccg 1020

gacagactgt cttctagctg ttgtcataca gttccagatt tccacttatc aagcccagct 1080

aagaatttct ttaagaaagc agctgctgct ctttttggtg gcaaaaaaga caaagcaaga 1140

tatattgaca cacataatag agtgtctaaa gaagatgaag acatctacaa gcttaggcat 1200

gatttgaaaa agacttcaat aactcagcaa cccagcaaac acaggacaga tgaggagctc 1260

cagccaccta ccaccacagt tgccaggtct ggaacacccg cagtagaaaa caagcagcag 1320

attggggatg ctattcggat gatagtcaga gggactcttg gcagctgtag cagcagcagt 1380

gaatgccttg aagacagtac catgggaagt gttgcagaca cagtggcaag ggttcttcgg 1440

ggatgtctgg aaaacatgcc ggaagctgat tgcattccca aagagcagct gagcacatca 1500

tttcagtggg tcaccaaatg ggttgattac tctaacaaat atggctttgg gtaccagctc 1560

tcagaccaca ccgtcggtgt ccttttcaac aatggtgctc acatgagcct ccttccagac 1620

aaaaaaacag ttcactatta cgcagagctt ggccaatgct cagttttccc agcaacagat 1680

gctcctgagc aatttattag tcaagtgacg gtgctgaaat acttttctca ttacatggag 1740

gagaacctca tggatggtgg agatctgcct agtgttactg atattcgaag acctcggctc 1800

tacctccttc agtggctaaa atctgataag gccctaatga tgctctttaa tgatggcacc 1860

tttcaggtga atttctacca tgatcataca aaaatcatca tctgtagcca aaatgaagaa 1920

taccttctca cctacatcaa tgaggatagg atatctacaa ctttcaggct gacaactctg 1980

ctgatgtctg gctgttcatc agaattaaaa aatcgaatgg aatatgccct gaacatgctc 2040

ttacaaagat gtaactga 2058

<210> 35
<211> 1968
<212> DNA
<213> Homo sapiens

<400> 35
atggagcgga aagtgcttgc gctccaggcc cgaaagaaaa ggaccaaggc caagaaggac 60

aaagcccaaa ggaaatctga aactcagcac cgaggctctg ctccccactc tgagagtgat 120

ctaccagagc aggaagagga gattctggga tctgatgatg atgagcaaga agatcctaat 180
gattattgta aaggaggtta tcatcttgtg aaaattggag atctattcaa tgggagatac 240
catgtgatcc gaaagttagg ctggggacac ttttcaacag tatggttatc atgggatatt 300
caggggaaga aatttgtggc aatgaaagta gttaaaagtg ctgaacatta cactgaaaca 360
gcactagatg aaatccggtt gctgaagtca gttcgcaatt cagaccctaa tgatccaaat 420
agagaaatgg ttgttcaact actagatgac tttaaaatat caggagttaa tggaacacat 480
atctgcatgg tatttgaagt tttggggcat catctgctca agtggatcat caaatccaat 540
tatcaggggc ttccactgcc ttgtgtcaaa aaaattattc agcaagtgtt acagggtctt 600
gattatttac ataccaagtg ccgtatcacc cacactgaca ttaaaccaga gaacatctta 660
ttgtcagtga atgagcagta cattcggagg ctggctgcag aagcaacaga atggcagcga 720
tctggagctc ctccgccttc cggatctgca gtcagtactg ctccccagcc taaaccagct 780
gacaaaatgt caaagaataa gaagaagaaa ttgaagaaga agcagaagcg ccaggcagaa 840
ttactagaga agcgaatgca ggaaattgag gaaatggaga aagagtcggg ccctgggcaa 900
aaaagaccaa acaagcaaga agaatcagag agtcctgttg aaagaccctt gaaagagaac 960
ccacctaata aaatgaccca agaaaaactt gaagagtcaa gtaccattgg ccaggatcaa 1020
acgcttatgg aacgtgatac agagggtggt gcagcagaaa ttaattgcaa tggagtgatt 1080
gaagtcatta attatactca gaacagtaat aatgaaacat tgagacataa agaggatcta 1140
cataatgcta atgactgtga tgtccaaaat ttgaatcagg aatctagttt cctaagctcc 1200
caaaatggag acagcagcac atctcaagaa acagactctt gtacacctat aacatctgag 1260
gtgtcagaca ccatggtgtg ccagtcttcc tcaactgtag gtcagtcatt cagtgaacaa 1320
cacattagcc aacttcaaga aagcattcgg gcagagatac cctgtgaaga tgaacaagag 1380
caagaacata acggaccact ggacaacaaa ggaaaatcca cggctggaaa ttttcttgtt 1440
aatccccttg agccaaaaaa tgcagaaaag ctcaaggtga agattgctga ccttggaaat 1500
gcttgttggg tgcacaaaca tttcactgaa gatattcaaa caaggcaata tcgttccttg 1560
gaagttctaa tcggatctgg ctataatacc cctgctgaca tttggagcac ggcatgcatg 1620
gcctttgaac tggccacagg tgactatttg tttgaacctc attcagggga agagtacact 1680
cgagatgaag atcacattgc attgatcata gaacttctgg ggaaggtgcc tcgcaagctc 1740
attgtggcag gaaaatattc caaggaattt ttcaccaaaa aaggtgacct gaaacatatc 1800
acgaagctga aaccttgggg cctttttgag gttctagtgg agaagtatga gtggtcgcag 1860
gaagaggcag ctggcttcac agatttctta ctgcccatgt tggagctgat ccctgagaag 1920
agagccactg ccgccgagtg tctccggcac ccttggctta actcctaa 1968

<210> 36
<211> 1617
<212> DNA
<213> Homo sapiens

<400> 36
atggaggaat ctgtaaacca aatgcagcca ctgaatgaga agcagatagc caattctcag 60
gatggatatg tatggcaagt cactgacatg aatcgactac accggttctt atgtttcggt 120
tctgaaggtg ggacttatta tatcaaagaa cagaagttgg gccttgaaaa tgctgaagct 180
ttaattagat tgattgaaga tggcagagga tgtgaagtga tacaagaaat aaagtcattt 240
agtcaagaag gcagaaccac aaagcaagag cctatgctct ttgcacttgc catttgttcc 300
cagtgctccg acataagcac aaaacaagca gcatttaaag ctgtttctga agtttgtcgc 360
attcctaccc atctctttac ttttatccag tttaagaaag atctgaagga aagcatgaaa 420
tgtggcatgt ggggtcgtgc cctccggaag gctatagcgg actggtacaa tgagaaaggt 480
ggcatggccc ttgctctggc agttacaaaa tataaacaga gaaatggctg gtctcacaaa 540
gatctattaa gattgtcaca tcttaaacct tccagtgaag gacttgcaat tgtgaccaaa 600
tatattacaa agggctggaa agaagttcat gaattgtata aagaaaaagc actctctgtg 660
gagactgaaa aattattaaa gtatctggag gctgtagaga aagtgaagcg cacaagagat 720
gagctagaag tcattcatct aatagaagaa catagattag ttagagaaca tcttttaaca 780
aatcacttaa agtctaaaga ggtatggaag gctttgttac aagaaatgcc gcttactgca 840
ttactaagga atctaggaaa gatgactgct aattcagtac ttgaaccagg aaattcagaa 900
gtatctttag tatgtgaaaa actgtgtaat gaaaaactat taaaaaaggc tcgtatacat 960
ccatttcata ttttgatcgc attagaaact tacaagacag gtcatggtct cagagggaaa 1020
ctgaagtggc gccctgatga agaaattttg aaagcattgg atgctgcttt ttataaaaca 1080
tttaagacag ttgaaccaac tggaaaacgt ttcttactag ctgttgatgt cagtgcttct 1140
atgaaccaaa gagttttggg tagtatactc aacgctagta cagttgctgc agcaatgtgc 1200
atggttgtca cacgaacaga aaaagattct tatgtagttg ctttttccga tgaaatggta 1260
ccatgtccag tgactacaga tatgacctta caacaggttt taatggctat gagtcagatc 1320
ccagcaggtg gaactgattg ctctcttcca atgatctggg ctcagaagac aaacacacct 1380
gctgatgtct tcattgtatt cactgataat gagacctttg ctggaggtgt ccatcctgct 1440
attgctctga gggagtatcg aaagaaaatg gatattccag ctaaattgat tgtttgtgga 1500
atgacatcaa atggtttcac cattgcagac ccagatgata gaggcatgtt ggatatgtgc 1560
ggctttgata ctggagctct ggatgtaatt cgaaatttca cattagatat gatttaa 1617

<210> 37
<211> 360
<212> DNA
<213> Homo sapiens

<400> 37
atgacccagc agggcgcggc gctgcagaac tacaacaacg agctggtcaa gtgcatagag 60
gagctgtgcc agaagcggga ggagctgtgc cggcagatcc aggaggagga ggacgagaag 120
cagcggctgc agaatgaggt gaggcagctg acagagaagc tggcccgcgt caacgagaac 180

ctggcacgca agattgcctc tcgcaacgag ttcgaccgga ccatcgcgga gacggaggcc 240

gcctacctca agatcctgga gagctcccag actttgctca gcgttctcaa gagggaagct 300

gggaacctga ccaaggctac agccccagac cagaaaagta gcggcggcag ggacagctga 360

<210> 38
<211> 1734
<212> DNA
<213> Homo sapiens

<400> 38
atgtctcaag ttcaagtgca agttcagaac ccatctgctg ctctctcagg gagccaaata 60

ctgaacaaga accagtctct tctctcacag cctttgatga gtattccttc tactactagc 120

tctctgccct ctgaaaatgc aggtagaccc attcaaaact ctgctttacc ctctgcatct 180

attacatcca ccagtgcagc tgcagaaagc ataaccccta ctgtagaact aaatgcactg 240

tgcatgaaac ttggaaaaaa accaatgtat aagcctgttg acccttactc tcggatgcag 300

tccacctata actacaacat gagaggaggt gcttatcccc cgaggtactt ttacccattt 360

ccagttccac ctttacttta tcaagtggaa ctttctgtgg gaggacagca atttaatggc 420

aaaggaaaga caagacaggc tgcgaaacac gatgctgctg ccaaagcgtt gaggatcctg 480

cagaatgagc ccctgccaga gaggctggag gtgaatggaa gagaatccga agaagaaaat 540

ctcaataaat ctgaaataag tcaagtgttt gagattgcac ttaaacggaa cttgcctgtg 600

aatttcgagg tggcccggga gagtggccca ccccacatga agaactttgt gaccaaggtt 660

tcggttgggg agtttgtggg ggaaggtgaa gggaaaagca agaagatttc aaagaaaaat 720

gccgccatag ctgttcttga ggagctgaag aagttaccgc ccctgcctgc agttgaacga 780

gtaaagccta gaatcaaaaa gaaaacaaaa cccatagtca agccacagac aagcccagaa 840

tatggccagg ggatcaatcc gattagccga ctggcccaga tccagcaggc aaaaaaggag 900

aaggagccag agtacacgct cctcacagag cgaggcctcc cgcgccgcag ggagtttgtg 960

atgcaggtga aggttggaaa ccacactgca gaaggaacgg gcaccaacaa gaaggtggcc 1020

aagcgcaatg cagccgagaa catgctggag atccttggtt tcaaagtccc gcaggcgcag 1080

cccaccaaac ccgcactcaa gtcagaggag aagacaccca taaagaaacc aggggatgga 1140

agaaaagtaa ccttttttga acctggctct ggggatgaaa atgggactag taataaagag 1200

gatgagttca ggatgcctta tctaagtcat cagcagctgc ctgctggaat tcttcccatg 1260

gtgcccgagg tcgcccaggc tgtaggagtt agtcaaggac atcacaccaa agattttacc 1320

agggcagctc cgaatcctgc caaggccacg gtaactgcca tgatagcccg agagttgttg 1380

tatgggggca cctcgcccac agccgagacc attttaaaga ataacatctc ttcaggccac 1440

gtaccccatg gacctctcac gagaccctct gagcaactgg actatctttc cagagtccag 1500

ggattccagg ttgaatacaa agacttcccc aaaaacaaca agaacgaatt tgtatctctt 1560

atcaattgct cctctcagcc acctctgatc agccatggta tcggcaagga tgtggagtcc 1620

tgccatgata tggctgcgct gaacatctta aagttgctgt ctgagttgga ccaacaaagt 1680

acagagatgc caagaacagg aaacggacca atgtctgtgt gtgggaggtg ctga 1734

<210> 39
<211> 1302
<212> DNA
<213> Homo sapiens

<400> 39
atggaggtgg tggacccgca gcagctgggc atgttcacgg agggcgagct gatgtcggtg 60

ggtatggaca cgttcatcca ccgcatcgac tccaccgagg tcatctacca gccgcgccgc 120

aagcgggcca agctcatcgg caagtacctg atgggggacc tgctggggga aggctcttac 180

ggcaaggtga aggaggtgct ggactcggag acgctgtgca ggagggccgt caagatcctc 240

aagaagaaga agttgcgaag gatccccaac ggggaggcca acgtgaagaa ggaaattcaa 300

ctactgagga ggttacggca caaaaatgtc atccagctgg tggatgtgtt atacaacgaa 360

gagaagcaga aaatgtatat ggtgatggag tactgcgtgt gtggcatgca ggaaatgctg 420

gacagcgtgc cggagaagcg tttcccagtg tgccaggccc acgggtactt ctgtcagctg 480

attgacggcc tggagtacct gcatagccag ggcattgtgc acaaggacat caagccgggg 540

aacctgctgc tcaccaccgg tggcaccctc aaaatctccg acctgggcgt ggccgaggca 600

ctgcacccgt tcgcggcgga cgacacctgc cggaccagcc agggctcccc ggctttccag 660

ccgcccgaga ttgccaacgg cctggacacc ttctccggct tcaaggtgga catctggtcg 720

gctggggtca ccctctacaa catcaccacg ggtctgtacc ccttcgaagg ggacaacatc 780

tacaagttgt ttgagaacat cgggaagggg agctacgcca tcccgggcga ctgtggcccc 840

ccgctctctg acctgctgaa agggatgctt gagtacgaac cggccaagag gttctccatc 900

cggcagatcc ggcagcacag ctggttccgg aagaaacatc ctccggctga agcaccagtg 960

cccatcccac cgagcccaga caccaaggac cggtggcgca gcatgactgt ggtgccgtac 1020

ttggaggacc tgcacggcgc ggacgaggac gaggacctct tcgacatcga ggatgacatc 1080

atctacactc aggacttcac ggtgcccgga caggtcccag aagaggaggc cagtcacaat 1140

ggacagcgcc ggggcctccc caaggccgtg tgtatgaacg gcacagaggc ggcgcagctg 1200

agcaccaaat ccagggcgga gggccgggcc cccaaccctg cccgcaaggc ctgctccgcc 1260

agcagcaaga tccgccggct gtcggcctgc aagcagcagt ga 1302

<210> 40
<211> 1482
<212> DNA
<213> Homo sapiens

<400> 40
atggactttc tcctggggaa cccgttcagc tctccagtgg gacagcgcat cgagaaagcc 60

acagatggct ccctgcagag cgaggactgg gccctcaaca tggagatctg cgacatcatc 120

aacgagacgg aggaaggtcc caaagatgcc ctccgagcag taaagaagag aatcgtgggg 180

aataagaact tccacgaggt gatgctggct ctcacagtct tagaaacctg tgtcaagaac 240

tgcgggcacc gcttccacgt gctggtggcc agccaggact tcgtggagag tgtgctggtg 300
aggaccatcc tgcccaagaa caacccaccc accatcgtgc atgacaaagt gctcaacctc 360
atccagtcct gggctgacgc gttccgcagc tcgcccgatc tgacaggtgt ggtcaccatc 420
tatgaggacc tgcggaggaa aggcctggag ttccccatga ctgacctgga catgctgtca 480
cccatccaca caccccagag gaccgtgttc aactcagaga cacaatcagg acaggattct 540
gtgggcactg actccagcca gcaagaggac tctggccagc atgctgcccc tctgcccgcc 600
ccgcccatac tctccggtga cacgcccata gcaccaaccc cggaacagat tgggaagctg 660
cgcagtgagc tggagatggt gagtgggaac gtgagggtga tgtcggagat gctgacggag 720
ctggtgccca cccaggccga gcccgcagac ctggagctgc tgcaggagct caaccgcacg 780
tgccgagcca tgcagcagcg ggtcctggag ctcatccctc agatcgccaa tgagcagctg 840
acagaggagc tgctcatcgt caatgacaat ctcaacaatg tgttcctgcg ccatgaacgg 900
tttgaacggt tccgaacagg ccagaccacc aaggccccaa gtgaggccga gccggcagct 960
gacctgatcg acatgggccc tgacccagca gccaccggca acctctcatc ccagctggca 1020
ggaatgaacc tgggctccag cagtgtgaga gctggcctgc agtctctgga ggcctctggt 1080
cgactggaag atgagtttga catgtttgcg ctgacacggg gcagctcact ggctgaccaa 1140
cggaaagagg taaaatacga agccccccaa gcaacagacg gcctggctgg agccctggac 1200
gcccggcagc agagcactgg cgcgatccca gtcacccagg cctgcctcat ggaggacatc 1260
gagcagtggc tgtccactga cgtgggtaat gatgcggaag agcctaaggg ggtcaccagc 1320
gaaggtaaat ttgacaaatt cctggaagaa cgggccaaag ccgcggaccg attgcccaac 1380
ctctccagcc cctcagctga ggggcccccg ggtcccccat ctggcccagc gccccggaag 1440
aagacccagg agaaagatga tgacatgctg tttgccttat ga 1482

<210> 41
<211> 1008
<212> DNA
<213> Homo sapiens

<400> 41
atggcggcgg gggcggctga ggcagctgta gcggccgtgg aggaggtcgg ctcagccggg 60
cagtttgagg agctgctgcg cctcaaagcc aagtccctcc ttgtggtcca tttctgggca 120
ccatgggctc cacagtgtgc acagatgaac gaagttatgg cagagttagc taaagaactc 180
cctcaagttt catttgtgaa gttggaagct gaaggtgttc ctgaagtatc tgaaaaatat 240
gaaattagct ctgttcccac ttttctgttt ttcaagaatt ctcagaaaat cgaccgatta 300
gatggtgcac atgccccaga gttgaccaaa aaagttcagc gacatgcatc tagtggctcc 360
ttcctaccca gcgctaatga acatcttaaa gaagatctca accttcgctt gaagaaattg 420
actcatgctg ccccctgcat gctgtttatg aaaggaactc ctcaagaacc acgctgtggt 480
ttcagcaagc agatggtgga aattcttcac aaacataata ttcagtttag cagttttgat 540

atcttctcag atgaagaggt tcgacaggga ctcaaagcct attccagttg gcctacctat 600

cctcagctct atgtttctgg agagctcata ggaggacttg atataattaa ggagctagaa 660

gcatctgaag aactagatac aatttgtccc aaagctccca aattagagga aaggctcaaa 720

gtgctgacaa ataaagcttc tgtgatgctc tttatgaaag gaaacaaaca ggaagcaaaa 780

tgtggattca gcaaacaaat tctggaaata ctaaatagta ctggtgttga atatgaaaca 840

ttcgatatat tggaggatga agaagttcgg caaggattaa aagcttactc aaattggcca 900

acataccctc agctgtatgt gaaaggggag ctggtgggag gattggatat tgtgaaggaa 960

ctgaaagaaa atggtgaatt gctgcctata ctgagaggag aaaattaa 1008

<210> 42
<211> 1500
<212> DNA
<213> Homo sapiens

<400> 42
atgaacggcc ctgaagatct tcccaagtcc tatgactatg accttatcat cattggaggt 60

ggctcaggag gtctggcagc tgctaaggag gcagcccaat atggcaagaa ggtgatggtc 120

ctggactttg tcactcccac ccctcttgga actagatggg gtcttggagg aacatgtgtg 180

aatgtgggtt gcatacctaa aaaactgatg catcaagcag ctttgttagg acaagccctg 240

caagactctc gaaattatgg atggaaagtc gaggagacag ttaagcatga ttgggacaga 300

atgatagaag ctgtacagaa tcacattggc tctttgaatt ggggctaccg agtagctctg 360

cgggagaaaa aagtcgtcta tgagaatgct tatgggcaat ttattggtcc tcacaggatt 420

aaggcaacaa ataataaagg caaagaaaaa atttattcag cagagagatt tctcattgcc 480

actggtgaaa gaccacgtta cttgggcatc cctggtgaca aagaatactg catcagcagt 540

gatgatcttt tctccttgcc ttactgcccg ggtaagaccc tggttgttgg agcatcctat 600

gtcgctttgg agtgcgctgg atttcttgct ggtattggtt tagacgtcac tgttatggtt 660

aggtccattc ttcttagagg atttgaccag gacatggcca acaaaattgg tgaacacatg 720

gaagaacatg gcatcaagtt tataagacag ttcgtaccaa ttaaagttga acaaattgaa 780

gcagggacac caggccgact cagagtagta gctcagtcca ccaatagtga ggaaatcatt 840

gaaggagaat ataatacggt gatgctggca ataggaagag atgcttgcac aagaaaaatt 900

ggcttagaaa ccgtaggggt gaagataaat gaaaagactg gaaaaatacc tgtcacagat 960

gaagaacaga ccaatgtgcc ttacatctat gccattggcg atatattgga ggataaggtg 1020

gagctcaccc cagttgcaat ccaggcagga agattgctgg ctcagaggct ctatgcaggt 1080

tccactgtca agtgtgacta tgaaaatgtt ccaaccactg tatttactcc tttggaatat 1140

ggtgcttgtg gcctttctga ggagaaagct gtggagaagt ttggggaaga aaatattgag 1200

gtttaccata gttacttttg gccattggaa tggacgattc cgtcaagaga taacaacaaa 1260

tgttatgcaa aaataatctg taatactaaa gacaatgaac gtgttgtggg ctttcacgta 1320

ctgggtccaa atgctggaga agttacacaa ggctttgcag ctgcgctcaa atgtggactg 1380

accaaaaagc agctggacag cacaattgga atccaccctg tctgtgcaga ggtattcaca 1440
acattgtctg tgaccaagcg ctctggggca agcatcctcc aggctggctg ctgaggttaa 1500

<210> 43
<211> 3201
<212> DNA
<213> Homo sapiens

<400> 43
atgccagtgt ttcatacgcg cacgatcgag agcatcctgg agccggtggc acagcagatc 60
tcccacctgg tgataatgca cgaggagggc gaggtggacg gcaaagccat tcctgacctc 120
accgcgcccg tggccgccgt gcaggcggcc gtcagcaacc tcgtccgggt tggaaaagag 180
actgttcaaa ccactgagga tcagattttg aagagagata tgccaccagc atttattaag 240
gttgagaatg cttgcaccaa gcttgtccag gcagctcaga tgcttcagtc agacccttac 300
tcagtgcctg ctcgagatta tctaattgat gggtcaaggg gcatcctctc tggaacatca 360
gacctgctcc ttaccttcga tgaggctgag gtccgtaaaa ttattagagt ttgcaaagga 420
attttggaat atcttacagt ggcagaggtg gtggagacta tggaagattt ggtcacttac 480
acaaagaatc ttgggccagg aatgactaag atggccaaga tgattgacga gagacagcag 540
gagctcactc accaggagca ccgagtgatg ttggtgaact cgatgaacac cgtgaaagag 600
ttgctgccag ttctcatttc agctatgaag atttttgtaa caactaaaaa ctcaaaaaac 660
caaggcatag aggaagcttt aaaaaatcgc aattttactc tagaaaaaat gagtgctgaa 720
attaatgaga taattcgtgt gttacaactc acctcttggg atgaagatgc ctgggccagc 780
aaggacactg aagccatgaa gagagcattg gcctccatag actccaaact gaaccaggcc 840
aaaggttggc tccgtgaccc tagtgcctcc ccaggggatg ctggtgagca ggccatcaga 900
cagatcttag atgaagctgg aaaagttggt gaactctgtg caggcaaaga acgcagggag 960
attctgggaa cttgcaaaat gctagggcag atgactgatc aagtggctga cctccgtgcc 1020
agaggacaag gatcctcacc ggtggccatg cagaaagctc agcaggtatc tcagggtctg 1080
gatgtgctca cagcaaaagt ggaaaatgca gctcgcaagc tggaagccat gaccaactca 1140
aagcagagca ttgcaaagaa gatcgatgct gctcagaact ggcttgcaga tccaaatggt 1200
ggaccggaag gagaagagca gattcgaggt gctttggctg aagctcggaa aatagcagaa 1260
ttatgtgatg atcctaaaga aagagatgac attctacgtt cccttgggga aatatctgct 1320
ctgacttcta aattagcaga tctacgaaga caggggaaag gagattctcc agaggctcga 1380
gccttggcca aacaggtggc cacggccctg cagaacctgc agaccaaaac caaccgggct 1440
gtggccaaca gcagaccggc caaagcagct gtacaccttg agggcaagat tgagcaagca 1500
cagcggtgga ttgataatcc cacagtggat gaccgtggag tcggtcaggc tgccatccgg 1560
gggcttgtgg ccgaagggca tcgtctggct aatgttatga tggggcctta tcggcaagat 1620
cttctcgcca agtgtgaccg agtggaccag ctgacagccc agctggctga cctggctgcc 1680

agaggggaag gggagagtcc tcaggcacga gcacttgcat ctcagctcca agactcctta 1740
aaggatctaa aagctcggat gcaggaggcc atgactcagg aagtgtcaga tgttttcagc 1800
gataccacaa ctcccatcaa gctgttggca gtggcagcca cggcgcctcc tgatgcgcct 1860
aacagggaag aggtatttga tgagagggca gctaactttg aaaaccattc aggaaagctt 1920
ggtgctacgg ccgagaaggc ggctgcggtt ggtactgcta ataaatcaac agtggaaggc 1980
attcaggcct cagtgaagac ggcccgagaa ctcacacccc aggtggtctc ggctgctcgt 2040
atcttactta ggaaccctgg aaatcaagct gcttatgaac attttgagac catgaagaac 2100
cagtggatcg ataatgttga aaaaatgaca gggctggtgg acgaagccat tgataccaaa 2160
tctctgttgg atgcttcaga agaagcaatt aaaaaagacc tggacaagtg caaggtagct 2220
atggccaaca ttcagcctca gatgctggtt gctggggcaa ccagtattgc tcgtcgggcc 2280
aaccggatcc tgctggtggc taagagggag gtggagaatt ccgaggatcc caagttccgt 2340
gaggctgtga aagctgcctc tgatgaattg agcaaaacca tctccccgat ggtgatggat 2400
gcaaaagctg tggctggaaa catttccgac cctggactgc aaaagagctt cctggactca 2460
ggatatcgga tcctgggagc tgtggccaag gtcagagaag ccttccaacc tcaggagcct 2520
gacttcccgc cgcctccacc agaccttgaa caactccgac taacagatga gcttgctcct 2580
cccaaaccac ctctgcctga aggtgaggtc cctccaccta ggcctccacc accagaggaa 2640
aaggatgaag agttccctga gcagaaggcc ggggaggtga ttaaccagcc aatgatgatg 2700
gctgccagac agctccatga tgaagctcgc aaatggtcca gcaagggcaa tgacatcatt 2760
gcagcagcca agcgcatggc tctgctgatg gctgagatgt ctcggctggt aagagggggc 2820
agtggtacca agcgggcact cattcagtgt gccaaggaca tcgccaaggc ctcagatgag 2880
gtgactcggt tggccaagga ggttgccaag cagtgcacag ataaacggat tagaaccaac 2940
ctcttacagg tatgtgagcg aatcccaacc ataagcaccc agctcaaaat cctgtccaca 3000
gtgaaggcca ccatgctggg ccggaccaac atcagtgatg aggagtctga gcaggccaca 3060
gagatgctgg ttcacaatgc ccagaacctc atgcagtctg tgaaggagac tgtgcgggaa 3120
gctgaagctg cttcaatcaa aattcgaaca gatgctggat ttacactgcg ctgggttaga 3180
aagactccct ggtaccagta g 3201

<210> 44
<211> 600
<212> DNA
<213> Homo sapiens

<400> 44
atggcgtcgg gcagcgggac aaaaaacttg gactttcgcc gaaagtggga caaagatgaa 60
tatgagaaac tcgccgagaa gaggctcacg gaagagagag aaaagaaaga tggaaaacca 120
gtgcagcctg tcaagcgaga gcttttacgg catagggact acaaggtgga cttggaatcc 180
aagcttggga agacaattgt cattaccaag acaacccctc aatctgagat gggaggatat 240
tactgcaatg tctgtgactg tgtggtgaag gactccatca actttctgga tcacattaat 300

ggaaagaaac atcagagaaa cctgggcatg tctatgcgtg tggaacgttc caccctggat 360

caggtgaaga aacgttttga ggtcaacaag aagaagatgg aagagaagca gaaggattat 420

gattttgagg aaaggatgaa ggagctcaga gaagaggagg aaaaggccaa agcgtacaag 480

aaagagaaac agaaggagaa gaaaaggagg gctgaggagg acttgacatt tgaggaggac 540

gatgagatgg cagctgtgat gggcttctct ggctttggtt ccaccaagaa gagttactga 600

<210> 45
<211> 1422
<212> DNA
<213> Homo sapiens

<400> 45
atgatgacca aggtactagg catggcccca gttctgggcc ctaggcctcc acaggagcag 60

gtggggcctc tgatggtaaa agtcgaggag aaagaagaga aaggcaagta ccttcctagc 120

ctggagatgt tccgccagcg cttcaggcag tttgggtacc atgatacccc tggaccccga 180

gaggccctga gccaactccg ggtgctctgc tgtgagtggc tgaggcccga gatccacacc 240

aaggagcaga tcctggagct actggtgctg gagcagttcc tgaccatcct gccccaggag 300

ctccaggcct gggtgcagga gcattgcccg gagagcgctg aagaggctgt cactctcctc 360

gaagatctgg agcgggaact ggatgagcca ggacaccagg tctcaactcc tccaaacgaa 420

cagaaaccgg tgtgggagaa gatatcctcc tcaggaactg caaaggaatc cccgagcagc 480

atgcagccac agcccttgga gaccagtcac aaatacgagt cttgggggcc cctgtacatc 540

caagagtctg gtgaggagca ggagttcgct caagatccaa gaaaggtccg agattgcaga 600

ttgagtaccc agcacgagga atcagcagat gagcagaaag gttctgaagc agaggggctc 660

aaaggggata taatttctgt gattatcgcc aataaacctg aggccagctt agagaggcag 720

tgcgtaaacc ttgaaaatga aaaaggaaca aaaccccctc ttcaagaggc aggctccaag 780

aaaggtagag aatcagttcc tactaaacct accccaggag agagacgtta tatatgtgct 840

gaatgtggca aagcctttag taatagctca aatctcacca aacacaggag aacacacact 900

ggggagaaac cttacgtgtg caccaagtgt gggaaagctt tcagccacag ctcaaacctc 960

accctccact acagaacaca cttggtggac cggccctatg actgtaagtg tggaaaagct 1020

tttgggcaga gctcagacct tcttaaacat cagagaatgc acacagaaga ggcgccatat 1080

cagtgcaaag attgtggcaa ggctttcagc gggaaaggca gcctcattcg tcactatcgg 1140

atccacactg gggagaagcc ttatcagtgt aacgaatgtg ggaagagctt cagtcagcat 1200

gcgggcctca gctcccacca gagactccac accggagaga agccatataa gtgtaaggag 1260

tgtgggaaag ccttcaacca cagctccaac ttcaataaac accacagaat ccacaccggg 1320

gaaaagccct actggtgtca tcactgtgga aagaccttct gtagcaagtc caatctttcc 1380

aaacatcagc gagtccacac tggagaggga gaagcaccgt aa 1422

## Description

### Field of the invention

The present invention relates to novel biomarkers for lupus and methods and kits for their use. Also provided are vaccines for the treatment or prevention of lupus.

### Background

Systemic lupus erythematosus (SLE) or lupus is a chronic autoimmune disease that can affect the joints and almost every major organ in the body, including heart, kidneys, skin, lungs, blood vessels, liver, and the nervous system. As in other autoimmune diseases, the body's immune system attacks the body's own tissues and organs, leading to inflammation. A person's risk to develop lupus appears to be determined mainly by genetic factors, but environmental factors, such as infection or stress may trigger the onset of the disease. The course of lupus varies, and is often characterised by alternating periods of flares, i.e. increased disease activity, and periods of remission.

It has been estimated that in the US, about 1 million people suffer from lupus, world-wide, conservative estimates indicate about 4 million lupus patients. Lupus occurs about 9 times more frequently in women than in men. Although lupus can occur at any age, it is most common in women of childbearing age.

There is presently no cure for lupus. However, there are means of reducing and dealing with flares, including drugs such as anti-inflammatory drugs, alternative medicines or life-style changes. The treatment regimes will depend on the severity of the disease, and the responsiveness of the patient. Disease-modifying antirheumatic drugs can be used preventively to reduce the incidence of flares. When flares occur, they are often treated with corticosteroids.

As mentioned above, lupus patients can present with a variety of diverse symptoms, and is therefore difficult to diagnose. A number of tests are used in order to make a diagnosis, including antinuclear antibody test (ANA), tests for other auto-antibodies such as anti-DNA antibodies, tests for serum complement levels, urine analysis, looking for elevated protein levels in urine, and sometimes biopsies of an affected organ are used. However, these tests do not necessarily give a definitive diagnosis; for example, a positive ANA test can occur due to infections or rheumatic diseases, and even healthy people without lupus can test positive. The sensitivity and specificity for the ANA test are 93% and 57% (Habash-Bseiso,D Clin Med Res. 2005 August; 3(3): 190-193). Lupus diagnosis often takes 7 to 10 years using current methods.

There is therefore a need for new or improved biomarkers for lupus that can be used in methods of diagnosing lupus, for the early detection of lupus, subclinical or presymptomatic lupus or a predisposition to lupus, or for monitoring the progression of lupus or the likelihood to transition from remission to flare or vice versa, or the efficacy of a therapeutic treatment thereof.

### Summary of the Invention

It has now been found that the presence of certain auto-antibodies or combinations of auto-antibodies in a patient, or a significant increase in the number or concentration of such auto-antibodies or combinations of auto-antibodies provide useful biomarkers for lupus. Early results indicate that the disclosed invention will also distinguish lupus from other diseases.

Therefore the present invention provides biomarkers valuable in diagnosing or detecting a susceptibility to lupus. The present invention also provides panels of biomarkers valuable in diagnosing lupus or detecting a susceptibility to lupus. The present disclosure includes panels with good specificity and / or selectivity as compared to prior art methods. Said biomarkers and panels of biomarkers are also useful in monitoring the progression or regression of lupus, or monitoring the progression to a flare of the disease or transition from a flare into remission or monitoring the efficacy of a therapeutic agent to lupus.

In one embodiment, the present invention provides methods / uses and products (e.g. biomarkers, kits, vaccines, panels and methods of using the same) which employ ZMAT2 (including variants thereof (see discussion below)) as an auto-antigen, optionally with at least 1, 2, 3, 4, 5, 7, 9, 10, 15 or 20 other auto-antigens (e.g. as recited in Tables 1, 2, 3, 4 or 5) also being used.

Suitably the at least 1, 2, 3, 4, 5, 7, 9, 10, 15, 20 or 25 other auto-antigens that are used are selected from the group consisting of:
(a) BPY2IP1 (MAPIS protein); CEBPG (CCAAT/enhancer binding protein (C/EBP), gamma); E1B-AP5 (E1B-55kDa-associated protein 5); FUS (Fusion (involved in t(12;16) in malignant liposarcoma)); HAGH (Hydroxyacylglutathione hydrolase); HMG20B (High-mobility group 20B); HOXB6 (Homeo box B6, transcript variant 2); KRT8 (Keratin 8); LIN28 (Lin-28 homolog (C. elegans)); NDUFV3 (NADH dehydrogenase (ubiquinone) flavoprotein 3, 10kDa); PABPC1 (Poly(A) binding protein, cytoplasmic 1); PHLDA1 (Pleckstrin homology-like domain, family A, member 1); PIAS2 (Msx-interacting-zinc finger, transcript variant alpha); RAB11FIP3 (RAB11 family interacting protein 3 (class II)); RAN (RAN, member RAS oncogene family); RARA (Retinoic acid receptor alpha); RBMS 1 (RNA binding motif, single stranded interacting protein 1, transcript variant); RDBP (RD RNA binding protein); RNF12 (Ring finger protein 12, transcript variant 1); RUFY1 (RUN and FYVE domain containing 1); SMN1 (Survival of motor neuron 1, telomeric); SRPK1 (SFRS protein kinase 1); SSNA1 (Sjogren's syndrome nuclear autoantigen 1); STAU. (Staufen, RNA binding protein (Drosophila), transcript variant T3); STK11 (Serine/threonine kinase 11 (Peutz-Jeghers syndrome)); TOM1 (Target of myb1 (chicken)); TXNL2 (Thioredoxin-like, clone MGC:12349); TXNRD1 (Thioredoxin reductase 1, transcript variant 5); and ZNF38 (Zinc fmger protein 38); or
(b) ASPSCR1 (alveolar soft part sarcoma chromosome region, candidate 1); CEBPG (CCAAT/enhancer binding protein (C/EBP), gamma); DDX55 (DEAD (Asp-Glu-Ala-Asp) box polypeptide 55); DOM3Z (dom-3 homolog Z (C. elegans)); E1B-APS (E1B-55kDa-associated protein 5) FUS (Fusion (involved in t(12;16) in malignant liposarcoma))); HMG20B (High-mobility group 20B); KRT8 (Keratin 8); LIN28 Lin-28 homolog (C. elegans)); LNX (ligand of numb-protein X); MAP1S (MAP1S protein); PABPC1 (Poly(A) binding protein, cytoplasmic 1); PHLDA1 (Pleckstrin homology-like domain, family A, member 1); PIAS2 (Msx-interacting-zinc finger, transcript variant alpha); PRKCBP1 (protein kinase C binding protein 1); PRKRA (protein kinase, interferon-inducible double stranded RNA dependent activator); RARA (Retinoic acid receptor, alpha); RDBP (RD RNA binding protein); RPL30 (ribosomal protein L30); RPL31 (ribosomal protein L31); SNK (serum-inducible kinase); SRPK1 (SFRS protein kinase 1); STAU1 (Staufen, RNA binding protein (Drosophila), transcript variant T3); TXNL2 (Thioredoxin-like, clone MGC:12349); VCL (vinculin); ZMAT2 (Zinc finger, matrin type 2); and ZNF38. (Zinc finger protein 38).

In addition to ZMAT2 and the at least 1, 2, 3, 4, 5, 7, 9, 10,15,20 or 25 other auto-antigens (e.g. as selected from group (a) or (b) above), one or more further auto-antigens may be employed wherein said one or more further auto-antigens are selected from the group consisting of: IFI16 (Interferon, gamma-inducible protein 16); PSME3 (Proteasome (prosome, macropain) activator subunit 3 (PA28 gamma; Ki), transc); RALBP1 (RalA-binding protein 1); SSA2 (Sjogren syndrome antigen A2 (60kDa, ribonucleoprotein autoantigen SS-A/Ro)); and BANK1 (B-cell scaffold protein with ankyrin repeats 1).

Thus, it will be appreciated that the methods of the invention may comprise testing a patient sample against ZMAT2 / a combination of auto-antigens which combination may be as outlined above (including variants thereof). In this way, the presence, absence or a modulated level of auto-antibodies in the sample may be detected. In some embodiments the auto-antigens are provided / used in the form of a panel and methods of using these panels as well as the panels themselves form part of the present invention.

Examples of particular methods / uses and products employing ZMAT2 / a combination of auto-antigens as outlined above will be apparent from the description and the appended claim set of this application. Also, specific examples of methods / uses and products as described herein may be modified so as to employ any particular combination of auto-antigens that can be derived from the above discussion of the methods / uses and products of the present invention.

The methods, biomarkers, kits and panels of the present invention may be used alone or in combination with (e.g. before, after or concurrently with) one or more other diagnostic / prognostic indicators or techniques. Such a combination may for instance be used to to: (i) diagnose lupus; (ii) detect a susceptibility to lupus; (iii) monitor the progression or regression of lupus; (iv) monitor the progression to a flare of the disease or transition from a flare into remission; or (v) monitor the efficacy of a therapeutic agent to lupus.

Examples of diagnostic / prognostic indicators or techniques which may be employed in combination with the disclosure of the present invention can include those which have utility in the diagnosis / prognosis of lupus. Examples may include: the presence of symptoms characteristic or indicative of lupus; a complete or incomplete SLEDAI (Systemic Lupus Erythematosus Disease Activity Index) score; anti-nuclear antibody (ANA) blood test; tests for other auto-antibodies such as anti-DNA antibodies; tests for serum complement levels; urine analysis (e.g. looking for elevated protein levels in urine); the use of one or more other biomarkers; anti-ds DNA test; blood counts; and biopsy analysis of an affected organ.

The present invention provides a method useful in diagnosing a patient as having or being predisposed to developing lupus, comprising testing a sample from the patient against a panel of auto-antigens to detect the presence, absence or a modulated level of auto-antibodies in the sample, wherein the panel of auto-antigens comprises:
(a) the auto-antigens listed in Table 2,
(b) the panel of (a) in which up to 5 auto-antigens are deleted or replaced, or
(c) the panel of (a) in which up to 6, 7, 8, 9 or 10 ore more auto-antigens are deleted or replaced.

As will be appreciated from the defmition of "diagnosis" (see below), the above method may be useful in providing a positive or negative diagnosis of lupus (or a predisposition thereto).

The auto-antigens of Table 2 are ZMAT2 (Zinc finger, matrin type 2); ASPSCR1 (alveolar soft part sarcoma chromosome region, candidate 1); BANK1 B-cell (scaffold protein with ankyrin repeats 1); CEBPG (CCAAT/enhancer binding protein (C/EBP), gamma); DDX55 (DEAD (Asp-Glu-Ala-Asp) box polypeptide 55); DOM3Z (dom-3 homolog Z (C. elegans)); E1B-AP5 (E1B-SSkDa-associated protein 5) FUS (Fusion (involved in t(12;16) in malignant liposarcoma))); HMG20B (High-mobility group 20B); IFI16 (Interferon, gamma-inducible protein 16); KRT8 (Keratin 8); LIN28 Lin-28 homolog (C. elegans)); LNX (ligand of numb-protein X); MAP1S (MAP1S protein); PABPC1 (Poly(A) binding protein, cytoplasmic 1); PHLDA1 (Pleckstrin homology-like domain, family A, member 1); PIAS2 (Msx-interacting-zinc finger, transcript variant alpha); PRKCBP1 (protein kinase C binding protein 1); PRKRA (protein kinase, interferon-inducible double stranded RNA dependent activator); PSME3 (Proteasome (prosome, macropain) activator subunit 3 (PA28 gamma; Ki)); RALBP1 (Ra1A binding protein 1); RARA (Retinoic acid receptor, alpha); RDBP (RD RNA binding protein); RPL30 (ribosomal protein L30); RPL31 (ribosomal protein L31); SNK (serum-inducible kinase); SRPK1 (SFRS protein kinase 1); SSA2 (Sjogren syndrome antigen A2 (60kDa, ribonucleoprotein autoantigen SS-A/Ro)); STAU1 (Staufen, RNA binding protein (Drosophila), transcript variant T3); TXNL2 (Thioredoxin-like, clone MGC:12349); VCL (vinculin); ZNF38 (Zinc finger protein 38).

The present invention provides a method useful in diagnosing a patient as having or being predisposed to developing lupus, comprising testing a sample from the patient against a panel of auto-antigens to detect the presence, absence or a modulated level of auto-antibodies in the sample, wherein the panel of auto-antigens comprises:
(a) the auto-antigens listed in Table 1,
(b) the panel of (a) in which up to 5 auto-antigens are deleted or replaced, or
(c) the panel of (a) in which up to 6, 7, 8, 9 or 10 or more auto-antigens are deleted or replaced.

The auto-antigens of Table 1 are ZMAT2 (Zinc finger, matrin type 2); BANK1 (B-cell scaffold protein with ankyrin repeats 1); BPY2IP1 (MAP1S protein); CEBPG (CCAAT/enhancer binding protein (C/EBP), gamma); E1B-APS (ElB-55kDaassociated protein 5); FUS (Fusion (involved in t(12;I6) in malignant liposarcoma)); HAGH (Hydroxyacylglutathione hydrolase); HMG20B (High-mobility group 20B); HOXB6 (Homeo box B6, transcript variant 2); IFI16 (Interferon, gamma-inducible protein 16); KRT8 (Keratin 8); LIN28 (Lin-28 homolog (C. elegans)); NDUFV3 (NADH dehydrogenase (ubiquinone) flavoprotein 3, 10kDa); PABPC1 (Poly(A) binding protein, cytoplasmic 1); PHLDA1 (Pleckstrin homology-like domain, family A, member 1); PIAS2 (Msx-interacting-zinc fmger, transcript variant alpha); RAB11FIP3 (RAB11 family interacting protein 3 (class II)); RALBP1 (Ra1A-binding protein 1); RAN (RAN, member RAS oncogene family); RARA (Retinoic acid receptor alpha); RBMS1 (RNA binding motif, single stranded interacting protein 1, transcript variant); RDBP (RD RNA binding protein); RNF12 (Ring finger protein 12, transcript variant 1); RUFY1 (RUN and FYVE domain containing 1); SMN1 (Survival of motor neuron 1, telomeric); SRPK1 (SFRS protein kinase 1); SSNA1 (Sjogren's syndrome nuclear autoantigen 1); STAU (Staufen, RNA binding protein (Drosophila), transcript variant T3); STK11 (Serine/threonine kinase 11 (Peutz-Jeghers syndrome)); TOM1 (Target ofmyb1 (chicken)); TXNL2 (Thioredoxin-like, clone MGC:12349); TXNRD1 (Thioredoxin reductase 1, transcript variant 5); ZNF38 (Zinc finger protein 38), PSME3 (Proteasome (prosome, macropain) activator subunit 3 (PA28 gamma; Ki), transc), and SSA2 (Sjogren syndrome antigen A2 (60kDa, ribonucleoprotein autoantigen SS-A/Ro)).

The present invention provides a method useful in diagnosing a patient as having or being predisposed to developing lupus, comprising testing a sample from the patient against a panel of auto-antigens to detect the presence, absence or a modulated level of auto-antibodies in the sample, wherein the panel of auto-antigens comprises:
(a) the auto-antigens listed in Table 4,
(b) the panel of (a) in which up to 5 auto-antigens are deleted or replaced, or
(c) the panel of (a) in which up to 6, 7, 8, 9 or 10 or more auto-antigens are deleted or replaced.

The auto-antigens of Table 4 are ZMAT2 (Zinc finger, matrin type 2); BANK1 (B-cell scaffold protein with ankyrin repeats 1); BPY2IP 1 or MAP1S (MAP1S protein); CEBPG (CCAAT/enhancer binding protein (C/EBP), gamma); E1B-AP5 (E1B-55kDaassociated protein 5); FUS (Fusion (involved in t(12;16) in malignant liposarcoma)); HMG20B (High-mobility group 20B); IFI16 (Interferon, gamma-inducible protein 16); KRT8 (Keratin 8); LIN28 (Lin-28 homolog (C. elegans)); PABPC1 (Poly(A) binding protein, cytoplasmic 1); PHLDA1 (Pleckstrin homology-like domain, family A, member 1); PIAS2 (Msx-interacting-zinc finger, transcript variant alpha); PSME3 (Proteasome (prosome, macropain) activator subunit 3 (PA28 gamma; Ki)); RALBP1 (RalA binding protein 1); RARA (Retinoic acid receptor, alpha); RDBP (RD RNA binding protein); SRPKI (SFRS protein kinase 1); SSA2 (Sjogren syndrome antigen A2 (60kDa, ribonucleoprotein autoantigen SS-A/Ro)); STAU (Staufen, RNA binding protein (Drosophila), transcript variant T3); TXNL2 (Thioredoxin-like, clone MGC:12349); ZNF38 (Zinc finger protein 38).

The present invention provides a method useful in diagnosing a patient as having or being predisposed to developing lupus, comprising testing a sample from the patient against a panel of auto-antigens to detect the presence, absence or a modulated level of auto-antibodies in the sample, wherein the panel of auto-antigens comprises:
(a) the auto-antigens listed in Table 3,
(b) the panel of (a) in which up to 5 auto-antigens are deleted or replaced, or
(c) the panel of (a) in which up to 10, 15, 20 or 25 or more auto-antigens are deleted or replaced.

The auto-antigens of Table 3 are ZMAT2 (Zinc finger, matrin type 2); ASPSCR1 (alveolar soft part sarcoma chromosome region, candidate 1); BANK1 (B-cell scaffold protein with ankyrin repeats 1); BPY2IPl or MAP1S (MAP1S protein); CEBPG (CCAAT/enhancer binding protein (C/EBP), gamma); DDX55 (DEAD (Asp-Glu-Ala-Asp) box polypeptide 55); DOM3Z (dom-3 homolog Z (C. elegant)); EIB-AP5 (E1B-55kDa-associated protein 5); FUS (Fusion (involved in t(12;16) in malignant liposarcoma)); HAGH (Hydroxyacylglutathione hydrolase); HMG20B (High-mobility group 20B); HOXB6 (Homeo box B6, transcript variant 2); IFI16 (Interferon, gamma-inducible protein 16); KRT8 (Keratin 8); LIN28 (Lin-28 homolog (C. elegans)); LNX (ligand of numb-protein X); NDUFV3 (NADH dehydrogenase (ubiquinone) flavoprotein 3, lOkDa); PABPC1 (Poly(A) binding protein, cytoplasmic 1); PHLDA1 (Pleckstrin homology-like domain, family A, member 1}; PIAS2 (Msx-interacting-zinc finger, transcript variant alpha); PRKCBP 1 (protein kinase C binding protein 1); PRKRA (protein kinase, interferon-inducible double stranded RNA dependent activator); PSME3 (Proteasome (prosome, macropain) activator subunit 3 (PA28 gamma; Ki)); RAB11FIP3 (RAB11 family interacting protein 3 (class II)); RALBP1 (RalA binding protein 1); RAN (RAN, member RAS oncogene family);RARA (Retinoic acid receptor, alpha); RBMS1 (RNA binding motif, single stranded interacting protein 1, transcript variant); RDBP (RD RNA binding protein); RNF12 (Ring finger protein 12, transcript variant 1); RPL30 (ribosomal protein L30); RPL31 (ribosomal protein L31); RLTFY1 (RUN and FYVE domain containing 1); SMN1 (Survival of motor neuron 1, telomeric); SNK (serum-inducible kinase); SRPK1 (SFRS protein kinase 1} ;SSA2 (Sjogren syndrome antigen A2 (60kDa, ribonucleoprotein autoantigen SS-A/Ro)); SSNA1 Sjogren's syndrome nuclear autoantigen 1); STAU (Staufen, RNA binding protein (Drosophila), transcript variant T3); STK11 (Serine/threonine kinase 11 (Peutz-Jeghers syndrome)); TOM1 (Target of myb1 (chicken)); TXNL2 (Thioredoxin-like, clone MGC:12349); TXNRD1 (Thioredoxin reductase 1, transcript variant 5); VCL (Vinculin); ZNF38 (Zinc finger protein 38).

The present invention also provides a method of diagnosing a patient as having or being predisposed to developing lupus, comprising detecting in a sample taken from said patient one or more auto-antibodies that bind an auto-antigen selected from the group consisting of the auto-antigens listed in Table 3 (e.g. ZMAT2), wherein the presence of said one or more auto-antibodies, or an increase in the concentration of said one or more auto-antibodies, indicates that the patient suffers from or is predisposed to develop lupus.

The present invention further provides a method useful in diagnosing a patient as having or being predisposed to developing lupus, comprising testing a sample from the patient against a panel of auto-antigens to detect the presence, absence or a modulated level of auto-antibodies in the sample, wherein the panel of auto-antigens comprises at least 10 auto-antigens selected from Table 3 or from Table 1 or from Table 2.

Preferably the panel comprises at least 15 or 20 auto-antigens and/or preferably the panel comprises ZMAT2.

In one embodiment the at least 10, 15 or 20 auto-antigens are selected from the group consisting of:
(i) ZMAT2 (Zinc finger, matrin type 2); BPY2IP1 (MAP1S protein); CEBPG (CCAAT/enhancer binding protein (C/EBP), gamma); E1B-AP5 (ElB-55kDa-associated protein 5); FUS (Fusion (involved in t(12;16) in malignant liposarcoma)); HAGH (Hydroxyacylglutathione hydrolase); HMG20B (High-mobility group 20B); HOXB6 (Homeo box B6, transcript variant 2); KRT8 (Keratin 8); LIN28 (Lin-28 homolog (C. elegans)); NDUFV3 (NADH dehydrogenase (ubiquinone) flavoprotein 3, 10kDa); PABPCl (Poly(A) binding protein, cytoplasmic 1); PHLDA1 (Pleckstrin homology-like domain, family A, member 1); PIAS2 (Msx-interacting-zinc finger, transcript variant alpha); RAB11FIP3 (RAB11 family interacting protein 3 (class II)); RAN (RAN, member RAS oncogene family); RARA (Retinoic acid receptor alpha); RBMS1 (RNA binding motif, single stranded interacting protein 1, transcript variant); RDBP (RD RNA binding protein); RNF12 (Ring finger protein 12, transcript variant 1); RUFY1 (RUN and FYVE domain containing 1); SMN1 (Survival of motor neuron 1, telomeric); SRPK1 (SFRS protein kinase 1); SSNA1 (Sjogren's syndrome nuclear autoantigen 1); STAU (Staufen, RNA binding protein (Drosophila), transcript variant T3); STK11 (Serine/threonine kinase 11 (Peutz-Jeghers syndrome)); TOM1 (Target of myb 1 (chicken)); TXNL2 (Thioredoxin-like, clone MGC:12349); TXNRD1 (Thioredoxin reductase 1, transcript variant 5); and ZNF38 (Zinc finger protein 38); or
(ii) ZMAT2 (Zinc finger, matrin type 2); ASPSCR1 (alveolar soft part sarcoma chromosome region, candidate 1); CEBPG (CCAAT/enhancer binding protein (C/EBP), gamma); DDX55 (DEAD (Asp-Glu-Ala-Asp) box polypeptide 55); DOM3Z (dom-3 homolog Z (C. elegans)); E1B-AP5 (E1B-55kDa-associated protein 5) FUS (Fusion (involved in t(12;16) in malignant liposarcoma)); HMG20B (High-mobility group 20B); KRT8 (Keratin 8); LIN28 Lin-28 homolog (C. elegans)); LNX (ligand of numb-protein X); MAP1S (MAP1S protein); PABPC 1 (Poly(A) binding protein, cytoplasmic 1); PHLDA 1(Pleckstrin homology-like domain, family A, member 1); PIAS2 (Msx-interacting-zinc finger, transcript variant alpha); PRKCBP1 (protein kinase C binding protein 1); PRKRA (protein kinase, interferon-inducible double stranded RNA dependent activator); RARA (Retinoic acid receptor, alpha); RDBP (RD RNA binding protein); RPL30 (ribosomal protein L30); RPL31 (ribosomal protein L31); SNK (serum-inducible kinase); SRPK1 (SFRS protein kinase 1); STAU1 (Staufen, RNA binding protein (Drosophila), transcript variant T3); TXNL2 (Thioredoxin-like, clone MGC:12349); VCL (vinculin); and ZNF38 (Zinc finger protein 38).

Conveniently in the methods of the present invention detecting the presence or an increased level of at least two auto-antibodies provides information useful for diagnosis, preferably detecting the presence of an increased level of at least three, preferably at least four and more preferably at least five auto-antibodies provides information useful for diagnosis.

Advantageously the sample from the patient used in the methods of the present invention is a body fluid, optionally selected from blood, serum, plasma, saliva, lymphatic fluid, wound secretion, urine, faeces, mucus or cerebrospinal fluid (CSF). Typically, the sample may be serum or plasma.

A further aspect of the present invention provides a method of monitoring the progression or regression of lupus, or monitoring the progression to a flare of the disease or transition from a flare into remission, comprising (a) performing methods described above, repeating step (a) using a second sample obtained from the patient at a time later than the first sample; and (c) comparing the auto-antibodies detected in said first and second samples; wherein a change in the level or presence or absence of the auto-antibodies detected between the first and second samples indicates the progression or regression of lupus, or the progression to a flare, or transition from a flare into remission.

Another aspect of the present invention provides a method of monitoring the efficacy in a patient of a therapeutic agent to lupus, comprising (a) performing methods described above (b) administering the therapeutic agent to the patient; (c) performing again method described above using a second sample from the patient taken after the administration of the therapeutic agent; and (d) comparing the auto-antibodies detected in said first and second samples; wherein a change in the level or presence or absence of the auto-antibodies detected in the first and second sample is indicative of the efficacy of the therapeutic agent to lupus.

The present invention also provides a panel of auto-antigens as used in any of the methods described herein. In one embodiment, the present invention provides a panel of auto-antigens, wherein said panel comprises:
(i)
   (a) the auto-antigens listed in Table 2,
   (b) the panel of (a) in which up to 5 auto-antigens are deleted or replaced, or
   (c) the panel of (a) in which up to 6, 7, 8, 9 or 10 ore more auto-antigens are deleted or replaced;
(ii)
   (a) the auto-antigens listed in Table 1,
   (b) the panel of (a) in which up to 5 auto-antigens are deleted or replaced, or
   (c) the panel of (a) in which up to 6, 7, 8, 9 or 10 or more auto-antigens are deleted or replaced;
(iii)
   (a) the auto-antigens listed in Table 4,
   (b) the panel of (a) in which up to 5 auto-antigens are deleted or replaced, or
   (c) the panel of (a) in which up to 6, 7, 8, 9 or 10 or more auto-antigens are deleted or replaced; or
(iv)
   (a) the auto-antigens listed in Table 3,
   (b) the panel of (a) in which up to 5 auto-antigens are deleted or replaced, or
   (c) the panel of (a) in which up to 10, 15, 20 or 25 or more auto-antigens are deleted or replaced.

As will be appreciated from the description of the invention, other panels of auto-antigens have also been described for use in the methods of the present invention and these panels may also form part of the present invention. In a preferred embodiment, a panel of the invention comprises ZMAT2.

In one embodiment a panel of the invention is for use in: (i) diagnosing lupus or determining the severity thereof; (ii) detecting a susceptibility to lupus; (iii) monitoring the progression or regression of lupus; (iv) monitoring the progression to a flare of the disease or transition from a flare into remission; or (v) monitoring the efficacy of a therapeutic agent to lupus.

### Figures

Figure 1 is a cartoon illustrating a method for the detection of auto-antibodies in serum.

### Description

As explained further in the Examples below, in order to elucidate biomarkers valuable in diagnosing or detecting susceptibility to lupus, the inventors developed microarrays of antigens suitable for detecting antibodies in samples. Subsequently_{;} a large number of body fluid samples obtained from individuals positively diagnosed as having lupus with existing methods were analysed for the presence of auto-antibodies binding to auto-antigens immobilized on microarrays. Data analysis of the results obtained allowed detection of useful biomarkers for lupus.

A first data analysis methodology allowed detection of a set of 35 biomarkers, auto-antigens to which the patient serum samples contained auto-antibodies, and which are set out in Table 1 below.

A second data analysis methodology allowed detection of a set of 32 biomarkers which are set out in Table 2 below.

Between the two analysis methods a total of 45 useful biomarkers were identified which are set out in Table 3 below. The first and second analysis methods identified 22 biomarkers in common. These shared biomarkers are set out in Table 4 below. The biomarkers which were unique to each set are set out in Table 5 below.

In one embodiment of the invention, modified versions of Tables 1, 2, 3, 4 or 5 are envisaged in which 1, 2, 3, 4 or all 5 of the following markers are removed from the Table: IFI16 (Interferon, gamma-inducible protein 16); PSME3 (Proteasome (prosome, macropain) activator subunit 3 (PA28 gamma; Ki), transc); RALBP1 (Ra1A-binding protein 1); SSA2 (Sjogren syndrome antigen A2 (60kDa, ribonucleoprotein autoantigen SS-A/Ro)); and BANK1 (B-cell scaffold protein with ankyrin repeats 1).

In such a scenario a reference to a panel comprising, say, 5, auto-antigens of Table I would be a reference to a panel comprising 5 auto-antigens selected from the group consisting of: ZMAT2 (Zinc finger, matrin type 2); BPY2IPI (MAP1S protein); CEBPG (CCAAT/enhancer binding protein (C/EBP), gamma); EIB-APS (E1B-55kDa-associated protein 5); FUS (Fusion (involved in t(12;16) in malignant liposarcoma)); HAGH (Hydroxyacylglutathione hydrolase); HMG20B (High-mobility group 20B); HOXB6 (Homeo box B6, transcript variant 2); KRT8 (Keratin 8); LIN28 (Lin-28 homolog (C. elegans)); NDUFV3 (NADH dehydrogenase (ubiquinone) flavoprotein 3, 10kDa); PABPC1 (Poly(A) binding protein, cytoplasmic 1); PHLDA1 (Pleckstrin-homology-like domain, family A, member 1); PIAS2 (Msx-interacting-zinc finger, transcript variant alpha); RAB11FIP3 (RAB11 family interacting protein 3 (class II)); RAN (RAN, member RAS oncogene family); RARA (Retinoic acid receptor alpha); RBMS1 (RNA binding motif, single stranded interacting protein 1, transcript variant); RDBP (RD RNA binding protein); RNF12 (Ring finger protein 12, transcript variant 1); RUFY1 (RUN and FYVE domain containing 1); SMN1 (Survival of motor neuron 1, telomeric); SRPK1 (SFRS protein kinase 1); SSNA1 (Sjogren's syndrome nuclear autoantigen 1); STAU (Staufen, RNA binding protein (Drosophila), transcript variant T3); STK1 1 (Serinelthreonine kinase 11 (Peutz-Jeghers syndrome)); TOM1 (Target of myb1 (chicken)); TXNL2 (Thioredoxin-like, clone MGC:12349); TXNRD1 (Thioredoxin reductase 1, transcript variant 5); ZNF38 (Zinc finger protein 38).

Likewise, in such a scenario, a reference to a panel comprising, say, 5, auto-antigens of Table 2 would be a reference to a panel comprising 5 auto-antigens selected from the group consisting of: ZMAT2 (Zinc finger, matrin type 2); ASPSCR1 (alveolar soft part sarcoma chromosome region, candidate 1); CEBPG (CCAAT/enhancer binding protein (C/EBP), gamma); DDX55 (DEAD (Asp-Glu-Ala-Asp) box polypeptide 55); DOM3Z (dom-3 homolog Z (C. elegans)); E1B-AP5 (E1B-55kDaassociated protein 5) FUS (Fusion (involved in t(12;16) in malignant liposarcoma)); HMG20B (High-mobility group 20B); KRT8 (Keratin 8); LIN28 Lin-28 homolog (C. elegans)); LNX (ligand of numb-protein X); MAP1S (MAP1S protein); PABPC1 (Poly(A) binding protein, cytoplasmic 1); PHLDA1 (Pleckstrin homology-like domain, family A, member 1); PIAS2 (Msx-interacting-zinc fmger, transcript variant alpha); PRKCBP1 (protein kinase C binding protein 1); PRKRA (protein kinase, interferon-inducible double stranded RNA dependent activator); RARA (Retinoic acid receptor, alpha); RDBP (RD RNA binding protein); RPL30 (ribosomal protein L30); RPL31 (ribosomal protein L31); SNK (serum-inducible kinase); SRPK1 (SFRS protein kinase 1); STAU1 (Staufen, RNA binding protein (Drosophila), transcript variant T3); TXNL2 (Thioredoxin-like, clone MGC: 12349); VCL (vinculin); ZNF38 (Zinc finger protein 38).

According to one aspect of the present invention therefore there is provided a method of diagnosing a patient as having or being predisposed to developing lupus, said method comprising detecting in a sample taken from said patient one or more auto-antibodies that bind one or more auto-antigens; wherein the presence of said one or more auto-antibodies, or an increase in the concentration of said one or more auto-antibodies, indicates that the patient suffers or is predisposed to develop lupus, wherein said one or more auto-antigens are selected from ZMAT2 (Zinc finger, matrin type 2); BANK1 (B-cell scaffold protein with ankyrin repeats 1); BPY2IP1 (MAP1 S protein); CEBPG (CCAAT/enhancer binding protein (C/EBP), gamma); E1B-AP5 (E1B-55kDa-associated protein 5); FUS (Fusion (involved in t(12;16) in malignant liposarcoma)); HAGH (Hydroxyacylglutathione hydrolase); HMG20B (High-mobility group 20B); HOXB6 (Homeo box B6, transcript variant 2); KRT8 (Keratin 8); LIN28 (Lin-28 homolog (C. elegans)); NDUFV3 (NADH dehydrogenase (ubiquinone) flavoprotein 3, 10kDa); PABPC1 (poly(A) binding protein, cytoplasmic 1); PHLDA1 (Pleckstrin homology-like domain, family A, member 1); PIAS2 (Msx-interacting-zinc finger, transcript variant alpha); RAB11FIP3 (RAB11family interacting protein 3 (class II)); RAN (RAN, memberRAS oncogene family); RARA (Retinoic acid receptor alpha); RBMS1 (RNA binding motif, single stranded interacting protein 1, transcript variant); RDBP (RD RNA binding protein); RNF12 (Ring finger protein 12, transcript variant 1); RUFY1 (RUN and FYVE domain containing 1); SMN1 (Survival of motor neuron 1, telomeric); SRPK1 (SFRS protein kinase 1); SSNA1 (Sjogren's syndrome nuclear autoantigen 1); STAU (Staufen, RNA binding protein (Drosophila), transcript variant T3); STK11 (Serine/threonine kinase 11 (Peutz-Jeghers syndrome)); TOM1 (Target of myb1 (chicken)); TXNL2 (Thioredoxin-like, clone MGC:12349); TXNRD1 (Thioredoxin reductase 1, transcript variant 5); or ZNF38 (Zinc finger protein 38).

Preferably, the method comprises detecting auto-antibodies to one or more of the auto-antigens listed above, in combination with one or more of the auto-antigens IFI16 (Interferon, gamma-inducible protein 16); PSME3 (Proteasome (prosome, macropain) activator subunit 3 (PA28 gamma; Ki), transc), SSA2 (Sjogren syndrome antigen A2 (60kDa, ribonucleoprotein autoantigen SS-A/Ro)), and RALBP1 (RaIA.-binding protein 1).

The sample may be a body fluid, and is preferably selected from blood, serum, plasma saliva, lymphatic fluid, wound secretion, urine, faeces, mucus and cerebrospinal fluid (CSF). Typically, the sample may be serum or plasma.

The term "diagnosis" (and for the avoidance of doubt, grammatical variants thereof such as "diagnosing'', "diagnostics" etc.) as used herein is intended to be construed broadly. Thus, for example, the methods and products of the present invention are envisaged as being useful in the following: making a positive or negative diagnosis of lupus in a patient (including subclinical or presymptomatic lupus); assessing the severity of lupus in a patient; assessing an individual's susceptibility to lupus; providing a prognosis of an individual suffering from lupus; monitoring the progression or regression of lupus; monitoring the progression to a flare of the disease or transition from a flare into remission; or monitoring the efficacy of a therapeutic agent to lupus. As indicated above, the methods and products (kits, biomarkers, panels etc.) of the present invention may be used alone to achieve any of the foregoing or may alternatively be combined with one or more other diagnostic / prognostic indicators (e.g. the identification of one or more symptoms characteristic of lupus, the results of one or more biochemical tests etc.). Also, given the difficulties encountered in diagnosing lupus, it should be appreciated that the term "diagnosis" does not necessarily entail an unequivocal diagnosis of lupus in a patient but may rather, for example, provide an indication of the likelihood of lupus being present or absent in an individual.

By a "patient" (the term is used interchangeably herein with the term "individual") is meant an individual from whom a sample has been obtained e.g. for testing in accordance with a method of the present invention. The term "patient" is not intended to imply that the individual is necessarily undergoing any treatment and thus the term may refer to any individual who is being tested in accordance with a protocol of the present invention.

By "bind" or "bound" used herein is meant a specific interaction strong enough to withstand standard washing procedures in typical protein binding assays.

Accordingly, the present invention comprehends the use of one or more auto-antibodies that bind to said one or more auto-antigens as biomarkers for lupus.

By an increase in the concentration of said one or more auto-antibodies is meant a statistically significant increase in the concentration of the one or more auto-antibodies in said sample taken from the patient as compared with a predetermined mean concentration found in non-diseased, normal patients. A surrogate normal sample can also be used, for example a pooled normal control measured in relation to normal and diseased samples. Typically, the mean concentration of auto-antibodies found in normal patients may be calculated by reference to samples taken from a population of non-diseased individuals or from pooled samples from such individuals. In some embodiments, an increase of more than 5%, more suitably 10%, and suitably more than 15% or 20%, as compared with such mean may be taken as statistically significant. It will be appreciated that the units are arbitrary. In the case of clinical drug evaluation, there will be a change in auto-antibody concentration upon drug treatment; it is anticipated that the levels of most of these auto-antibodies may decrease post drug treatment.

Suitably, said method may comprise the detection of auto-antibodies that bind two or more of said auto-antigens, preferably 3 or more of said auto-antigens, even more preferably 4, 5, 6, 7, 8,9,10,11,12,13,14,15, 16, 17, 18, 19, 20, 21, 22, 23, 24, 25, 26, 27, 28, 29, 30 or 31 of these auto-antigens. In some embodiments, the patient may be pre-symptomatic.

In another aspect of the present invention there is provided a method of diagnosing a patient as having or being predisposed to developing lupus, said method comprising detecting in a sample from said patient auto-antibodies to a panel comprising 5 or more, preferably 6, 7, 8, 9, 10, 11, 12, 13, 14, 15, 16, 17, 18, 19, 20, 21, 22, 23, 24, 25, 26, 27, 28, 29, 30, 31, 32, 33, 34 or more, even more preferably to a panel comprising all 35, auto-antigens selected from the group consisting of those auto-antigens listed in Table 1 below; wherein the presence or separately a significant increase in the concentration of said auto-antibodies in the sample to substantially all members of the panel indicates that the patient suffers or is predisposed to develop lupus. In some embodiments, said panel may comprise additional auto-antigens (or other antigens) to those specified.

A significant increase in the concentration of the auto-antibodies means a statistically significant increase as determined by methods for statistically analysing experimental results of the kind commonly used in the art. By "substantially all members of the panel" is meant that the sample contains auto-antibodies, or an elevated concentration of auto-antibodies, to at least 50%, preferably at least 75% or 80% and typically at least 90% or 95%, of the auto-antigens included in the panel. It is envisaged that in some embodiments, the presence of auto-antibodies to one or more members of the panel and an increase in the respective concentrations of auto-antibodies to one or more other members of the panel will be indicative of lupus, it being unnecessary in most cases for there to be an increased concentration for all auto-antibodies detected. However, in some cases, the mere presence of auto-antibodies to substantially all members of the panel will be significant.

A significant decrease in the concentration of the auto-antibodies means a statistically significant decrease as determined by methods for statistically analysing experimental results of the kind commonly used in the art. By "substantially all members of the panel" is meant that the sample contains auto-antibodies, or a reduced concentration of auto-antibodies, to at least 50%, preferably at least 75% or 80% and typically at least 90% or 95%, of the auto-antigens included in the panel. It is envisaged that in some embodiments, the presence of auto-antibodies to one or more members of the panel and a decrease in the respective concentrations of auto-antibodies to one or more other members of the panel will be indicative of lupus, it being unnecessary in most cases for there to be a decreased concentration for all auto-antibodies detected.

It is expected that such a panel of biomarkers according to the present invention will provide greatly improved sensitivity or improved specificity in such methods of diagnosis as compared with known biomarkers for lupus.

A panel is defined as a collection of two or more individual markers where the information provided by the panel produces a greater sensitivity and/or specificity than that provided by the markers individually.

As mentioned above, the auto-antigens for lupus are listed in the Tables below (e.g. Table 1). References to gene sequences and uniprot accession numbers for such auto-antigens are also given. However, it will be appreciated that variants of the sequences given, for example allelic variants, orthologues from other mammalian species or other variants (e.g. fragments or antigens with post-translational modifications) that would be bound by the respective auto-antibodies may also be used. Accordingly, it will be understood that the use of such variants are contemplated as being within the scope of the present invention and that references to particular auto-antigens or biomarkers should be construed accordingly. In particular, the present invention comprehends the use of auto-antigens being encoded from respective gene sequences that individually have at least an 80% identity to the corresponding sequences listed in the Tables below (e.g. Table 1). Suitably, such sequences have at least an 85% or 90% identity to the corresponding sequences in the Tables, and preferably they have at least a 95% identity to such sequences, e.g., more than a 96%, 97%, 98% or 99% identity. The skilled person will appreciate that such variants should retain the ability to be bound by the auto-antibodies in a positive patient sample which auto-antibodies recognize the wild-type antigen.

**Table 1**

| Gene symbol | Name | Synonyms | Entrez Gene ID and protein sequence link |
|---|---|---|---|
| ZMAT2 | Zinc finger, matrin type 2 | FLJ31121 | 153527; http://ca.expasy.org/uniprot/Q96NCO |
| BANK1 | B-cell scaffold protein with ankyrin repeats 1 | BANK; FLJ20706; FLJ34204 | 55024; http://ca.expasy.org/uniprot/Q8NDB2 |
| BPY21P1 | MAP1S protein | MAP8; C19orf5; VCY21PI; FLJ10669; VCY21P-1; MGC133087 | 55201; http://www.ncbi.nlm.nih.gov/ entrez/viewer.fcgi?db=protein&val=AAH06358 .2 |
| CEBPG | CCAAT/enhancer binding protein (C/EBP), gamma | | 1054; http://ca.expasy.org/uniprot/P53567 |
| E1B-AP5 | E1B-55kDa-associated protein 5 | E1BAP5; E1B-AP5; HNRPUL1; FLJ12944 | 11100; http://ca.expasy.org/uniprot/076022 |
| FUS | Fusion (involved in t(12;16) in malignant liposarcoma) | TLS; CHOP; FUS1; FUS-CHOP; TLS/CHOP; hnRNP-P2 | 2521; http://ca.expasy.org/unipro/P35637 |
| HAGH | Hydroxyacylglutat hione hydrolase | GL02; GLX2; GLXII; HAGH1 | 3029; http://ca-expasy.org/uniprot/Q16775 |
| HMG20B | High-mobility group 20B | SOXL; HMGX2; BRAF25; BRAF35; PP7706; pp8857; FLJ26127; SMARCE1r | 10362; http://ca.expasy.org/uniprot/Q9POW2 |
| HOXB6 | Homeo box B6, transcript variant 2 | HOX2; HU-2; HOX2B; Hox-2.2 | 3216; http://ca.expasy.org/uniprot/P17509 |
| IFI16 | Interferon, gamma-inducible protein 16 | PYHIN2; IFNGIP1; MGC9466 | 3428; http://ca.expasy.org/uniprot/Q16666 |
| KRT8 | Keratin 8 | K8; KO; CK8; CYK8; K2C8; CARD2 | 3856; http://ca.expasy.org/uniprot/P05787 |
| LIN28 | Lin-28 homolog (C. elegans) | CSDD1; LIN-28; LIN28A; ZCCHC1; FLJ12457 | 79727; http://ca.expasy.org/uniprot/Q9N9Z2 |
| NDUFV3 | NADH dehydrogenase (ubiquinone) flavoprotein 3, 10kDa | CI-9KD | 4731; http://ca.expasy.org/uniprot/P56181 |
| PABPC1 | Poly(A) binding protein, cytoplasmic 1 | PAB1; PABP; PABP1; PABPC2; PABPL1 | 26986; http://ca.expasy.org/uniprot/P11940 |
| PHLDA1 | Pleckstrin homology-like domain, family A, member 1 | PHRIP; TDAG51; DT1P1B11; MGC131738 | 22822; http://ca.expasy.org/uniprot/Q8WV24 |
| PIAS2 | Msx-interacting-zinc finger, transcript variant alpha | miz; MIZI; SIZ2; PIASX; ZMIZ4; MGC102682; PIASX-BETA; PIASX-ALPHA | 9063; http://ca.expasy.org/uniprot/Q13105 |
| PSME3 | Proteasome (prosome, macropain) activator subunit 3 (PA28 gamma; Ki), transc | Ki; PA28G; REG-GAMMA; PA28-gamma | 10197; http://ca.expasy.org/uniprot/Q12920 |
| RAB11FI P3 | RAB11 family interacting protein 3 (class II) | KIAA0665; Rab11-FIP3 | 9727; http://ca.expasy.org/uniprot/075154 |
| RALBP1 | RalA binding protein 1 | RIP; RIP1; RLIP76 | 10928; http://ca.expasy.org/uniprot/Q15311 |
| RAN | RAN, member RAS oncogene family, | TC4; Gsp1; ARA24 | 5901; http://ca.expasy.org/uniprot/P17080 |
| RARA | Retinoic acid receptor, alpha | RAR; NR1B1 | 5914; http://ca.expasy.org/uniprot/P10276 |
| RBMS1 | RNA binding motif, single stranded interacting protein 1, transcript variant | YC1; MSSP; SCR2; MSSP-1; MSSP-2; MSSP-3; MGC3331; MGC15146 | 5937; http://ca.expasy.org/uniprot/P29558 |
| RDBP | RD RNA binding protein | RD; RDP; D6S45; NELF-E | 7936; http://ca.expasy.org/uniprot/P18615 |
| RNF12 | Ring finger protein 12, transcript variant 1 | RLlM; MGC15161; NY-REN-43 | 51132; http://ca.expasy.org/uniprot/Q9NVW2 |
| RUFY1 | RUN and FYVE domain containing 1 | RABIP4; ZFYVE12; FLJ22251 | 80230; http://ca.expasy.org/uniprot/Q96T51 |
| SMN1 | Survival of motor neuron 1, telomeric | SMA; SMN; SMA1; SMA2; SMA3; SMA4; SMA@; SMNT; BCD541; T-BCD541 | 6606; http://ca.expasy.org/uniDrot/Q16637 |
| SRPK1 | SFRS protein kinase 1 | SFRSK1 | 6732; http://ca.expasy.org/uniprot/Q96SB4 |
| SSA2 | Sjogren syndrome antigen A2 (60kDa, ribonucleoprotein autoantigen SS-AlRo) | RO60; SSA2; TROVE2 | 6738; http://ca.expasy.org/uniprot/P10155 |
| SSNA1 | Sjogren's syndrome nuclear autoantigen 1 | N14; NA14; NA-14 | 8636; http://ca.expasy.org/uniprot/O43805 |
| STAU | Staufen, RNA binding protein (Drosophila), transcript variant T3 | STAU; FLJ25010 | 6780; http://ca.expasy.org/uniprot/O95793 |
| STK11 | Serinefthreonine kinase 11 (Peutz-Jeghers syndrome) | PJS; LKB1 | 6794; http://ca.expasy.org/uniprot/Q15831 |
| TOM1 | Target of myb1 (chicken) | FLJ33404 | 10043; http://ca.expasy.org/uniprot/O60784 |
| TXNL2 | Thioredoxin-like, clone MGC:12349 | GRX3; GRX4; GLRX4; PICOT; TXNL2; TXNL3; FLJ11864; bA500G 10.4; GLRX3 | 10539; http://ca.expasy.prg/uniprot/O76003 |
| TXNRD1 | Thioredoxin reductase 1, transcript variant 5 | TR; TR1; TXNR; TRXR1; GRIM-12; MGC9145 | 7296; http://ca.expasy.org/uniprot/Q16881 |
| ZNF38 | Zinc finger protein 38 | ZNF38; Zipro1; NY-REN-21; DKFZp434L134; DKFZp686H1025 4 | 7589; http://ca.expasy.org/uniprot/Q9Y5A6 |

The term "sequence identity" refers to the degree to which two polynucleotide or polypeptide sequences are identical on a residue-by-residue basis over a particular region of comparison. The term "percentage of sequence identity" is calculated by comparing two optimally aligned sequences over that region of comparison, determining the number of positions at which the identical nucleotide base or amino acid occurs in both sequences to yield the number of matched positions, dividing the number of matched positions by the total number of positions in the region of comparison (i. e., the window size), and multiplying the result by 100 to yield the percentage of sequence identity. The identity between two DNA or protein sequences may be determined using computer programs known in the art, such as GAP software provided in the GCG program package with default parameters (Needleman and Wunsch 1970 J Mol BioI 48: 443-453). The identity between two protein sequences is also displayed after searching a protein database with Blast2 (Altschul et al (1997) Nucleic Acids Res. 25 (17) 3389-3402).

It is anticipated that the methods of the present invention may provide greater sensitivity and / or specificity for the diagnosis of lupus than previously available in the art.

By "sensitivity" is meant the proportion of persons with the disease phenotype who test positive.

By "specificity" is meant the proportion of persons without the disease phenotype who test negative.

Advantageously, the methods of the invention may have a sensitivity of>67%, preferably >70%, even more preferably >75%, 80%, 85%, 90%, and most preferably >95%, or a specificity of>85%, preferably >90%, even more preferably >95%, and most preferably > 98%.

According to yet another aspect of the invention there is provided a method of monitoring the progression or regression of lupus in a patient, said method comprising detecting in a first sample obtained from a patient one or more auto-antibodies that bind one or more auto-antigens selected from the group consisting of ZMAT2 (Zinc finger, matrin type 2); BANK1 (B-cell scaffold protein with ankyrin repeats 1); BPY2IP1 (MAP1S protein); CEBPG (CCAAT/enhancer binding protein (C/EBP), gamma); E1B-AP5 (E1B-55kDa-associated protein 5); FUS (Fusion (involved in t(12;16) in malignant liposarcoma)); HAGH (Hydroxyacylglutathione hydrolase); HMG20B (High-mobility group 20B); HOXB6 (Homeo box B6, transcript variant 2); KRT8 (Keratin 8); LIN28 (Lin-28 homolog (C. elegans)); NDUFV3 (NADH dehydrogenase (ubiquinone) flavoprotein 3, 10kDa); PABPC1 (Poly(A) binding protein, cytoplasmic 1); PHLDA1 (Pleckstrin homology-like domain, family A, member 1); PIAS2 (Msx-interacting-zinc finger, transcript variant alpha); RAB11FIP3 (RAB11 family interacting protein 3 (class II)); RAN (RAN, member RAS oncogene family); RARA (Retinoic acid receptor alpha); RBMS 1 (RNA binding motif, single stranded interacting protein 1, transcript variant); RDBP (RD RNA binding protein); RNF12 (Ring finger protein 12, transcript variant 1); RUFY1 (RUN and FYVE domain containing 1); SMN1 (Survival of motor neuron 1, telomeric); SRPK1 (SFRS protein kinase 1); SSNA1 (Sjogren's syndrome nuclear autoantigen 1); STAU (Staufen, RNA binding protein (Drosophila), transcript variant T3); STK11 (Serine/threonine kinase 11 (Peutz-Jeghers syndrome)); TOM1 (Target of myb1 (chicken)); TXNL2 (Thioredoxin-like, clone MGC:12349); TXNRD1 (Thioredoxin reductase 1, transcript variant 5); or ZNF38 (Zinc finger protein 38), or auto-antibodies that bind to a panel comprising 5 or more, preferably 6, 7, 8, 9, 10, 11, 12, 13, 14, 15, 16, 17, 18, 19, 20, 21, 22, 23, 24, 25, 26, 27, 28, 29, 30, 31, 32, 33, 34 or more, even more preferably to a panel comprising all 35, auto-antigens selected from the group consisting of those auto-antigens listed in Table 1; detecting said auto- . antibodies in a second sample obtained from the patient at a time later than the first sample; wherein a change in the auto-antibodies detected in the first and second samples is indicative of the progression or regression of lupus.

According to yet another aspect of the invention there is provided a method of monitoring the progression towards a flare of the disease, or the transition from a flare to remission, of lupus in a patient, said method comprising detecting in a first sample obtained from a patient one or more auto-antibodies that bind one or more auto-antigens selected from the group consisting of ZMAT2 (Zinc finger, matrin type 2); BANK1 (B-cell scaffold protein with ankyrin repeats 1); BPY2IP1 (MAP1S protein); CEBPG (CCAAT/enhancer binding protein (C/EBP), gamma); E1B-AP5 (E1B-55kDa-associated protein 5); FUS (Fusion (involved in t(12;16) in malignant liposarcoma)); HAGH (Hydroxyacylglutathione hydrolase); HMG20B (High-mobility group 20B); HOXB6 (Homeo box B6, transcript variant 2); KRT8 (Keratin 8); LIN28 (Lin-28 homolog (C. elegans)); NDUFV3 (NADH dehydrogenase (ubiquinone) flavoprotein 3, 10kDa); PABPC1 (Poly(A) binding protein, cytoplasmic 1); PHLDA1 (Pleckstrin homology-like domain, family A, member 1); PIAS2 (Msx-interacting-zinc finger, transcript variant alpha); RAB11FIP3 (RAB11 family interacting protein 3 (class II); RAN (RAN, member RAS oncogene family); RARA (Retinoic acid receptor alpha); RBMS1 (RNA binding motif, single stranded interacting protein 1, transcript variant); RDBP (RD RNA binding protein); RNF12 (Ring finger protein 12, transcript variant 1); RUFY1 (RUN and FYVE domain containing 1); SMN1 (Survival of motor neuron 1, telomeric); SRPK1 (SFRS protein Idnase 1); SSNA1 (Sjogren's syndrome nuclear autoantigen 1); STAU (Staufen, RNA binding protein (Drosophila), transcript variant T3); STK11_(Serine/threonine kinase 11 (Peutz-Jeghers syndrome)); TOM1 (Target of myb1 (chicken)); TXNL2 (Thioredoxin-like, clone MGC:i2349); TXNRD1 (Thioredoxin reductase 1, transcript variant 5); or ZNF38 (Zinc finger protein 38), or auto-antibodies that bind to a panel comprising 5 or more, preferably 6, 7, 8, 9, 10, 11, 12, 13, 14, 15, 16, 17, 18, 19, 20, 21, 22, 23, 24, 25, 26, 27, 28, 29, 30, 31, 32, 33, 34 or more, even more preferably to a panel comprising all 35, auto-antigens selected from the group consisting of those auto-antigens listed in Table 1; detecting said auto-antibodies in a second sample obtained from the patient at a time later than the first sample; wherein a change in the auto-antibodies detected in the first and second samples is indicative of the progression towards a flare, or transition from a flare to a period of remission of the disease.

A change in the auto-antibodies may be a change in the number of different auto-antigens to which auto-antibodies are detected in the first as compared to the second sample, or it may be a change in the concentration of an auto-antibody in the first as compared to the second sample, or it may be a combination of both.

Whilst the biomarkers specifically disclosed herein in relation to the present invention are elevated in lupus patients (and therefore an increased auto-antibody concentration of the biomarkers are indicative of lupus), auto-antibodies for which a decrease in concentration indicates that a patient suffers from or is presymptomatic or predisposed to develop lupus are also envisaged, and may also provide useful biomarkers for lupus. Such markers may be used in combination with the methods or products of the present invention so that an increase in certain biomarkers is indicative of lupus whilst a decrease in certain other biomarkers is also indicative of lupus.

According to yet another aspect of the present invention there is provided a method of monitoring the efficacy of a therapeutic agent to lupus, comprising detecting in a first sample obtained from a patient the presence of one or more auto-antibodies that bind one or more auto-antigens selected from the group consisting of ZMAT2 (Zinc finger, matrin type 2); BANK1 (B-cell scaffold protein with ankyrin repeats 1); BPY2IPl (MAP1S protein); CEBPG (CCAAT/enhancer binding protein (C/EBP), gamma); E1B-APS (E1B-55kDa-associated protein 5); FUS (Fusion (involved in t(12;16) in malignant liposarcoma)); HAGH (Hydroxyacylglutathione hydrolase); HMG20B (High-mobility group 20B); HOXB6 (Homeo box B6, transcript variant 2); KRT8 (Keratin 8); LIN28 (Lin-28 homolog (C. elegans));. NDUFV3 (NADH dehydrogenase (ubiquinone) flavoprotein 3, 10kDa); PABPC 1 (Poly(A) binding protein, cytoplasmic 1); PHLDA1 (Pleckstrin homology-like domain, family A, member 1); PIAS2 (Msx-interacting-zinc finger, transcript variant alpha); RAB11FIP3 (RAB11 family interacting protein 3 (class II)); RAN (RAN, member RAS oncogene family); RARA (Retinoic acid receptor alpha); RBMS 1 (RNA binding motif, single stranded interacting protein 1, transcript variant); RDBP (RD RNA binding protein); RNF12 (Ring finger protein 12, transcript variant 1); RUFY1 (RUN and FYVE domain containing 1); SMN1 (Survival of motor neuron 1, telomeric); SRPK1 (SFRS protein kinase 1); SSNA1 (Sjogren's syndrome nuclear autoantigen 1); STAU (Staufen, RNA binding protein (Drosophila), transcript variant T3); STK11_(Serine/threonine kinase 11 (Peutz-Jeghers syndrome)); TOM1 (Target of myb 1 (chicken)); TXNL2 (Thioredoxin-like, clone MGC:12349); TXNRD1 (Thioredoxin reductase 1, transcript variant 5); or ZNF38 (Zinc finger protein 38), or auto-antibodies that bind to a panel comprising 5 or more, preferably 6, 7, 8, 9,10, 11, 12, 13, 14, 15, 16, 17, 18, 19, 20, 21, 22, 23, 24, 25, 26, 27, 28, 29, 30, 31, 32, 33, 34 or more, even more preferably to a panel comprising all 35, auto-antigens selected from the group consisting of those auto-antigens listed in Table 1; administering the therapeutic agent to the patient; detecting said one or more auto-antibodies in a second sample taken from the patient taken after the administration of the therapeutic agent; and comparing the one or more auto-antibodies in said first and second samples respectively, wherein a change in the auto-antibodies in the first and second sample is indicative of the efficacy of the therapeutic agent to lupus.

The first sample may be taken prior to the first administration of a therapeutic agent, or after treatment has already commenced. The method can be used to monitor the efficacy of treatment over time, e.g., over days, weeks, months or years.

The therapeutic agent may be any therapeutic agent that can be beneficial for the treatment of lupus, for example anti-rheumatic agents or anti-inflammatory agents.

According to yet another aspect of the present invention, there is provided a method of predicting whether a patient suffering from lupus will respond to treatment with a therapeutic agent, comprising detecting the presence or absence of one or more auto-antibodies that bind to one or more auto-antigens selected from the group consisting of ZMAT2 (Zinc finger, matrin type 2); BANK1 (B-cell scaffold protein with ankyrin repeats 1); BPY2IP1 (MAP1S protein); CEBPG (CCAAT/enhancerbinding protein (C/EBP), gamma); E1B-AP5 (E1B-55kDa-associated protein 5); FUS (Fusion (involved in t(12;16) in malignant liposarcoma)); HAGH (Hydroxyacylglutathione hydrolase); HMG20B (High-mobility group 20B); HOXB6 (Homeo box B6, transcript variant 2); KRT8 (Keratin 8); LIN28 (Lin-28 homolog (C. elegans)); NDUFV3 (NADH dehydrogenase (ubiquinone) flavoprotein 3, 10kDa); PABPC1 (Poly(A) binding protein, cytoplasmic 1); PHLDA1 (Pleckstrin homology-like domain, family A, member 1); PIAS2 (Msx-interacting-zinc finger, transcript variant alpha); RAB11FIP3 (RAB11 family interacting protein 3 (class II)); RAN (RAN, member RAS oncogene family); RARA (Retinoic acid receptor alpha); RBMS 1 (RNA binding motif, single stranded interacting protein 1, transcript variant); RDBP (RD RNA binding protein); RNF12 (Ring finger protein 12, transcript variant 1); RUFY1 (RUN and FYVE domain containing 1); SMN1 (Survival of motor neuron 1, telomeric); SRPK1 (SFRS protein kinase 1); SSNA1 (Sjogren's syndrome nuclear autoantigen 1); STAU (Staufen, RNA binding protein (Drosophila), transcript variant T3); STK11_(Serine/threonine kinase 11 (Peutz-Jeghers syndrome)); TOM1 (Target of myb1 (chicken)); TXNL2 (Thioredoxin-like, clone MGC:12349); TXNRD1 (Thioredoxin reductase 1, transcript variant 5); or ZNF38 (Zinc fmger protein 38), or auto-antibodies that bind to a panel comprising 5 or more, preferably 6, 7, 8, 9, 10, 11, 12, 13, 14, 15, 16, 17, 18, 19, 20,21, 22, 23, 24, 25, 26, 27, 28, 29, 30, 31, 32, 33, 34 or more, even more preferably to a panel comprising all 35, auto-antigens selected from the group consisting of those auto-antigens listed in Table 1; wherein the presence or absence of said one or more auto-antibodies indicates that the patient will or will not respond to treatment with said therapeutic agent.

The auto-antibodies may be detected by any means known in the art. This includes the use of protein arrays, bead-based assays, Western blots, immunoprecipitation, silver staining, dot blots, etc. The binding of the auto-antibodies can be detected by any means, including enzyme-linked assays such as ELISAs, radioimmunoassays (RIAs), DELFIA assays, fluorescence-based detection methods, bead assays such as Luminex bead assays, etc.

In some embodiments, arrays of said auto-antigens may be employed for detecting said auto-antibodies, for example arrays prepared according to the methods disclosed by WO-A-01/57198 or WO-A-2003/064656; preferred detection methods are fluorescence-based detection methods.

The patient may have or be predisposed to develop the disease naturally. However, a patient may also develop lupus as a result of environmental factors, for example as a side effect of treatment with therapeutic agents or as a result of exposure to toxic materials, or as a result of manipulation, e.g., in animal models used in pharmaceutical research and development. The patient may be a human being, but can also be a non-human animal such, for example, as a mouse, rat, guinea pig, cat, dog, horse, pig, cow, non-human primate (e.g. monkey), or any other animal used for research and drug testing.

Each of the aspects and features of the invention and methods as described above are applicable to the use of auto-antigens from Tables 1, 2, 3, 4 and 5.

Therefore according to the present invention there is provided a method of diagnosing a patient as having or being predisposed to developing lupus, said method comprising detecting in a sample taken from said patient one or more auto-antibodies that bind one or more auto-antigens; wherein the presence of said one or more auto-antibodies, or an increase in the concentration of said one or more auto-antibodies, indicates that the patient suffers from or is predisposed to develop lupus, wherein said one or more antigens are selected from the auto-antigens set out in Table 2.

**Table 2**

| **Genesymbol** | **Name** | **Synonyms** | **Entrez Gene ID and protein sequence link** |
|---|---|---|---|
| ZMAT2 | Zinc finger, matrin type 2 | FLJ31121 | 153527; http:/ca.expasy.org/unipr ot/Q96NC0 |
| ASPSCR1 | alveolar soft part sarcoma chromosome region, candidate 1 | TUG; ASPL; ASPS; RCC17; UBXD9; UBXN9; ASPCR1; FLJ45380; ASPSCR1 | 79058; http://ca.expasy.org/unipr ot/Q7Z6N7 |
| BANK1 | B-cell scaffold protein with ankyrin repeats 1 | BANK; FLJ20706; FLJ34204 | 55024; http://ca.expasy.org/unipr ot/Q8NDB2 |
| CEBPG | CCAAT/enhancer binding protein (C/EBP), gamma | GPE1BP; IG/EBP-1; CEBPG | 1054; http://ca.expasy.org/unipr ot/P53567 |
| DDX55 | DEAD (Asp-Glu-Ala-Asp) box polypeptide 55 | FLJ16577; KIAA1595; MGC33209; DDX55 | 57696; http://ca.expasv.org/unipr ot/Q8NHQ9 |
| DOM3Z | dom-3 homolog Z (C. elegans) | NG6; DOM3L; DOM3Z | 1797; http://ca.expasy.org/unipr ot/077932 |
| E1B-AP5 | E1B-55kDa-associated protein 5 | E1BAP5; E1B-AP5; HNRPUL1; FLJ12944 | 11100; hftp://ca.expasy.org/unipr ot/O76022 |
| FUS | Fusion (involved in t(12;16) in malignant liposarcoma) | TLS; CHOP; FUS1; FUS-CHOP; TLS/CHOP; hnRNP-P2 | 2521; http://ca.expasy.org/unipr ot/P35637 |
| HMG20B | High-mobility group 20B | SOXL; HMGX2; BRAF25; BRAF35; PP7706; pp8857; FLJ26127; SMARCE1r | 10362; http://ca.expasy.org/unipr ot/Q9POW2 |
| IFI16 | Interferon, gamma-inducible protein 16 | PYHIN2; IFNGIP1; MGC9466 | 3428; http://ca.expasy.org/unipr ot/Q16666 |
| KRT8 | Keratin 8 | K8; KO; CK8; CYK8; K2C8; CARD2 | 3856; http://ca.expasy.org/unipr ot/P05787 |
| LIN28 | Lin-28 homolog (C. elegans) | CSDD1; LIN-28; LIN28A; ZCCHC1; FLJ12457 | 79727; http://ca.expasy.org/unipr ot/Q9H9Z2 |
| LNX | ligand of numb-protein X | LNX; MPDZ; PDZRN2; LNX1 | 84708; http://ca.expasy.org/unipr ot/Q8TBB1 |
| MAP1S | MAP1S protein | MAP8; C19orf5; VCY2IP1; FLJ10669; VCY2IP-1; MGC133087 | 55201; hftp://ca.expasy.org/unipr ot/Q66K74 |
| PABPC1 | Poly(A) binding protein, cytoplasmic 1 | PAB1; PABP; PABP1; PABPC2; PABPL1 | 26986; http://ca.expasy.orq/unipr ot/P11940 |
| PHLDA1 | Pleckstrin homology-like domain, family A, member 1 | PHRIP; TDAG51; DT1P1B11; MGC131738 | 22822; http://ca.expasy.org/unipr ot/Q8WV24 |
| PIAS2 | Msx-interacting-zinc finger, transcript variant alpha | miz; MIZ1; SIZ2; PIASX; ZMIZ4; MGC102682; PIASX-BETA; PIASX-ALPHA | 9063; hftp://ca.expasy.org/unipr ot/Q13105 |
| PRKCBP1 | protein kinase C binding protein 1 | RACK7; PRKCBP1; PRO2893; MGC31836; ZMYND8 | 23613; http://ca.expasy.org/unipr ot/Q9H2G5 |
| PRKRA | protein kinase, interferon-inducible double stranded RNA dependent activator | RAX; PACT; DYT16; HSD14; PRKRA | 8575; http://ca.expasy.org/unipr ot/O75569 |
| PSME3 | Proteasome (prosome, macropain) activator subunit 3 (PA28 gamma; Ki) | Ki; PA28G; REG-GAMMA; PA28-gamma | 10197; hftp://ca.expasy.org/unipr ot/Q12920 |
| RALBP1 | Ra1A binding protein 1 | RIP; RIP1; RLIP76 | 10928; http://ca.expasy.org/unipr ot/Q15311 |
| RARA | Retinoic acid receptor, alpha | RAR; NR1B1 | 5914; http:/Ica.expasy.org/unipr ot/P10276 |
| RDBP | RD RNA binding protein | RD; RDP; D6S45: NELF-E | 7936; http://ca.expasy.org/unipr ot/P18615 |
| RPL30 | ribosomal protein L30 | RPL30 | 6156; http://ca.expasy.org/unipr ot/P04645 |
| RPL31 | ribosomal protein L31 | MGC88191; RPL31 | 6160; http://ca.expasy.org/unipr ot/P62899 |
| SNK | serum-inducible kinase | SNK; PLK2 | 10769; http://ca.expasy.org/unipr ot/Q9NYY3 |
| SRPK1 | SFRS protein kinase 1 | SFRSK1 | 6732; http://ca.expasy.org/unipr ot/Q96S84 |
| SSA2 | Sjogren syndrome antigen A2 (60kDa, ribonucleoprotein autoantigen SS-A/Ro) | RO60; SSA2; TROVE2 | 6738; http://ca.expasy.org/unipr ot/P10155 |
| STAU1 | Staufen, RNA binding protein (Drosophila), transcript variant T3 | STAU; FLJ25010 | 6780; http://ca.expasy.org/unipr ot/O95793 |
| TXNL2 | Thioredoxin-like, clone MGC:12349 | GRX3; GRX4; GLRX4; PICOT; TXNL2; TXNL3; FLJ11864; bA500G10.4; GLRX3 | 10539; http://ca.expasy.org/unipr ot/O76003 |
| VCL | vinculin | MVCL; CMD1W; VCL | 7414; http://ca.expasy.org/unipr ot/P18206 |
| ZNF38 | Zinc finger protein 38 | ZNF38; Zipro1; NY-REN-21; DKFZp434L134; DKFZp686H10254 | 7589; http://ca.expasy.org/unipr ot/Q9Y5A6 |

In another aspect of the present invention provides a method of diagnosing a patient as having or being predisposed to developing lupus, said method comprising detecting in a sample from said patient auto-antibodies to a panel comprising 5 or more, preferably 6,7,8,9,10, 11,12,13,14,15,16,17,18,19,20,21,22,23,24,25,26,27, 28, 29, 30, 31, or more, even more preferably to a panel comprising all 32 auto-antigens selected from the group of auto-antigens listed in Table 2.

According to yet another aspect of the invention there is provided a method of monitoring the progression or regression of lupus in a patient, said method comprising detecting in a first sample obtained from a patient one or more auto-antibodies that bind one or more auto-antigens selected from the group of auto-antigens set out in Table 2, or auto-antigens that bind to a panel comprising 5 or more, preferably, 6, 7, 8, 9, 10, 11, 12,13,14,15,16,17,18,19,20,21,22,23,24,25,26,27,28,29,30,31,or more or even more preferably to a panel comprising all 32, auto-antigens selected from the group of auto-antigens listed in Table 2.

According to yet another aspect of the present invention there is provided a method of monitoring the efficacy of the therapeutic agent lupus, comprising detecting in a first sample obtained from a patient the presence of one or more auto-antibodies that bind to one or more auto-antigens selected from the group consisting of the auto-antigens in Table 2 or to antigens that bind to a panel comprising 5 or more, preferably 6, 7, 8, 9, 10, 11, 12, 13, 14, 15, 16, 17, 18, 19,20,21,22,23,24,25,26,27,28,29,30,31 or more or even more preferably to a panel comprising all 32, auto-antigens selected from the group of auto-antigens listed in Table 2.

According to yet another aspect of the present invention there is provided a method of predicting whether a patient suffering from lupus will respond to treatment with a therapeutic agent comprising detecting the presence or absence of one or more auto-antibodies that bind to one or more auto-antigens selected from the group consisting of the auto-antigens set out in Table 2, or auto-antibodies that bind to a panel comprising 5 or more, preferably 6, 7, 8, 9, 10,11,12,13,14,15, 16, 17, 18, 19, 20, 21, 22, 23, 24, 25, 26, 27, 28, 29, 30, 31, or more, or preferably to a panel comprising all 32 auto-antigens selected from those auto-antigens listed in Table 2.

As explained above the combined sets of 35 and 32 biomarkers produces 45 biomarkers which have been found to be useful in detecting lupus. These biomarkers are set out in Table 3 below.

**Table 3**

| **Gene symbol** | **Name** | **Synonyms** | **Entrez Gene ID and protein sequence link** |
|---|---|---|---|
| ZMAT2 | Zinc finger, matrin type 2 | FLJ31121 | 153527; http://ca.expasy.org/uni prot/Q96NC0 |
| ASPSCR1 | alveolar soft part sarcoma chromosome region, candidate 1 | TUG; ASPL; ASPS; RCC17; UBXD9;UBXN9; ASPCR1; FLJ45380; ASPSCR1 | 79058; http://ca.expasy.org/uni prot/Q7Z6N7 |
| BANK1 | B-cell scaffold protein with ankyrin repeats 1 | BANK; FLJ20706; FLJ34204 | 55024; http://ca.expasy.org/uni prot/Q8NDB2 |
| PY2IP1 | MAP1S protein | MAP8; C19orf5; VCY2IP1; FLJ10669; VCY2IP-1; MGC133087 | 55201; http://ca.expasy.org/uni prot/Q66K74 |
| CEBPG | CCAA/enhancer binding protein (C/EBP), gamma | GPE1BP; IG/EBP-1; CEBPG | 1054; http://ca.expasy.org/uni prot/P53567 |
| DDX55 | DEAD (Asp-Glu-Ala-Asp) box polypeptide 55 | FLJ16577; KIAA1595; MGC33209; DDX55 | 57696; http://ca.expasy.org/uni prot/Q8NHQ9 |
| DOM3Z | dom-3 homolog Z (C. elegans) | NG6; DOM3L; DOM3Z | 1797; http://ca.expasy.org/uni prot/O7793 |
| E1B-AP5 | E1B-55kDa-associated protein 5 | ELBAP5; E1B-AP5; HNRPUL1;FLJ12944 | 11100; http://ca.expasy.org/uni prot/O76022 |
| FUS | Fusion (involved in t(12;16) in malignant liposarcoma) | TLS; CHOP; FUS1; FUS-CHOP; TLS/CHOP; hnRNP-P2 | 2521; http://ca.expasy.org/uni prot/P35637 |
| HAGH | Hydroxyacylglutathione hydrolase | GLO2; GLX2; GLXII; HAGH1 | 3029; http://ca.expasy.org/uni prot/Q16775 |
| HMG20B | High-mobility group 20B | SOXL; HMGX2; BRAF25; BRAF35; PP7706; pp8857; FLJ26127; SMARCE1r | 10362; http://ca.expasy.org/uni prot/Q9P0W2 |
| HOXB6 | Homeo box B6, transcript variant 2 | HOX2; HU-2; HOX2B; Hox-2.2 | 3216; http://ca.expasy.org/uni prot/P17509 |
| IFI16 | Interferon, gamma-inducible protein 16 | PYHIN2; IFNGIP1; MGC9466 | 3428; http://ca.expasy.org/uni prot/Q16666 |
| KRT8 | Keratin 8 | K8;KO; CK8; CYK8;K2C8; CARD2 | 3856; http://ca.expasy.org/uni prot/P05787 |
| LIN28 | Lin-28 homolog (C. elegans) | CSDD1; LIN-28; LIN28A; ZCCHC1; FLJ12457 | 79727; http://ca.expasy.org/uni prot/Q9H9Z2 |
| LNX | ligand of numb-protein X | LNX; MPDZ; PDZRN2; LNX1 | 84708; http://ca.epasy.org/uni prot/Q8TBB1 |
| NDUFV3 | NADH dehydrogenase (ubiquinone) flavoprotein 3, 10kDa | CI-9KD | 4731; http://ca.expasy.org/uni prot/P56181 |
| PABPC1 | Poly(A) binding protein, cytoplasmic 1 | PAB1; PABP; PABP1; PABPC2; PABPL1 | 26986; http://ca.expasy.org/uni prot/P11940 |
| PHLDA1 | Pleckstrin homology-like domain, family A, member 1 | PHRIP; TDAG51; DT1P1B11; MGC131738 | 22822; http://ca.expasy.org/uni prot/Q8WV24 |
| PIAS2 | Msx-interacting-zinc finger, transcript variant alpha | miz; MIZ1; SIZ2; PIASX; ZMIZ4; MGC102682; PIASX-BETA; PIASX-ALPHA | 9063; http://ca.expasy.org/uni prot/Q13105 |
| PRKCBP1 | protein kinase C binding protein1 protein 1 | RACK7; PRKCBP1; PRO2893; *MGC31836* ZMYND8 | 23613; http://ca.expasy.org/uni prot/Q9H2G5 |
| PRKRA | protein kinase, interferon-inducible double stranded RNA dependent activator | RAX; PACT; DYT16; HSD14; PRKRA | 8575; hftp://ca.expasy.org/uni prot/O75569 |
| PSME3 PSME3 | Proteasome (prosome, macropain) activator subunit 3 (PA28 gamma; Ki) | Ki; PA28G; REG-GAMMA; PA28-gamma | 10197; http://ca.expasy.org/uni prot/Q12920 |
| RAB11FIP 3 | RAB11 family interacting protein 3 (class II) | KIAA0665; Rab11-FIP3 | 9727; http://ca.expasy.org/uni prot/O75154 |
| RALBP1 | RaIA binding protein 1 | RIP; RIP1; RLIP76 | 10928; http://ca.expasy.org/uni prot/Q15311 |
| RAN | RAN, member RAS oncogene family | TC4; Gsp1; ARA24 | 5901; http://ca.expasy.org/uni prot/P17080 |
| RARA | Retinoic acid receptor, alpha | RAR; NR1B1 | 5914; http://ca.expasy.org/uni prot/P10276 |
| RBMS1 | RNA binding motif, single stranded interacting protein 1, transcript variant | YC1; MSSP; SCR2; MSSP-1; MSSP-2; MSSP-3; MGC3331; MGC15146 | 5937; http://ca.expasy.org/uni prot/P29558 |
| RDBP | RD RNA binding protein | RD; RDP; D6S45; NELF-E | 7936; http://ca.expasy.org/uni prot/P18615 |
| RNF12 | Ring finger protein 12, transcript variant 1 | RLIM; MGC15161; NY-REN-43 | 51132; http://ca.expasy.org/uni prot/Q9NVW2 |
| RPL30 | ribosomal protein L30 | RPL30 | 6156; http://ca.expasy.org/uni prot/P04645 |
| RPL31 | ribosomal protein L31 | MGC88191; RPL31 | 6160; http://ca.expasy.org/uni prot/P62899 |
| RUFY1 | RUN and FYVE domain containing 1 | RABIP4; ZFYVE12; FLJ22251 | 80230; http://ca.expasy.org/uni prot/Q96T51 |
| SMN1 | Survival of motor neuron 1, telomeric | SMA; SMN; SMA1; SMA2; SMA3; SMA4; SMA@; SMNT; BCD541; T-BCD541 | 6606; http://ca.expasy.org/uni prot/Q16637 |
| SNK | serum-inducible kinase | SNK; PLK2 | 10769; http://ca.expasy.org/uni prot/Q9NYY3 |
| SRPK1 | SFRS protein kinase 1 | SFRSK1 | 6732; http://ca.expasy.org/uni prot/Q96SB4 |
| SSA2 | Sjogren syndrome antigen A2 (60kDa, ribonucleoprotein autoantigen SS-A/Ro) | RO60; SSA2; TROVE2 | 6738; http://ca.expasy.org/uni prot/P10155 |
| SSNA1 | Sjogren's syndrome nuclear autoantigen 1 | N14; NA14; NA-14 | 8636; http://ca.expasy.org/uni prot/O43805 |
| STAU | Staufen, RNA binding protein (Drosophila), transcript variant T3 | STAU; FLJ25010 | 6780; http://ca.expasy.org/uni prot/O95793 |
| STK11 | Serine/threonine kinase 11 (Peutz-Jeghers syndrome) | PJS; LKB1 | 6794; http://ca.expasy.org/uni prot/Q15831 |
| TOM1 | Target of myb1 (chicken) | FLJ33404 | 10043; http://ca.expasy.org/uni prot/O60784 |
| TXNL2 | Thioredoxin-like, clone MGC:12349 | GRX3; GRX4; GLRX4; PICOT; TXNL2; TXNL3; FLJ11864; bA500G10.4; GLRX3 | 10539; http://ca.expasy.org/uni prot/O76003 |
| TXNRD1 | Thioredoxin reductase 1, transcript variant 5 | TR; TR1; TXNR; TRXR1; GRIM-12; MGC9145 | 7296; http://ca.expasy.org/uni prot/Q16881 |
| VCL | vinculin | MVCL; CMD1W; VCL | 7414; http://ca.expasy.org/uni prot/P18206 |
| ZNF38 | Zinc finger protein 38 | ZNF38; Zipro1; NY-REN-21; DKFZp434L134; DKFZp686H10254 | 7589; http://ca.expasy.org/uni prot/Q9Y5A6 |

Therefore according to the present invention there is provided a method of diagnosing a patient as having or being predisposed to developing lupus, said method comprising detecting in a sample taken from said patient one or more auto-antibodies that bind one or more auto-antigens; wherein the presence of said one or more auto-antibodies, or an increase in the concentration of said one or more auto-antibodies, indicates that the patient suffers or is predisposed to develop lupus, wherein said one or more antigens are selected from the auto-antigens set out in Table 3.

In another aspect of the present invention there is provided a method of diagnosing a patient as having or being predisposed to developing lupus, said method comprising detecting in a sample from said patient auto-antibodies to a panel comprising 5 or more, preferably 6, 7, 8,9, 10, 11, 12, 13, 14, 15, 16, 17, 18, 19, 20, 21, 22, 23, 24, 25, 26, 27, 28,29,30,31,33,34, 35,36,37,38,39,40,41,42,43,44,or more, even more preferably to a panel comprising all 45 auto-antigens selected from the group consisting of those auto-antigens listed in Table 3.

According to yet another aspect of the invention there is provided a method of monitoring the progression or regression of lupus in a patient, said method comprising detecting in a first sample obtained from a patient one or more auto-antibodies that bind one or more auto-antigens selected from the group of auto-antigens set out in Table 2, or auto-antigens that bind to a panel comprising 5 or more, preferably, 6, 7, 8, 9,10,11, 12, 13, 14, 15, 16, 17, 18, 19,20,21,22,23,24,25,26,27,28,29,30,31,32,33,34, 35, 36, 37, 38, 39, 40, 41, 42, 43, 44, or 45 or more, auto-antigens selected from the auto-antigens listed in Table 3.

According to yet another aspect of the present invention there is provided a method of monitoring the efficacy of the therapeutic agent lupus, comprising detecting in a first sample obtained from a patient the presence of one or more auto-antibodies that bind to one or more auto-antigens selected from the group consisting of the auto-antigens in Table 3 or to antigens that bind to a panel comprising 5 or more, preferably 6, 7, 8, 9, 10,11,12,13,14,15,16,17,18,19,20,21,22,23,24,25,26,27,28,29,30,31,32, 33, 34, 35, 36, 37, 38, 39, 40, 41, 42, 43, 44, or more or even more preferably to a panel comprising all 45 auto-antigens selected from the auto-antigens listed in Table 3.

According to yet another aspect of the present invention there is provided a method of predicting whether a patient suffering from lupus will respond to treatment with a therapeutic agent comprising detecting the presence or absence of one or more auto-antibodies that bind to one or more auto-antigens selected from the group consisting of the auto-antigens set out in Table 2, or auto-antibodies that bind to a panel comprising 5 or more, preferably 6, 7,8,9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44 or more or preferably to a panel comprising all 45 auto-antigens selected from those auto-antigens listed in Table 3.

Comparison of the set 35 biomarkers in Table 1 with the set of 32 biomarkers in Table 2 reveals a group of shared biomarkers or biomarkers in common.

The shared biomarkers are set out in Table 4, whilst the unshared biomarkers, or those biomarkers unique to each analysis methodology, are set out in Table 5.

**Table 4**

| **Gene symbol** | **Name** | **Synonyms** | **Entrez Gene ID and protein sequence link** |
|---|---|---|---|
| ZMAT2 | Zinc finger, matrin type 2 | FLJ31121 | 153527; http://ca.expasy.org/uni prot/Q96NC0 |
| BANK1 | B-cell scaffold protein with ankyrin repeats 1 | BANK; FLJ20106; FLJ34204 | 55024; http://ca.expasy.org/uni prot/Q8NDB2 |
| BPY2IP1 MAP1S | MAP1S protein | MAP8; C19orf5; VCY2IP1; FLJ10669; VCY21P-1; MGC133087 | 55201; http://ca.expasy.org/uni prot/Q66K74 |
| CEBPG | CCAAT/enhancer binding protein (C/EBP), gamma | GPE1BP; IG/EBP-1; CEBPG | 1054; http://ca.expasy.org/uni prot/P53567 |
| E1B-AP5 | E1B-55kDa-associated protein 5 | E1BAP5; E1B-AP5; HNRPUL1; FLJ12944 | 11100; http://ca.expasy.org/uni prot/O76022 |
| FUS | Fusion (involved in t(12;16) in malignant liposarcoma) | TLS; CHOP; FUS1; FUS-CHOP; TLS/CHOP; hnRNP-P2 | 2521; http://ca.expasy.org/uni prot/P35637 |
| HMG20B | High-mobility group 20B | SOXL; HMGX2; BRAF25; BRAF35; PP7706; pp8857; FLJ26127; SMARCE1r | 10362; http://ca.expasy.org/uni prot/Q9POW2 |
| IFI16 | Interferon, gamma-inducible protein 16 | PYHIN2; IFNGIP1; MGC9466 | 3428; http://ca.expasy.org/uni prot/Q16666 |
| KRT8 | Keratin 8 | K8; KO; CK8; CYK8; K2C8; CARD2 | 3856; http://ca.expasy.org/uni prot/P05787 |
| LIN28 | Lin-28 homolog (C. elegans) | CSDD1; LIN-28; LIN28A; ZCCHC1; FLJ12457 | 79727; http://ca.expasy.org/uni prot/Q9H9Z2 |
| PABPC1 | Poly(A) binding protein, cytoplasmic 1 | PAB1; PABP; PABP1; PABPC2; PABPL1 | 26986; http://ca.expasy.org/uni prot/P11940 |
| PHLDA1 | Pleckstrin homology-like domain, family A, member 1 | PHRIP; TDAG51; DT1P1B11: MGC131738 | 22822; http://ca.expasy.org/uni prot/Q8W V24 |
| PIAS2 | Msx-interacting-zinc finger, transcript variant alpha | miz; MIZ1; SIZ2; PIASX; ZMIZ4; MGC102682; PIASX-BETA; PIASX-ALPHA | 9063; http://ca.expasy.org/uni prot/Q13105 |
| PSME3 | Proteasome (prosome, macropain) activator subunit 3 (PA28 gamma; Ki) | Ki; PA28G; REG-GAMMA; PA28-gamma | 10197; http://ca.expasy,org/uni prot/Q12920 |
| RALBP1 | RaIA binding protein 1 | RIP; RIP1; RLIP76 | 10928; http://ca.expasy.org/uni prot/Q15311 |
| RARA | Retinoic acid receptor, alpha | RAR; NR1B1 | 5914; http://ca.expasy.org/uni prot/P10276 |
| RDBP | RD RNA binding protein | RD; RDP; D6S45; NELF-E | 7936; http://ca.expasy.org/uni prot/P18615 |
| SRPK1 | SFRS protein kinase 1 | SFRSK1 | 6732; http://ca.expasy.org/uni prot/Q96SEi4 |
| SSA2 | Sjogren syndrome antigen A2(60kDa, ribonucleoprotein autoantigen SS-A/Ro) | RO60; SSA2; TROVE2 | 6738; http://ca.expasy.org/uni prot/P10155 |
| STAU | Staufen, RNA binding protein (Drosophila), transcript variant T3 | STAU; FLJ25010 | 6780; http://ca.expasy.org/uni prot/O95793 |
| TXNL2 | Thioredoxin-like, clone MGC:12349 | GRX3; GRX4; GLRX4; PICOT; TXNL2; TXNL3; FLJ11864; bA500G10.4; GLRX3 | 10539; http://ca.expasy.org/uni prot/O76003 |
| ZNF38 | Zinc finger protein 38 | ZNF38; Zipro1; NY-REN-21; DKFZp434L134; DKFZp686H10254 | 7589; http://ca.expasy.org/uni prot/Q9Y5A6 |

**Table 5**

| Gene symbol | Name | Synonyms | Entrez Gene ID and protein sequence link |
|---|---|---|---|
| ASPSCR1 | alveolar soft part sarcoma chromosome region, candidate 1 | TUG; ASPL; ASPS; RCC17; UBXD9; UBXN9; ASPCR1; FLJ45380; ASPSCR1 | 79058; http://ca.expasy.org/uni prot/Q7Z6N7 |
| DDX55 | DEAD (Asp-Glu-Ala-Asp) box polypeptide 55 | FLJ16577; KIAA1595; MGC33209: DDX55 | 57696; http://ca.expasy.org/uni prot/Q8NHO9 |
| DOM3Z | dom-3 homolog Z (C. elegans) | NG6; DOM3L; DOM3Z | 1797; http://ca.expasy.org/uni prot/O77832 |
| HAGH | Hydroxyacylglutathione hydrolase | GLO2; GLX2; GLXll; HAGH1 | 3029; http://ca.expasy.org/uni prot/Q16775 |
| HOXB6 | Homeo box B6, transcript variant 2 | HOX2; HU-2; HOX2B; Hox-2.2 | 3216; http://ca.expasy.org/uni prot/P17509 |
| LNX | ligand of numb-protein X | LNX; MPDZ; PDZRN2; LNX1 | 84708; http://ca.expasy.org/uni prot/Q8TBB1 |
| NDUFV3 | NADH dehydrogenase (ubiquinone) flavoprotein 3, 10kDa | Cl-9KD | 4731; http://ca.expasy.org/uni prot/P56181 |
| PRKCBP1 | protein kinase C binding protein 1 | RACK7; PRKCBP1; PRO2893; MGC31836; ZMYND8 | 23613; http://ca.expasy.org/uni prot/Q9H2G5 |
| PRKRA | protein kinase, interferon-inducible double stranded RNA dependent activator | RAX; PACT; DYT16; HSD14; PRKRA | 8575; http://ca.expasy.org/uni prot/O75589 |
| RAB11FIP 3 | RAB11 family interacting protein 3 (class II) | KIAA0665; Rab11-FlP3 | 9727; http://ca.expasy.org/uni prot/O75154 |
| RAN | RAN, member RAS oncogene family | TC4; Gsp1; ARA24 | 5901; http://ca.expasy.org/uni prot/P17080 |
| RBMS1 | RNA binding motif, single stranded interacting protein 1, transcript variant | YC1; MSSP; SCR2; MSSP-1; MSSP-2; MSSP-3; MGC3331; MGC15146 | 5937; http://ca.expasy.org/uni prot/P29558 |
| RNF12 | Ring finger protein 12, transcript variant 1 | RLIM; MGC15161; NY-REN-43 | 51132; http://ca.expasy.org/uni prot/Q9NVW2 |
| RPL30 | ribosomal protein L30 | RPL30 | 6156; http://ca.expasy.org/uni prot/PQ4645 |
| RPL31 | ribosomal protein L31 | MGC88191; RPL31 | 6160; http://ca.expasy.org/uni prot/P62899 |
| RUFY1 | RUN and FYVE domain containing 1 | RABlP4; ZFYVE12; FLJ22251 | 80230; http://ca.expasy.org/uni prot/Q96T51 |
| SMN1 | Survival of motor neuron 1, telomeric | SMA; SMN; SMA1; SMA2; SMA3; SMA4; SMA@; SMNT; BCD541; T-BCD541 | 6606; http://ca.expasy.org/uni prot/Q16637 |
| SNK | serum-inducible kinase | SNK; PLK2 | 10769; http://ca.expasy.org/uni prot/Q9NYY3 |
| SSNA1 | Sjogren's syndrome nuclear autoantigen 1 | N14; NA14; NA-14 | 8636; http://ca.expasy.org/uni prot/O43805 |
| STK11 | Serine/threonine kinase 11 (Peutz-Jeghers syndrome) | PJS;LKB1 | 6794; http://ca.expasy.org/uni prot/Q15831 |
| TOM1 | Target of myb1 (chicken) | FLJ33404 | 10043; http://ca.expasy.org/uni prot/O60784 |
| TXNRD1 | Thioredoxin reductase 1, transcript variant 5 | TR; TR1; TXNR; TRXR1; GRIM-12; MGC9145 | 7296; http://ca.expasy.org/uni prot/Q16881 |
| VCL | vinculin | MVCL; CMD1W; VCL | 7414; http://ca.expasy.org/uni prot/P18206 |

In other embodiments, a method is provided which is useful in diagnosing a patient as having or being predisposed to developing lupus, comprising testing a sample from the patient against a panel of auto-antigens to detect the presence, absence or a modulated level, of auto-antibodies in the sample, or in the panel of auto-antigens comprises at least 10 auto-antigens selected from Table 3. In other embodiments, the at least 10 auto-antigens may be selected from Table 1, or from Table 2. Preferably a panel comprises at least 20 auto-antigens. Optionally a panel may comprise 11, 12, 13, 14, 15, 16, 17, 18, 19, 20, 21, 22, 23, 24, 25, 26, 27, 28, 29 or 30 or more auto-antigens.

The panel of auto-antigens useful in the diagnosis of lupus may comprise the auto-antigens set out in Table 1, or in Table 2, or in Table 3. In some embodiments the panel may have up to 5 auto-antigens deleted or replaced, or optionally up to 6, 7, 8, 9 or 10 or more auto-antigens deleted or replaced provided the required specificity and selectivity of the method is retained.

It is expected that a clinically valuable panel of biomarkers should be large enough to allow a disease to be detected accurately, minimizing false positives and false negatives, whilst also being small enough to be practical.

In embodiments the methods of the present invention use a panel of auto-antigens comprising the auto-antigens listed in Table 4. Such a method may use a panel comprising 1 or more additional auto-antigens selected from the group consisting of the auto-antigens of Table 5.

In the methods of the present invention detecting the presence or an increased level of at least 2 auto-antibodies provides information useful for diagnosis. Optionally, detecting the presence of an increased level of at least 3, preferably at least 4 and more preferably at least 5 auto-antibodies provides information useful for diagnosis.

By analysing the relationship between the panel of biomarkers and the status of the samples used to derive the panel, it is possible to derive a mathematical relationship between the presence, absence or a modulated level of auto-bodies directed against proteins in the panel and the presence, absence, progression or regression of lupus. The application of this mathematical relationship to naive samples to be tested can be used to derive information useful for diagnosis, progression or regression of lupus.

Where it is not possible to derive such a mathematical relationship, it is possible to derive a statistical relationship between the presence, absence or modulated level of auto-antibodies directed against proteins in the panel and the presence, absence, progression or regression of lupus. The application of this statistical relationship to naive sample to be tested can be used to derive information useful for diagnosis, progression or regression of lupus.

According to a different aspect of the present invention there is provided a diagnostic kit for use in diagnosing a patient as having or being predisposed to developing lupus, or having presymptomatic lupus, said kit comprising one or more auto-antigens selected from the group consisting of ZMAT2 (Zinc finger, matrin type 2); BANK1 (B-cell scaffold protein with ankyrin repeats 1); BPY2IP1 (MAP1S protein); CEBPG (CCAAT/enhancer binding protein (C/EBP), gamma); E1B-AP5 (E1B-55kDa-associated protein 5); FUS (Fusion (involved in t(12;16) in malignant liposarcoma)); HAGH (Hydroxyacylglutathione hydrolase); HMG20B (High-mobility group 20B); HOXB6 (Homeo box B6, transcript variant 2); KRT8 (Keratin 8); LIN28 (Lin-28 homolog (C. elegans)); NDUFV3 (NADH dehydrogenase (ubiquinone) flavoprotein 3, 10kDa); PABPC1 (Poly(A) binding protein, cytoplasmic 1); PHLDA1 (Pleckstrin homology-like domain, family A, member 1); PIAS2 (Msx-interacting-zinc finger, transcript variant alpha); RAB11FIP3 (RAB11 family interacting protein 3 (class II)); RAN (RAN, member RAS oncogene family); RARA (Retinoic acid receptor alpha); RBMS 1 (RNA binding motif, single stranded interacting protein 1, transcript variant); RDBP (RD RNA binding protein); RNF12 (Ring finger protein 12, transcript variant 1); RUFY1 (RUN and FYVE domain containing 1); SMN1 (Survival of motor neuron 1, telomeric); SRPK1 (SFRS protein kinase 1); SSNA1 (Sjogren's syndrome nuclear autoantigen 1); STAU (Staufen, RNA binding protein (Drosophila), transcript variant T3); STK11₋(Serine/threonine kinase 11 (Peutz-Jeghers syndrome)); TOM1 (Target of myb1 (chicken)); TXNL2 (Thioredoxin-like, clone MGC:12349); TXNRD1 (Thioredoxin reductase 1, transcript variant 5); or ZNF38 (Zinc finger protein 38), which one or more auto-antigens are immobilised on a substrate.

In one embodiment, the one or more auto-antigens includes ZMAT2.

In addition to one or more of the auto-antigens above, the kit may comprise one or more of the auto-antigens BANK1 (B-cell scaffold protein with ankyrin repeats 1); IFI16 (Interferon, gamma-inducible protein 16); PSME3 (Proteasome (prosome, macropain) activator subunit 3 (PA28 gamma; Ki), transc); RALBP1 (Ra1A-binding protein 1); and SSA2 (Sjogren syndrome antigen A2 (60kDa, ribonucleoprotein autoantigen SS-A/Ro)).

Alternatively, said kit may comprise a panel comprising 5 or more, preferably 6, 7, 8,9,10,11,12,13,14,15,16,17,18,19,20,21,22,23,24,25,26,27,28,29,30,31, 32, 33, 34 or more, even more preferably to a panel comprising all 35, auto-antigens selected from the auto-antigens listed in Table 1, wherein the auto-antigens are immobilised on a substrate.

According to another aspect of the present invention a diagnostic kit is provided for use in diagnosing a patient as having or being predisposed to developing lupus, or having pre-symptomatic lupus, said kit comprising one or more auto-antigens selected from the group of auto-antigens of Table 2.

Alternatively, said kit may comprise a panel comprising 5 or more, preferably 6, 7, 8, 9, 10, 11, 12, 13, 14, 15, 16, 17, 18, 19,20,21,22,23,24,25,26,27,28,29,30,31 or more, even more preferably to a panel comprising all 32 auto-antigens selected from the auto-antigens listed in Table 2, wherein the auto-antigens are immobilised on a substrate.

According to another aspect of the present invention there is provided a diagnostic kit for use in diagnosing a patient as having or being predisposed to developing lupus, or having pre-symptomatic lupus, said kit comprising 1 or more auto-antigens selected from the group of auto-antigens of Table 3.

Alternatively said kit may comprise a panel comprising 5 or more, preferably, 6, 7, 8, 9,10,11, 12, 13, 14, 15,16, 17, 18, 19, 20, 21, 22, 23, 24, 25, 26, 27, 28, 29, 30, 31, 32, 33, 34, 35, 36, 37, 38, 39, 40, 41, 42, 43, 44 or more, even more preferably to a panel comprising all 45 auto-antigens selected from the auto-antigens listed in Table 3, wherein the auto-antigens are immobilised on a substrate.

In some embodiments, said substrate may comprise a strip, a slide, beads or a well of a microtitre plate or a "chip" for use with Surface Plasmon Resonance (Biacore) or planar waveguide technology. Suitably, the substrate may comprise a disposable test strip of the kind widely used in diagnostic kits for use at the point of care.

The panel of auto-antigens may be carried on said substrate in the form of an addressable array. Said array may be a microarray. The auto-antigens of the panel may constitute at least 5%, 10%, 15%, 20%, 25%, 40% 50%, typically at least 70% or 80%, of the total number of proteins present on the array. For example, the auto-antigens of the panel may constitute more than 85%, 90% or 95% of the total number of proteins on the array.

Alternatively, said diagnostic kit may comprise said panel of auto-antigens carried on a substrate in the form of an array, which array includes one or more other proteins or polypeptides, and means for automatically analysing the results of tests performed using said array, said analysing means including computer-readable instructions in which results from said one or more other proteins or polypeptides are blinded during the analysis.

In addition to the auto-antigens (and optional one or more other proteins or polypeptides) forming said panel, the array may further comprise one or more control proteins or polypeptides. Suitably, the array may comprise one or more replicates of the auto-antigens or controls, preferably replicates of each auto-antigen and each control.

Advantageously, the array comprises an array of correctly folded auto-antigens, which may be prepared in accordance with the methods disclosed by WO-A-01/57198 or WO-A-02/27327, for example, the contents of which are incorporated herein by reference. Preferably, the array comprises an array of correctly folded tagged auto-antigens that are attached to said substrate via the tag, which array may be prepared, for example, in accordance with the methods disclosed by WO 03/064656 or WO-A-2004/046730, the contents of which are also incorporated herein by reference.

Said kit may further comprise an anti-human immunoglobulin antibody or other protein that specifically binds immunoglobulin, and means for detecting the anti-human immunoglobulin antibody or other immunoglobulin-binding protein.

Said detection means may comprise a colorimetric or fluorescent assay or utilise label-free methods such as surface plasmon resonance, mass spectrometry or planar waveguide technology.

The anti-human immunoglobulin antibody may be an anti-human IgM antibody, an anti-human IgG antibody, an anti-human IgA antibody, an anti-human IgE antibody or a combination of any two or more of such anti-human antibodies; preferably, the anti-human immunoglobulin antibody is an anti-human IgG antibody. Alternatively, or additionally, a protein that specifically binds an antibody is included in the kit. Preferably, this protein is protein A or protein G, or a recombinant form thereof.

According to yet another aspect of the present invention there is provided the use of one or more auto-antigens selected from the aforementioned groups as shown in Tables 1, 2, 3 or 4, or antigenic fragments or antigenic epitopes thereof, or a nucleic acid encoding the auto-antigen or fragment or epitope thereof, as an active agent in the manufacture of a vaccine for the treatment or prevention of lupus. Immunization with such an active agent may be protective against aberrant expression of the said antigen in lupus. In one embodiment the vaccine comprises ZMAT2, optionally in combination with one or more further antigens specified in Tables 1, 2, 3 or 4.

Said active agent may be formulated in a pharmaceutical composition in accordance with method is well known in the art. Said composition may further comprise a pharmaceutically acceptable vehicle. Said vehicle may comprise one or more excipients, carriers, buffers, stabilisers or other materials well known to those skilled in the art. Such materials should be non-toxic and should not interfere with the efficacy of the active agent. The precise nature of the carrier or other material may depend on the route of administration, e.g., oral, intravenous, cutaneous or subcutaneous, nasal, intramuscular, intraperitoneal or patch routes.

Pharmaceutical compositions for oral administration may be in tablet, capsule, powder or liquid form. A tablet may include a solid carrier such as gelatin or an adjuvant. Liquid pharmaceutical compositions generally include a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included.

For intravenous, cutaneous or subcutaneous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers, antioxidants and/or other additives may be included, as required.

Whether it is a polypeptide, peptide, or nucleic acid molecule, other pharmaceutically useful compound according to the present invention that is to be given to an individual, administration is preferably in a "prophylactically effective amount" or a "therapeutically effective amount" (as the case may be, although prophylaxis may be considered therapy), this being sufficient to show benefit to the individual. The actual amount administered, and rate and time-course of administration, will depend on the nature and severity of what is being treated. Prescription of treatment, e.g. decisions on dosage etc, is within the responsibility of general practitioners and other medical doctors, and typically takes account of the disorder to be treated, the condition of the individual patient, the site of delivery, the method of administration and other factors known to practitioners. Examples of the techniques and protocols mentioned above can be found in REMINGTON'S PHARMACEUTICAL SCIENCES, 16th edition, Osol, A. (ed), 1980.

Alternatively, targeting therapies may be used to deliver the active agent more specifically to certain types of cell, by the use of targeting systems such as antibody or cell specific ligands. Targeting may be desirable for a variety of reasons; for example if the agent is unacceptably toxic, or if it would otherwise require too high a dosage, or if it would not otherwise be able to enter the target cells.

Instead of administering these agents directly, they could be produced in the target cells by expression from an encoding gene introduced into the cells, e.g. in a viral vector (a variant of the VDEPT technique - *see* below). The vector could be targeted to the specific cells to be treated, or it could contain regulatory elements, which are switched on more or less selectively by the target cells.

Alternatively, the agent could be administered in a precursor form, for conversion to the active form by an activating agent produced in, or targeted to, the cells to be treated. This type of approach is sometimes known as ADEPT or VDEPT; the former involving targeting the activating agent to the cells by conjugation to a cell-specific antibody, while the latter involves producing the activating agent, e.g. a vaccine or fusion protein, in a vector by expression from encoding DNA in a viral vector *(see* for example, EP-A-415731 and WO 90/07936).

The vaccines of the present invention also include one or more adjuvant compounds, i.e. a compound or compounds that increases an immunogenic response or the immunogenicity of an antigen or vaccine. Adjuvant compounds useful in the present invention include Complete Freund's Adjuvant (CFA); Incomplete Freund's Adjuvant (IFA); Montanide ISA (incomplete seppic adjuvant); Ribi Adjuvant System (RAS); TiterMax; Syntex Adjuvant Formulation (SAF); Aluminum Salt Adjuvants; Nitrocellulose-adsorbed antigen; Encapsulated or entrapped antigens; Immune-stimulating complexes (ISCOMs); and Gerbu^{R} adjuvant.

Following is a description by way of example only with reference to the accompanying drawings of embodiments of the present invention.

In the drawings, **Figure 1** is a cartoon illustrating a method for the detection of auto-antibodies in serum.

### Examples

### Example 1: A microarray of antigens for discovering auto-antibody biomarkers in patient serum that are diagnostic for lupus

Full-length open reading frames for 330 target genes covering a wide range of protein classes were cloned in-frame with a sequence encoding a C-terminal E. *coli* BCCP-myc tag (WO 03/064656; Boutell) in a baculovirus transfer vector and sequence-verified. Recombinant baculoviruses were generated, amplified and expressed in Sf9 cells grown in suspension using standard methods adapted for 24-well deep well plates (Whatman, Maidstone, UK; Chambers, Zhao). Recombinant protein expression was analyzed for protein integrity and biotinylation by Western blotting. Cells harboring recombinant protein were lysed and lysates were spotted in quadruplicate using a QArray2 Microarrayer (Genetix, New Milton, UK) equipped with 300 µm solid pins on to streptavidin-coated glass slides (Schott Nexterion, Jena, Germany). Slides were stored at -20°C. A range of protein activities have been demonstrated to be stable under these conditions for over 12 months. Proteins spotted on the array project into an aqueous environment and orient away from the surface of the glass chips, exposing them for binding by auto-antibodies.

In addition to the 330 proteins, four control proteins for the BCCP-myc tag (BCCP, BCCP-myc, β-galactosidase-BCCP-myc and β-galactosidase-BCCP) were arrayed, along with Cy3/Cy5-labeled biotin-BSA, dilution series of biotinylated-IgG and biotinylated IgM, a biotinylated-myc peptide dilution series and buffer only spots.

### Example 2: Detection of auto-antibodies in serum samples from patients suffering from lupus

Serum samples of 169 individuals diagnosed with lupus (median age 47 years, 95% females) were obtained from 2 commercial sources. As controls, samples of 170 individuals with no known history of lupus (median age 50 years, 95% females) were obtained. These samples were assayed for the presence of auto-antibodies using the protein arrays described in Example 1.

Serum samples were clarified (centrifuged at 10-13K rcf for 3 minutes at 4°C to remove particulates, including lipids) and diluted 200-fold in 0.1 % v/v Triton/0. 1% w/v BSA in 1X PBS (Triton-BSA-PBS buffer) and then applied to the arrays. Each diluted serum sample (2.0 mL) was applied to a separate array and incubated in a dish for 2 hours at room temperature (RT, 20°C) with gentle orbital shaking (~50 rpm). Arrays were then carefully removed from the dish and any excess probing solution was removed by blotting the sides of the array onto lint-free tissue. Probed arrays were washed twice in fresh Triton-BSA-PBS buffer in a new plastic container with screw-on lid at room temperature for 5 minutes with rapid orbital shaking. The washed slides were then blotted onto lint-free tissue to remove excess wash buffer and were incubated in a secondary antibody solution (prepared just prior to use) at room temperature for 2 hours, with gentle orbital shaking and protected from light. The secondary antibody solution was a Cy3 labeled rabbit anti-human IgG antibody diluted in Triton-BSA-PBS buffer to give optimal detection of bound serum IgG. The slides were washed three times in Triton-BSA-PBS buffer for 5 minutes at RT with gentle orbital shaking, rinsed briefly (5-10 seconds) in distilled water, and centrifuged for 2 minutes at 240 rcf in a container suitable for centrifugation. To help wick away excess liquid on the arrays, a lint-free tissue was placed at the bottom of the arrays during centrifugation.

The probed and dried arrays were then scanned using a microarray scanner capable of using an excitation wavelength suitable for the detection of the secondary staining solution, such as the Molecular Devices Genepix 4000B microarray scanner, to identify auto-antibodies bound by the array and to determine intensity of auto-antibody binding. The microarray scans produced TIFF images for each array that were used to determine the intensity of fluorescence bound to each protein spot.

Raw median signal intensity (also referred to as the relative fluorescent unit, RFU) of each protein feature (also referred to as a spot or antigen) on the array was determined from the TIFF images using GenePix Pro microarray data analysis software or similar software and subtracted from the local median background intensity. It is also possible to use other measures of spot intensity such as the mean fluorescence, total fluorescence, as is known by anyone skilled in the art.

The resulting net fluorescent intensities of all protein features on each array were then normalized using the quantile normalization (Bolstad) method. Data for sera from two lupus patients and one control serum were excluded because many of their antigen binding signals were masked by high background binding. Other methods for data normalization suitable for the current data include, amongst others, multiplication of net fluorescent intensities by a normalization factor consisting of the product of the 1^{st} quartile of all intensities of a sample and the mean of the 1^{st} quartiles of all samples and the vsn method (Huber). Such normalization methods are known to anyone skilled in the art of microarray analysis. The normalized fluorescent intensities were then averaged for each protein feature.

The normalized mean data were used to select biomarkers for lupus. The data were randomly split into training and test sets 10 times (10 fold cross-validation)

Proteins were selected and ranked with the significance analysis of microarrays (SAM) algorithm (Tusher) using the data in the training subset. Similar methods that are applicable to this task include, amongst others, Prediction Analysis of Microarrays (PAM, Tibshirani), MP test (Fox), fold change (Witten) and principal component analysis (Bessant). Such data analysis methods are known to anyone skilled in the art of microarray analysis. The data were analysed as two class ―unpaired (diseased vs control) using a non-parametric test statistic. The protein features selected in each cross-validation fold with the highest scores and a q-value of ≤30°lo were selected and those with q-values ≤30% in most of the 10 cross-validation iterations were selected and are shown in Table 2 alongside the frequency of selection in cross-validation.

**Table la lists the protein features for which the q-value was ≤30% by SAM analysis alongside the selection frequency in cross-validation**

| Gene symbol | cross-validation selection frequency |
|---|---|
| ZNF38 | 10 |
| BPY21P1 | 10 |
| CEBPG | 10 |
| E1B-AP5 | 10 |
| FUS | 10 |
| HMG20B | 10 |
| LIN28 | 10 |
| NDUFV3 | 10 |
| PABPC1 | 10 |
| PHLDA1 | 10 |
| PIAS2 | 10 |
| PSME3 | 10 |
| RAB11FIP3 | 10 |
| RALBP1 | 10 |
| RAN | 10 |
| RARA | 10 |
| RBMS1 | 10 |
| RNF12 | 10 |
| SSA2 | 10 |
| STAU | 10 |
| STK11 | 10 |
| TOM1 | 10 |
| ZMAT2 | 10 |
| SRPK1 | 9 |
| TXNRD1 | 9 |
| BANK1 | 8 |
| IFI16 | 8 |
| KRT8 | 8 |
| RUFY1 | 8 |
| SMN1 | 8 |
| SSNA1 | 8 |
| HAGH | 7 |
| HOXB6 | 7 |
| RDBP | 7 |
| TXNL2 | 7 |

The proteins listed in Table 1a are more frequently bound by sera from diseased than control subjects. The proteins listed in Table 1a are considered as biomarkers useful in diagnosis of lupus.

The normalized data of the training set for the proteins selected in each cross-validation fold were used to construct a classification algorithm. An algorithm was established based on using logistic regression (Liao) algorithms. Similar methods that can be used to construct classification algorithms for the sample data of this study include, amongst others, PAM (Tibshirani), artificial neural networks (Bessant), support vector machine (SVM) (Brown) and regularized discriminant analysis (Guo). Such data analysis methods are known to anyone skilled in the art of microarray analysis. The constructed algorithm was then used to classify the samples of the test set into diseased and control samples in order to check its validity as classification method for lupus. In order to get an estimate of the classification errors the mean specificity and sensitivity were examined using 10 fold cross validation. The cross validation mean sensitivity was 67 %, the mean specificity was 85 %. The cross validation approach ensured robust measurements of classification errors.

### Example 3: Detection of auto-antibodies in serum samples from patients suffering from lupus using a modified analysis approach

The raw data (169 lupus and 170 controls samples) as described in Example 2 was subjected to modified analysis in order to mine the data still further and identify additional markers indicative of lupus.

Raw median signal intensity (relative fluorescent unit, RFU) of each protein feature on the array was determined from the TIFF images using ArrayPro microarray data analysis software and subtracted from the local median background intensity.

The resulting net fluorescent intensities of all protein features from all three array types were then normalized together using the vsn normalization method (Huber 2002), or multiplication of net fluorescent intensities by a normalization factor consisting of the product of the 1st quartile of all intensities of a sample and the mean of the 1st quartiles of all samples, or the quantile or robust quantile method (Bolstad 2003). The normalized fluorescent intensities were then averaged for each protein feature.

The normalized mean data were used to select biomarkers for lupus. In order to estimate the specificity and sensitivity of a classification method based on biomarkers selected, data were initially split randomly into training and test sets 10 times (10 fold cross-validation).

Proteins were selected and ranked with the MP test (Fox 2006), or Prediction Analysis of Microarrays (PAM, Tibshirani 2002), significance analysis of microarrays (SAM) algorithm (Tusher 2001, Hueber 2005), fold change (Witten 2007), or a cut-off method based on a confidence interval derived from the data of the controls, using the data in the training subset. The data were analysed as two class (diseased vs control).

The normalized data of the training set for the proteins selected in each cross-validation fold were used to construct a classification algorithm. Features derived from the same clones that were selected on more than one array type were left in for the classification algorithm construction. An algorithm was established based on penalised logistic regression (Antoniadis 2003, Liao 2007), or PAM (Tibshirani 2002), or support vector machine (SVM, Brown 2000, Statnikov 2008) or a cut-off method based on a confidence interval derived from the data of the controls. The constructed algorithms were then used to classify the samples of the test set into diseased and control samples in order to check its validity as classification method for lupus. In order to get an estimate of the classification errors the mean specificity and sensitivity were examined using 10 fold cross validation.

The proteins selected by SAM analysis in at least 9 of the 10 cross validation folds are shown in Table 2a. The analysis parameters used were: q-value cut-off ≤20%and proteins with elevated responses included.

**Table 2a lists the protein features for which the q-value was ≤20% by SAM analysis and 10 fold cross validation selection frequency ≥9.**

| Gene symbol | Selection frequency |
|---|---|
| ASPSCR1 | 10 |
| BANK1 | 10 |
| CEBPG | 10 |
| DDX55 | 10 |
| DOM3Z | 9 |
| E1B-AP5 | 10 |
| FUS | 10 |
| HMG20B | 10 |
| IFI16 | 10 |
| KRT8 | 10 |
| LIN28 | 10 |
| LNX | 10 |
| MAP1S | 10 |
| PABPC1 | 10 |
| PHLDA1 | 10 |
| PIAS2 | 10 |
| PRKCBP1 | 10 |
| PRKRA | 10 |
| PSME3 | 10 |
| RALBP1 | 10 |
| RARA | 10 |
| RDBP | 10 |
| RPL31 | 9 |
| RPL30 | 10 |
| SNK | 10 |
| SRPK1 | 10 |
| SSA2 | 10 |
| STAU1 | 10 |
| TXNL2 | 10 |
| VCL | 10 |
| ZMAT2 | 9 |
| ZNF38 | 9 |

The proteins listed in Table 2a are more frequently bound by sera from diseased than control subjects. The proteins listed in Table 2a are considered as biomarkers useful in diagnosis of lupus.

Classification by SVM gave a cross validation mean sensitivity of 74 % and a mean specificity of 86 %.

### References

Antoniadis, A. Penalized Logistic Regression and Classification of Microarray Data. Bioconductor Workshop Milan 2003 (http://www.bioconductor.org/workshops/2003/Milan/Lectures/ anestisMilan3.pdf).
Bessant, C. Microarray Analysis Software and its Applications, in The Handbook of Biosensors and Biochips (Eds. R. Marks, D. Cullen, I. Karubc, H. Weetall, C. Lowe), Wiley, 2007.
Bolstad BM, Irizarry RA, Astrand M, Speed TP. A comparison of normalization methods for high density oligonucleotide array data based on variance and bias. Bioinformatics. 2003 Jan 22;19(2):185-93.
Boutell, J.M., Hart, D.J., Godber, B.L., Kozlowski, R.Z. and Blackburn, J.M. Functional protein microarrays for parallel characterisation of p53 mutants. Proteomics 2004 4(7), 1950-1958
Brown MP, Grundy WN, Lin D, Cristianini N, Sugnet CW, Furey TS, Ares M Jr, Haussler D. Knowledge-based analysis of microarray gene expression data by using support vector machines. Proc Natl Acad Sci U S A. 2000 Jan 4;97(1):262-7.
Chambers S.P., Austen D.A., Fulghum J.R., Kim W.M. High-throughput screening for soluble recombinant expressed kinases in Escherichia coli and insect cells. Protein Expr Purif. 2004 36(1):40-7
Fox RJ, Dimmic MW. A two-sample Bayesian t-test for microarray data.BMC Bioinformatics. 2006 Mar 10;7:126.
Guo, Y. et al. (2004) Regularized Discriminant Analysis and Its Application in Microarrays, Technical Report, Department of Statistics, Stanford University.
Huber W, von Heydebreck A, Scultmann H, Poustka A, and Vingron M. Variance stablization applied to microarray data calibration and to quantification of differential expression. Bioinformatics, 18:S96-S104, 2002.
Liao JG, Chin KV. Logistic regression for disease classification using microarray data: model selection in a large p and small n case. Bioinformatics. 2007 Aug 1;23(15):1945-51.
Statnikov A, Wang L, Aliferis CF. A comprehensive comparison of random forests and support vector machines for microarray-based cancer classification. BMC Bioinformatics. 2008 Jul 22;9:319.
Tibshirani R, Hastie T, Narasimhan B, Chu G. Diagnosis of multiple cancer types by shrunken centroids of gene expression. Proc Natl Acad Sci U S A. 2002 May 14;99(10):6567-72.
Tusher, Tibshirani and Chu (2001). Significance analysis of microarrays applied to the ionizing radiation response. PNAS 2001 98: 5116-5121.
Witten, D. and Tibshirani, R. A comparison of fold change and the t-statistic for microarray data analysis. Technical Report, Stanford University, 2007.
Zhao Y, Chapman DA, Jones IM. Improving baculovirus recombination. Nucleic Acids Res. 20033 1 (2):E6-6

### Gene symbol, Entrez Gene ID, DNA Accession number and sequence of antigens.

| | |
|---|---|
| Gene symbol: | ASPSCR1 |
| Entrez Gene ID: | 79058 |
| dna/ac: | BC018722.1 |
| sequence: (SEQ ID NO: 1) | |
| atggcggccccggcaggcggcggaggctccgcggtgtcggtgctggccccgaacggccggcgccacacggt gaaggtgacgccgagcaccgtgctgcttcaggttctggaggacacgtgccggcggcaggacttcaacccctgtgaatatga tctgaagtttcagaggagcgtgctcgacctttctctccagtggagatttgccaacctgcccaacaatgccaagctggagatggt gcccgcttcccggagccgtgaggggcctgagaacatggttcgcatcgctttgcagctggacgatggctcgaggttgcagga ctctttctgttcaggccagaccctctgggagcttctcagccattttccacagatcagggagtgcctgcagcaccccggcgggg ccacccccagtctgcgtgtacacgagggatgaggtgacgggtgaagctgccctgcggggcacgacgctgcagtcgctggg cctgaccgggggcagcgccaccatcaggtttgtcatgaagtgctacgaccccgtgggcaagaccccaggaagcctgggct cgtcagcgtcggctggccaggcagccgccagcgctccacttcccttggaatctggggagctcagccgcggcgacttgagc cgtccggaggacgcggacacctcagggccctgctgcgagcacactcaggagaagcagagcacaagggcacccgcagct gcccccttttgttcctttctcgggtgggggacagagacagggggggccctcctgggcccacgaggcctctgacatcatcttcag ctaagttgccgaagtccctctccagccctggaggcccctccaagccaaagaagtccaagtcgggccaggatccccagcag gagcaggagcaggagcgggagcgggatccccagcaggagcaggagcgggagcggcccgtggaccgggagcccgtg gaccgggagccggtggtgtgccaccccgacctggaggagcggctgcaggcctggccagcggagctgcctgatgagttctt tgagctgacggtggacgacgtgagaagacgcttggcccagctcaagagtgagcggaagcgcctggaagaagccccttg gtgaccaaggccttcagggaggcgcagataaaggagaagctggagcgctacccaaaggtggctctgagggtcctgttccc cgaccgctacgtcctacagggcttcttccgccccagcgagacagtgggggacttgcgagacttcgtgaggagccacctggg gaaccccgagctgtcattttacctgttcatcacccctccaaaaacagtcctggacgaccacacgcagaccctctttcaggcga acctcttcccggccgctctggtgcacttgggagccgaggagccggcaggtgtctacctggagcctggcctgctggagcatg ccatctccccatctgcggccgacgtgctggtggccaggtacatgtccagggccgccgggtccccttccccattgccagcccc tgaccctgcacctaagtctgagccagctgctgaggagggggcgctggtcccccctgagcccatcccagggacggcccagc ccgtgaagaggagcctgggcaaggtgcccaagtggctgaagctgccggccagcaagaggtga | |
| symbol: Gene | BANK1 |
| Entrez Gene ID: | 55024 |
| dna/ac: | BC032241.1 |
| sequence: (SEQ ID NO: 2) | |
| atgctgccagcagcgccaggcaaggggcttgggagcccggaccccgccccctgcggcccagcgcccccagg aaatacaaaagatataataatgatatatgaagaagatgctgaggaatgggctctgtacttgacagaagtatttttacatgttgtga aagggaagccatcctgttatatcgcttggagaatttctcttttcggcatttggagttgctgaacttaacgtcttacaaatgtaaact tttgatattatcaaatagcctgcttagagacctaactccaaagaaatgtcagtttctggaaaagatacttcattcaccaaaaagtgt agttactttgctttgtggagtgaagagttcagatcagctctatgaattactaaatatctctcaaagcagatgggagatctcaactga acaggaacctgaagactacatctctgtaatccagagtatcatattcaaagattctgaagactactttgaggtcaacattccaaca gacctacgagcaaaacattctggggaaataagtgagagaaaggaaattgaagaactatcagaagcttcaagaaacaccata ccactagcagtggtgcttcccactgaaattccatgtgagaatcctggtgaaatattcataatttgagagatgaagtaattggtga tactgtagaggttgaatttacatcaagtaataagcgcattagaacacggccagccctttggaataagaaagtctggtgcatgaa | |
| agctttagagtttcctgctggttcagtccatgtcaatgtctactgtgatggaatcgttaaagctacaaccaaaattaagtactaccc aacagcaaaggcaaaggaatgcctattcagaatggcagattcaggagagagtttgtgccagaatagcattgaagaacttgat ggtgtccttacatccatattcaaacatgagataccatattatgagttccagtctcttcaaactgaaatttgttctcaaaacaaatata ctcatttcaaagaacttccaactcttctccactgtgcagcaaaatttggcttaaagaacctggctattcatttgcttcaatgttcagg agcaacctgggcatctaagatgaaaaatatggagggttcagaccccgcacatattgctgaaaggcatggtcacaaagaactc aagaaaatcttcgaagacttttcaatccaagaaattgacataaataatgagcaagaaaatgattatgaagaggatattgcctcatt ttccacatatattccttccacacagaacccagcatttcatcatgaaagcagaaagacatacgggcagagtgcagatggagctg aggcaaatgaaatggaaggggaaggaaaacagaatggatcaggcatggagaccaaacacagcccactagaggttggcag tgagagttctgaagaccagtatgatgacttgtatgtgttcattcctggtgctgatccagaaaataattcacaagagccactcatga gcagcagacctcctctcccccccgccgcgacctgtagctaatgccttccaactggaaagacctcacttcaccttaccagggaca atggtggaaggccaaatggaaagaagtcaaaactggggtcatcctggtgttagacaagaaacaggagatgaacccaaagg agaaaaagagaagaaagaagaggaaaaagagcaggaggaggaagaagacccatatactttgctgagattgatgacagtg aatatgacatgatattggccaatctgagtataaagaaaaaaactgggagtcggtctttcattataaatagacctcctgcccccac accccgacccacaagtatacctccaaaagaggaaactacaccttacatagctcaagtgtttcaacaaaagacagccagaaga caatctgatgatgacaagttccgtggtcttcctaagaaacaagacagagctcggatagagagtccagccttttctactctcagg ggctgtctaactgatggtcaggaagaactcatcctcctgcaggagaaagtaaagaatggaaaaatgtctatggatgaagctct ggagaaatttaaacactggcagatgggaaaaagtggcctggaaatgattcagcaggagaaattacgacaactacgagactg cattattgggaaaaggccagaagaagaaaatgtctataataaactcaccattgtgcaccatccaggtggtaaggaaactgccc acaatgaaaataagttttataatgtacacttcagcaataagcttcctgctcgaccccaagttgaaaaggaatttggtttctgttgca agaaagatcattaa | |
| Gene symbol: | CEBPG |
| Entrez Gene ID: | 1054 |
| dna/ac: | BC007582.1 |
| sequence: (SEQ ID NO: 3) | |
| atgagcaagatatcgcagcaaaacagcactccaggggtgaacggaattagtgttatccatacccaggcacatgcc agcggcttacagcaggttcctcagctggtgcctgctggccctgggggaggaggcaaagctgtggctcccagcaagcagag caaaaagagttcgcccatggatcgaaacagtgacgagtatcggcaacgccgagagaggaacaacatggctgtgaaaaaga gccggttgaaaagcaagcagaaagcacaagacacactgcagagagtcaatcagctcaaagaagagaatgaacggttgga agcaaaaatcaaattgctgaccaaggaattaagtgtactcaaagatttgtttcttgagcatgcacacaaccttgcagacaacgta cagtccattagcactgaaaatacgacagcagatggcgacaatgcaggacagtag | |
| Gene symbol: | DDX55 |
| Entrez Gene ID: | 57696 |
| dna/ac: | BC030020.2 |
| sequence: (SEQ ID NO: 4) | |
| atggagcatgtgacagagggctcctgggagtcgctgcctgtgccgctgcacccgcaggtgctgggcgcgctgc gggagctgggcttcccgtacatgacgccggtgcagtccgcaaccatccctctgttcatgcgaaacaaagatgtcgctgcaga agcggtcacaggtagtggcaaaacactcgcttttgtcatccccatcctggaaattcttctgagaagagaagagaagttaaaaaa gagtcaggttggagccataatcatcacccccactcgagagctggccattcaaatagacgaggtcctgtcgcatttcacgaagc acttccccgagttcagccagattctttggatcggaggcaggaatcctggagaagatgttgagaggtttaagcaacaaggtggg aacatcattgtggccactccaggccgcttggaggacttgttccggaggaaggccgaaggcttggatctggccagctgtgtgc gatccctggatgtcctggtgttggatgaggcagacagacttctggacatggggtttgaggcaagcatcaacaccattctggag | |
| tttttgccaaagcagaggagaacaggccttttctctgccactcagacgcaggaagtggagaacctggtgagagcgggcctcc ggaaccctgtccgggtctcagtgaaggagaagggcgtggcagccagcagtgcccagaagacccccctcccgcctggaaaa ctactacatggtatgcaaggcagatgagaaatttaatcagctggtccatttcttcgcaatcataagcaggagaaacacctggtc ttcttcagcacctgtgcctgtgtggaatactatgggaagactctggaagtgctggtgaagggcgtgaagattatgtgcattcac ggaaagatgaaatataaacgcaataagatcttcatggagttccgcaaattgcaaagtgggattttagtgtgcactgatgtgatgg cccggggaattgatattcctgaagtcaactgggttttgcagtatgaccctcccagcaatgcaagtgccttcgtgcatcgctgcg gtcgcacagctcgcattggccacgggggcagcgctctggtgttcctcctgcccatggaagagtcatacatcaatttccttgcaa ttaaccaaaaatgccccctgcaggagatgaagccccagagaaacacagcggaccttctgccaaaactcaagtccatggccc tggctgacagagctgtgtttgaaaagggcatgaaagcttttgtgtcatatgtccaagcttatgcaaagcatgaatgcaacctgat tttcagattaaaggatcttgatttgccagccttgctcgaggttttgccctgctgaggatgcccaagatgccagaattgagaggg aagcagtttccagattttgtgcccgtggacgttaataccgacacgattccatttaaagataaaatcagagaaaagcagaggcag aaactcctggagcaacaaagaagagagaaaacagaaaatgaagggagaagaaaattcataaaaaataaagcttggtcaaa gcagaaggccaaaaaagaaaagaagaaaaaaatgaatgagaaaaggaaaagggaagagggttctgatattgaagatgag gacatggaagaacttcttaatgacacaagactcttgaaaaaacttaagaaaggcaaaataactgaagaagaatttgagaaggg cttgttgacaactggcaaaagaacaatcaagacagtggatttagggatctcagatttggaagatggctgctaa | |
| Gene symbol: | DOM3Z |
| Entrez Gene ID: | 1797 |
| dna/ac: | BC019083.2 |
| sequence: (SEO ID NO: 5) | |
| atggatcccaggggggaccaagagaggagctgagaagacagaggtagctgagcctcggaacaaactacctcgt ccagcaccttctctgcccacagaccctgccctctactctgggcccttttcctttctaccggcgcccttcggaactgggctgcttct ccctggatgctcaacgccagtaccatggagatgcccgagccctgcgctactatagcccaccccccactaacggtccaggcc ccaactttgacctcagagacggatacccggatcgataccagccccgggacgaggaggtccaggaaaggctggaccacctg ctgtgctggctcctggaacaccgaggccggttggagggggggtccaggctggctggcagaggccatagtgacgtggcggg ggcacctgacaaaactgctgacgacaccgtatgagcggcaggagggctggcagctggcagcctcccggttccagggaac actatacctgagtgaagtggagacaccgaacgctcgggcccagaggcttgctcggccaccgctcctccgggagcttatgta catgggatacaaatttgagcagtacatgtgtgcagacaaacctggaagctccccagacccctctggggaggttaacaccaac gtggccttctgctctgtgctacgcagccgcctgggaagccaccctctgctcttctcaggggaggtagactgcacagaccccc aagccccatccacacagcccccaacctgctatgtggagctcaagacctccaaggagatgcacagccctggccaatggagg agtttctacagacacaagctcctgaaatggtgggctcagtcattcctcccaggggtcccgaatgttgttgctggcttccgtaacc cagacggttttgtctcttccctcaagacctttcctaccatgaagatgtttgaatatgtcaggaatgaccgtgacggctggaatccc tctgtgtgcatgaacttctgtgccgccttccttagctttgcccagagcacggttgtccaggatgaccccaggctcgttcatctctt ctcttgggagcctggcggcccagtcaccgtgtctgtacaccaagatgcaccttacgccttcctgcccatatggtatgtggaagc tatgactcaggacctcccatcacccccccaagactccctctcccaaatag | |
| Gene symbol: | E1B-AP5 |
| Entrez Gene ID: | 11100 |
| dna/ac: | BC009988.2 |
| sequence: (SEQ ID NO: 6) | |
| atggacaatattaccaggcagaaccaattctacgatacccaagtcatcaaacaagaaaacgagtcaggctacgag aggagaccactggaaatggagcagcagcaggcctatcgtccagaaatgaagacagagatgaagcaaggagcacccacca | |
| gcttcctcccgcctgaagcttctcaactcaagccagacaggcagcaattccagagtcgaaagaggccttatgaagaaaaccg gggacgggggtactttgagcaccgagaggataggaggggccgctctcctcagcctcctgctgaagaggatgaagatgactt tgatgatacccttgttgctattgacacctataactgcgacctccacttcaaggtggcccgagatcggagtagtggctatccgctc acaattgagggctttgcatacctgtggtcaggagcccgtgccagctatggggtcagaagggccgtgtatgcttcgagatga agatcaatgaggaaatctccgtgaagcaccttccgtctacagagcctgacccccacgtggtccgtatcggctggtccctggac tcctgcagcacccagctaggcgaagagcctttctcctatggctatggaggcactgggaagaagtccaccaatagccggtttg aaaactacggagacaagtttgcagagaacgatgtgattggctgctttgcggattttgaatgtggaaatgacgtggaactgtcttt taccaagaatggaaagtggatgggcattgctttccgaatccagaaggaagccttgggggggtcaggccctctatcctcatgtcc tggtgaagaattgcgcagtggagttcaacttcggacagagagcagagccctactgttctgtcctcccggggtttaccttcatcc agcaccttccccttagtgagcgtatccggggcaccgttggaccaaagagcaaggcagaatgtgagattctgatgatggtggg cctgcctgctgctggcaagaccacatgggccatcaaacatgcagcctccaacccttccaagaagtacaacatcctgggtacc aatgccatcatggataagatgcgggtgatgggcctacgccggcagcggaactatgctggccgctgggatgtcctgatccag caggccacccagtgcctcaaccgcctcatccagattgctgcccgcaagaaacgcaactatatcctagatcagacaaatgttta tgggtcagcccagagacgaaaaatgagaccatttgaaggcttccagcgcaaagctattgtaatttgtcccactgacgaggacc taaaagaccgaacaataaagcgaaccgacgaggaagggaaggatgtcccagatcatgcggtcttagaaatgaaagccaac ttcacgttgccagatgttggggacttcctggatgaggttctgttcattgagctgcagcgggaggaagcggacaagctagtgag gcagtacaacgaggaaggccgcaaggctgggccaccccctgaaaagcgctttgacaaccgaggtggtggtggcttccgg ggccgcgggggtggtggtggcttccagcgctatgaaaaccgaggaccccctggaggcaaccgtggcggcttccagaacc gagggggaggcagcggtggaggaggcaactaccgaggaggtttcaaccgcagcggaggtggtggctatagccagaacc gctggggtaacaacaaccgggataacaacaactccaacaacagaggcagctacaaccgggctccccagcaacagccgcc accacagcagcctccgccaccacagccaccaccccagcagccaccgccaccacccagctacagccctgctcggaacccc ccaggggccagcacctacaataagaacagcaacatccctggctcaagcgccaataccagcacccccaccgtcagcagcta cagccctccacagccgagttacagccagccaccctacaaccagggaggttacagccagggctacacagccccaccgcctc cacctccaccaccacctgcctacaactatgggagctacggcggttacaacccggccccctataccccaccgccacccccca ccgcacagacctaccctcagcccagctataaccagtatcagcagtatgcccagcagtggaaccagtactatcagaaccagg gccagtggccgccatactacgggaactacgactacgggagctactccgggaacacacagggtggcacaagtacacagtag | |
| Gene symbol: | FUS |
| Entrez Gene ID: | 2521 |
| dna/ac: | BC000402.2 |
| sequence: (SEQ ID NO: 7) | |
| atggcctcaaacgattatacccaacaagcaacccaaagctatggggcctaccccacccagcccgggcagggct attcccagcagagcagtcagccctacggacagcagagttacagtggttatagccagtccacggacacttcaggctatggcca gagcagctattcttcttatggccagagccagaacacaggctatggaactcagtcaactccccagggatatggctcgactggc ggctatggcagtagccagagctcccaatcgtcttacgggcagcagtcctcctatcctggctatggccagcagccagctccca gcagcacctcgggaagttacggtagcagttctcagagcagcagctatgggcagccccagagtgggagctacagccagcag cctagctatggtggacagcagcaaagctatggacagcagcaaagctataatccccctcagggctatggacagcagaaccag tacaacagcagcagtggtggtggaggtggaggtggaggtggaggtaactatggccaagatcaatcctccatgagtagtggt ggtggcagtggtggcggttatggcaatcaagaccagagtggtggaggtggcagcggtggctatggacagcaggaccgtg gaggccgcggcaggggtggcagtggtggcggcggcggcggcggtggtggttacaaccgcagcagtggtggctatg aacccagaggtcgtggaggtggccgtggaggcagaggtggcatgggcggaagtgaccgtggtggcttcaataaatttggt ggccctcgggaccaaggatcacgtcatgactccgaacaggataattcagacaacaacaccatctttgtgcaaggcctgggtg agaatgttacaattgagtctgtggctgattacttcaagcagattggtattattaagacaaacaagaaaacgggacagcccatgat taatttgtacacagacagggaaactggcaagctgaaggagaggcaacggtctcttttgatgacccaccttcagctaaagca gctattgactggtttgatggtaaagaattctccggaaatcctatcaaggtctcatttgctactcgccgggcagactttaatcgggg tggtggcaatggtcgtggaggccgagggcgaggaggacccatgggccgtggaggctatggaggtggtggcagtggtggt | |
| ggtggccgaggaggatttcccagtggaggtggtggcggtggaggacagcagcgagctggtgactggaagtgtcctaatcc cacctgtgagaatatgaacttctcttggaggaatgaatgcaaccagtgtaaggcccctaaaccagatggcccaggaggggg accaggtggctctcacatgggggggtaactacggggatgatcgtcgtggtggcagaggaggctatgatcgaggcggctacc ggggccgcggcggggaccgtggaggcttccgaggggggccggggtggtggggacagaggtggctttggccctggcaag atggattccaggggtgagcacagacaggatcgcagggagaggccgtattaa | |
| Gene symbol: | HAGH |
| Entrez Gene ID: | 3029 |
| dna/ac: | BC000840.1 |
| sequence: (SEQ ID NO: 8) | |
| atgaaggtagaggtgctgcctgccctgaccgacaactacatgtacctggtcattgatgatgagaccaaggaggct gccattgtggatccggtgcagccccagaaggtcgtggacgcggcgagaaagcacggggtgaaactgaccacagtgctca ccacccaccaccactgggaccatgctggcgggaatgagaaactggtcaagctggagtcgggactgaaggtgtacggggggt gacgaccgtatcgggggccctgactcacaagatcactcacctgtctacactgcaggtggggtctctgaacgtcaagtgcctgg cgaccccgtgccacacttcaggacacatttgttacttcgtgagcaagcccggaggctcggagccccctgccgtgttcacagg tgacaccttgtttgtggctggctgcgggaagttctatgaaggactgcggatgagatgtgtaaagctctgctggaggtcttggg ccggctcccccccggacacaagagtctactgtggccacgagtacaccatcaacaacctcaagtttgcacgccacgtggagcc cggcaatgccgccatccgggagaagctggcctgggccaaggagaagtacagcatcggggagcccacagtgccatccacc ctggcagaggagtttacctacaaccccttcatgagagtgagggagaagacggtgcagcagcacgcaggtgagacggaccc ggtgaccaccatgcgggccgtgcgcagggagaaggaccagttcaagatgccccgggactga | |
| Gene symbol: | HMG20B |
| Entrez Gene ID: | 10362 |
| dna/ac: | BC002552.2 |
| sequence: (SEQ ID NO: 9) | |
| atgtcccacggccccaagcagcccggcgcggccgccgcgccggcgggcggcaaggctccgggccagcatg ggggcttcgtggtgactgtcaagcaagagcgcggcgagggtccacgcgcgggcgagaaggggtcccacgaggaggag ccggtgaagaaacgcggctggcccaagggcaagaagcggaagaagattctgccgaatgggcccaaggcaccggtcacg ggctacgtgcgcttcctgaacgagcggcgcgagcagatccgcacgcgccacccggatctgccctttcccgagatcaccaa gatgctgggcgccgagtggagcaagctgcagccaacggaaaagcagcggtacctggatgaggccgagagagagaagca gcagtacatgaaggagctgcgggcgtaccagcagtctgaagcctataagatgtgcacggagaagatccaggagaagaaga tcaagaaagaagactcgagctctgggctcatgaacactctcctgaatggacacaagggtggggactgcgatggcttctccac cttcgatgttcccatcttcactgaagagttcttggaccaaaacaaagcgcgtgaggcggagcttcggcgcttgcggaagatga atgtggccttcgaggagcagaacgcggtactgcagaggcacacgcagagcatgagcagcgcgcgcgagcgtctggagc aggagctggcgctggaggagcggaggacgctggcgctgcagcagcagctccaggccgtgcgccaggcgctcaccgcc agcttcgcctcactgccggtgccgggcacgggcgaaacgcccacgctgggcactctggacttctacatggcccggcttcac ggagccatcgagcgcgaccccgcccagcacgagaagctcatcgtccgcatcaaggaaatcctggcccaggtcgccagcg agcacctgtga | |
| Gene symbol: | HOXB6 |
| Entrez Gene ID: | 3216 |
| dna/ac: | BC014651.1 |
| sequence: (SEQ ID NO: 10) | |
| atgagttcctatttcgtgaactccaccttccccgtcactctggccagcgggcaggagtccttcctgggccagctacc gctctattcgtcgggctatgcggacccgctgagacattaccccgcgccctacgggccagggccgggccaggacaagggct ttgccacttcctcctattacccgccggcgggcggtggctacggccgagcggcgccctgcgactacgggccggcgccggcc ttctaccgcgagaaagagtcggcctgcgcactctccggcgccgacgagcagcccccgttccaccccgagccgcggaagtc ggactgcgcgcaggacaagagcgtgttcggcgagacagaagagcagaagtgctccactccggtctacccgtggatgcag cggatgaattcgtgcaacagttcctcctttgggcccagcggccggcgaggccgccagacatacacacgttaccagacgctg gagctggagaaggagtttcactacaatcgctacctgacgcggcggcggcgcatcgagatcgcgcacgccctgtgcctgac ggagaggcagatcaagatatggttccagaaccgacgcatgaagtggaaaaaggagagcaaactgctcagcgcgtctcagc tcagtgccgaggaggaggaagaaaaacaggccgagtga | |
| Gene symbol: | IFI16 |
| Entrez Gene ID: | 3428 |
| dna/ac: | BC017059.1 |
| sequence: (SEO ID NO: 11) | |
| atgggaaaaaaatacaagaacattgttctactaaaggattagaggtcatcaatgattatcattttagaatggttaagt ccttactgagcaacgatttaaaacttaatttaaaaatgagagaagagtatgacaaaattcagattgctgacttgatggaagaaaa gttccgaggtgatgctggtttgggcaaactaataaaaattttcgaagatataccaacgcttgaagacctggctgaaactcttaaa aaagaaaagttaaaagtaaaggaccagccctatcaagaaagaggaagaaggaagtggatgctacttcacctgcaccctcc acaagcagcactgtcaaaactgaaggagcagaggcaactcctggagctcagaaaagaaaaaaatcaaccaaagaaaagg ctggacccaaagggagtaaggtgtccgaggaacagactcagcctccctctcctgcaggagccggcatgtccacagccatg ggccgttccccatctcccaagacctcattgtcagctccacccaacacttcttcaactgagaacccgaaaacagtggccaaatgt caggtaactcccagaagaaatgttctccaaaaacgcccagtgatagtgaaggtactgagtacaacaaagccatttgaatatga gaccccagaaatggagaaaaaaataatgtttcatgctacagtggctacacagacacagttcttccatgtgaaggttttaaacac cagcttgaaggagaaattcaatggaaagaaaatcatcatcatatcagattatttggaatatgatagtctcctagaggtcaatgaa gaatctactgtatctgaagctggtcctaaccaaacgtttgaggttccaaataaaatcatcaacagagcaaaggaaactctgaag attgatattcttcacaaacaagcttcaggaaatattgtatatggggtatttatgctacataagaaaacagtaaatcagaagaccac aatctacgaaattcaggatgatagaggaaaaatggatgtagtggggacaggacaatgtcacaatatcccctgtgaagaagga gataagctccaacttttctgctttcgacttagaaaaaagaaccagatgtcaaaactgatttcagaaatgcatagttttatccagata aagaaaaaaacaaacccgagaaacaatgaccccaagagcatgaagctaccccaggaacagagtcagcttccaaatccttca gaggccagcacaaccttccctgagagccatcttcggactcctcagatgccaccaacaactccatccagcagtttcttcaccaa gaaaagtgaagacacaatctccaaaatgaatgacttcatgaggatgcagatactgaaggaagggagtcattttccaggaccgt tcatgaccagcataggcccagctgagagccatccccacactcctcagatgcctccatcaacaccaagcagcagtttcttaacc acgttgaaaccaagactgaagactgaacctgaagaagtttccatagaagacagtgcccagagtgacctcaaagaagtgatg gtgctgaacgcaacagaatcatttgtatatgagcccaaagagcagaagaaaatgtttcatgccacagtggcaactgagaatga agtcttccgagtgaaggtttttaatattgacctaaaggagaagttcaccccaaagaagatcattgccatagcaaattatgtttgcc gcaatgggttcctggaggtatatcctttcacacttgtggctgatgtgaatgctgaccgaaacatggagatcccaaaaggattgat tagaagtgccagcgtaactcctaaaatcaatcagctttgctcacaaactaaaggaagttttgtgaatggggtgtttgaggtacat aagaaaaatgtaaggggtgaattcacttattatgaaatacaagataatacagggaagatggaagtggtggtgcatggacgact gaccacaatcaactgtgaggaaggagataaactgaaactcacctgctttgaattggcaccgaaaagtgggaataccgggga gttgagatctgtaattcatagtcacatcaaggtcatcaagaccaggaaaaacaagaaagacatactcaatcctgattcaagtat ggaaacttcaccagactttttcttctaa | |
| Gene symbol: | KRT8 |
| Entrez Gene ID: | 3856 |
| dna/ac: | BC008200.1 |
| sequence: (SEQ ID NO: 12) | |
| atgaacaaggtagagctggagtctcgcctggaagggctgaccgacgagatcaacttcctcaggcagctatatgaa gaggagatccgggagctgcagtcccagatctcggacacatctgtggtgctgtccatggacaacagccgctccctggacatg gacagcatcattgctgaggtcaaggcacagtacgaggatattgccaaccgcagccgggctgaggctgagagcatgtacca gatcaagtatgaggagctgcagagcctggctgggaagcacggggatgacctgcggcgcacaaagactgagatctctgaga tgaaccggaacatcagccggctccaggctgagattgagggcctcaaaggccagagggcttccctggaggccgccattgca gatgccgagcagcgtggagagctggccattaaggatgccaacgccaagttgtccgagctggaggccgccctgcagcggg ccaagcaggacatggcgcggcagctgcgtgagtaccaggagctgatgaacgtcaagctggccctggacatcgagatcgcc acctacaggaagctgctggagggcgaggagagccggctggagtctgggatgcagaacatgagtattcatacgaagaccac cagcggctatgcaggtggtctgagctcggcctatggggggcctcacaagccccggcctcagctacagcctgggctccagcttt ggctctggcgcgggctccagctccttcagccgcaccagctcctccagggccgtggttgtgaagaagatcgagacacgtgat gggaagctggtgtctgagtcctctgacgtcctgcccaagtga | |
| Gene symbol: | LIN28 |
| Entrez Gene ID: | 79727 |
| dna/ac: | BC028566.2 |
| sequence: (SEQ ID NO: 13) | |
| atgggctccgtgtccaaccagcagtttgcaggtggctgcgccaaggcggcagaagaggcgcccgaggaggcg ccggaggacgcggcccgggcggcggacgagcctcagctgctgcacggtgcgggcatctgtaagtggttcaacgtgcgca tggggttcggcttcctgtccatgaccgcccgcgccggggtcgcgctcgacccccagtggatgtctttgtgcaccagagtaa gctgcacatggaagggttccggagcttgaaggagggtgaggcagtggagttcacctttaagaagtcagccaagggtctgga atccatccgtgtcaccggacctggtggagtattctgtattgggagtgagaggcggccaaaaggaaagagcatgcagaagcg cagatcaaaaggagacaggtgctacaactgtggaggtctagatcatcatgccaaggaatgcaagctgccaccccagcccaa gaagtgccacttctgccagagcatcagccatatggtagcctcatgtccgctgaaggcccagcagggccctagtgcacaggg aaagccaacctactttcgagaggaagaagaagaaatccacagccctaccctgctcccggaggcacagaattga | |
| Gene symbol: | LNX |
| Entrez Gene ID: | 84708 |
| dna/ac: | BC022983.1 |
| sequence: (SEQ ID NO: 14) | |
| atgaaccagccagagtctgccaacgatcctgaacccctgtgtgcagtgtgtggccaagcccactccttggaggaa aaccacttctacagctatccagaggaagtggatgatgacctcatctgccacatctgcctgcaggctttgctggacccccctggac actccgtgtggacacacctactgcaccctctgcctcaccaacttcctggtggagaaggacttctgtcccatggaccgcaagcc tctggttctgcagcactgcaagaagtccagcatcctggtcaacaaactcctcaacaagctactggtgacctgcccattcaggg agcactgcacccaggtgttgcagcgctgtgacctcgagcatcactttcaaaccagctgtaaaggtgcctcccactacggcctg accaaagataggaagaggcgctcacaagatggctgtccagacggctgtgcgagcctcacagccacggctccctccccaga | |
| ggtttctgcagctgccaccatctccttaatgacagacgagcctggcctagacaaccctgcctacgtgtcctcggcagaggacg ggcagccagcaatcagcccagtggactctggccggagcaaccgaactagggcacggccctttgagagatccactattaga agcagatcatttaaaaaataaatcgagctttgagtgttcttcgaaggacaaagagcgggagtgcagttgccaaccatgccga ccagggcagggaaaattctgaaaacaccactgcccctgaagtctttccaaggttgtaccacctgattccagatggtgaaattac cagcatcaagatcaatcgagtagatcccagtgaaagcctctctattaggctggtgggaggtagcgaaaccccactggtccata tcattatccaacacatttatcgtgatggggtgatcgccagagacggccggctactgccaggagacatcattctaaaggtcaac gggatggacatcagcaatgtccctcacaactacgctgtgcgtctcctgcggcagccctgccaggtgctgtggctgactgtgat gcgtgaacagaagttccgcagcaggaacaatggacaggccccggatgcctacagaccccgagatgacagctttcatgtgat tctcaacaaaagtagccccgaggagcagcttggaataaaactggtgcgcaaggtggatgagcctggggttttcatcttcaatg tgctggatggcggtgtggcatatcgacatggtcagcttgaggagaatgaccgtgtgttagccatcaatggacatgatcttcgat atggcagcccagaaagtgcggctcatctgattcaggccagtgaaagacgtgttcacctcgtcgtgtcccgccaggttcggca gcggagccctgacatctttcaggaagccggctggaacagcaatggcagctggtccccagggccaggggagaggagcaac actcccaagcccctccatcctacaattacttgtcatgagaaggtggtaaatatccaaaaagaccccggtgaatctctcggcatg accgtcgcagggggagcatcacatagagaatgggatttgcctatctatgtcatcagtgttgagcccggaggagtcataagca gagatggaagaataaaaacaggtgacattttgttgaatgtggatggggtcgaactgacagaggtcagccggagtgaggcag tggcattattgaaaagaacatcatcctcgatagtactcaaagctttggaagtcaaagagtatgagccccaggaagactgcagc agcccagcagccctggactccaaccacaacatggccccacccagtgactggtccccatcctgggtcatgtggctggaattac cacggtgcttgtataactgtaaagatattgtattacgaagaaacacagctggaagtctgggcttctgcattgtaggaggttatga agaatacaatggaaacaaaccttttttcatcaaatccattgttgaaggaacaccagcatacaatgatggaagaattagatgtggt gatattcttcttgctgtcaatggtagaagtacatcaggaatgatacatgcttgcttggcaagactgctgaaagaacttaaaggaa gaattactctaactattgtttcttggcctggcactttttatag | |
| Gene symbol: | MAPIS |
| Entrez Gene ID: | 55201 |
| dna/ac: | BC006358.1 |
| sequence: (SEQ ID NO: 15) | |
| atgggcgtgggccggctggacatgtatgtgctgcacccgccctccgccggcgccgagcgcacgctggcctgtg tgtgcgccctgctggtgtggcaccccgccggccccggcgagaaggtggtgcgcgtgctgttccccggttgcaccccgccc gcctgcctcctggacggcctggtccgcctgcagcacttgaggttcctgcgagagcccgtggtgacgccccaggacctggag gggccggggcgagccgagagcaaagagagcgtgggctcccgggacagctcgaagagagagggcctcctggccaccca ccctagacctggccaggagcgccctggggtggcccgcaaggagccagcacgggctgaggccccacgcaagactgagaa agaagccaagacccccgggagttgaagaaagaccccaaaccgagtgtctcccggacccagccgcgggaggtgcgccg ggcagcctcttctgtgcccaacctcaagaagacgaatgcccaggcggcacccaagccccgcaaagcgcccagcacgtcc cactctggcttcccgccggtggcaaatggaccccgcagcccgcccagcctccgatgtggagaagccagcccccccagtgc ggcctgcggctctccggcctcccagctggtggccacgcccagcctggagctggggccgatcccagccggggaggagaa ggcactggagctgcctttggccgccagctcaatcccaaggccacgcacaccctcccctgagtcccaccggagccccgcag agggcagcgagcggctgtcgctgagcccactgcggggcggggaggccgggccagacgcctcacccacagtgaccaca cccacggtgaccacgccctcactacccgcagaggtgggctccccgcactcgaccgaggtggacgagtccctgtcggtgtc ctttgagcaggtgctgccgccatccgcccccaccagtgaggctgggctgagcctcccgctgcgtggcccccgggcgcggc gctcggcttccccacacgatgtggacctgtgcctggtgtcaccctgtgaatttgagcatcgcaaggcggtgccaatggcacc ggcacctgcgtcccccggcagctcgaatgacagcagtgcccggtcacaggaacgggcaggtgggctgggggccgagga gacgccacccacatcggtcagcgagtccctgcccaccctgtctgactcggatcccgtgcccctggcccccggtgcggaga ctcagacgaagacacagagggctttggagtccctcgccacgaccctttgcctgacccccctcaaggtcccccaccactgcct gacccatccagcatctgcatggtggaccccgagatgctgcccccaagacagcacggcaaacggagaacgtcagccgca cccggaagcccctggcccgccccaactcacgcgctgccgcccccaaagccactccagtggctgctgccaaaaccaaggg gcttgctggtggggaccgtgccagccgaccactcagtgcccggagtgagcccagtgagaaggaggccgggcacccctg | |
| tccagaaagtcctcaacccccaagactgccactcgaggcccgtcggggtcagccagcagccggcccggggtgtcagcca ccccacccaagtccccggtctacctggacctggcctacctgcccagcgggagcagcgcccacctggtggatgaggagttct tccagcgcgtgcgcgcgctctgctacgtcatcagtggccaggaccagcgcaaggaggaaggcatgcgggccgtcctgga cgcgctactggccagcaagcagcattgggaccgtgacctgcaggtgaccctgatccccactttcgactcggtggccatgcat acgtggtacgcagagacgcacgcccggcaccaggcgctgggcatcacggtgttgggcagcaacagcatggtgtccatgc aggatgacgccttcccggcctgcaaggtggagttctag | |
| Gene symbol: | NDUFV3 |
| Entrez Gene ID: | 4731 |
| dna/ac: | BC021217.2 |
| sequence: (SEQ ID NO: 16) | |
| atggctgccccgtgtttgctgcggcaaggacgagccggggcgctgaagactatgctccaggaagcccaggtgtt tcgaggacttgcttctacggtttctttgtctgcggaatcagggaagagtgaaaagggtcagccacagaattccaagaagcaaa gtccaccaaaaaatgtagtggaaccaaaggagaggggcaagctcctagccacccagacagcagctgaattgtctaaaaact tatcttcacccagttcttacccgccagctgtgaataagggcaggaaggtagctagtcccagtcccagtggcagcgtgctattca cagatgaaggggttccgaaattttgtcaagaaagactttggtagagtttccacagaaagttctgtctccattcagaaaacaggg ctctgattcagaagctcgtcaggtgggtcggaaagtgacgtcgccttcgtcttcatcctcgtccagctcctctgattctgaatctg atgatgaggctgacgtttcagaggtcactcctcgagtggtgagcaaaggcagagggggcttcgaaaaccagaggcctctc attcctttgaaaacagagcccccgagttacagtatcagcaaaagagaaaaccttgctgcagaagccgcatgtggacattact gatccagagaagccccaccagccaaagaagaaagggtcccctgctaagccatcagaaggcagggaaaatgcgagacca aaaaccacaatgcccagatctcaagtagatgaagagtttttgaagcaaagtttaaaggaaaaacaattgcagaaaacatttaga ttaaatgaaatagataaagaaagccaaaagccatttgaagttaaaggacccttacctgtccacacaaaatcagggttgtctgcg ccaccgaagggcagcccagcgcctgctgtgttggcagaagaggccagagcagaggggcagctgcaagccagtcctcctg gggcggcagaggggcatctggaaaaacccgtgccagagccccagcgcaaggcggcccctcccctgcccagaaaggaa acctcagggacgcagggaatagaaggccacctgaagggtggacaggcaatcgtggaagatcagataccaccaagcaattt ggagacagttcctgttgagaataaccacggtttccatgaaagacagcagcgctgaagcttgaggccgaggcgaggccat ggaagatgcagccgcgccagggaacgaccgaggcggcacacaggagccagccccagtgcctgctgagccgtttgacaa cactacctacaagaacctgcagcatcatgactacagcacgtacaccttcttagacctcaacctcgaactctcaaaattcaggat gcctcagccctcctcaggccgggagtcacctcgacactga | |
| Gene symbol: | PABPC1 |
| Entrez Gene ID: | 26986 |
| dna/ac: | BC015958.2 |
| sequence: (SEQ ID NO: 17) | |
| atgaaccccagtgcccccagctaccccatggcctcgctctacgtgggggacctccaccccgacgtgaccgagg cgatgctctacgagaagttcagcccggccgggcccatcctctccatccgggtctgcagggacatgatcacccgccgctcctt gggctacgcgtatgtgaacttccagcagccggcggacgcggagcgtgctttggacaccatgaattttgatgttataaagggca agccagtacgcatcatgtggtctcagcgtgatccatcacttcgcaaaagtggagtaggcaacatattcattaaaaatctggaca aatccattgataataaagcactgtatgatacattttctgcttttggtaacatcctttcatgtaaggtggtttgtgatgaaaatggttcc aagggctatggatttgtacactttgagacgcaggaagcagctgaaagagctattgaaaaaatgaatggaatgctcctaaatgat cgcaaagtatttgttggacgatttaagtctcgtaaagaacgagaagctgaacttggagctagggcaaaagaattcaccaatgtt tacatcaagaattttggagaagacatggatgatgagcgccttaaggatctctttggcaagtttgggcctgccttaagtgtgaaag | |
| taatgactgatgaaagtggaaaatccaaaggatttggatttgtaagctttgaaaggcatgaagatgcacagaaagctgtggatg agatgaacggaaaggagctcaatggaaaacaaatttatgttggtcgagctcagaaaaaggtggaacggcagacggaactta agcgcaaatttgaacagatgaaacaagataggatcaccagataccagggtgttaatctttatgtgaaaaatcttgatgatggtat tgatgatgaacgtctccggaaagagttttctccatttggtacaatcactagtgcaaaggttatgatggagggtggtcgcagcaa agggtttggttttgtatgtttctcctccccagaagaagccactaaagcagttacagaaatgaacggtagaattgtggccacaaa gccattgtatgtagctttagctcagcgcaaagaagagcgccaggctcacctcactaaccagtatatgcagagaatggcaagtg tacgagctgttcccaaccctgtaatcaacccctaccagccagcacctccttcaggttacttcatggcagctatcccacagactc agaaccgtgctgcatactatcctcctagccaaattgctcaactaagaccaagtcctcgctggactgctcagggtgccagacct catccattccaaaatatgcccggtgctatccgcccagctgctcctagaccaccatttagtactatgagaccagcttcttcacagg ttccacgagtcatgtcaacacagcgtgttgctaacacatcaacacagacaatgggtccacgtcctgcagctgcagccgctgca gctactcctgctgtccgcaccgttccacagtataaatatgctgcaggagttcgcaatcctcagcaacatcttaatgcacagcca caagttacaatgcaacagcctgctgttcatgtacaaggtcaggaacctttgactgcttccatgttggcatctgcccctcctcaag agcaaaagcaaatgttgggtgaacggctgtttcctcttattcaagccatgcaccctactcttgctggtaaaatcactggcatgttg ttggagattgataattcagaacttcttcatatgctcgagtctccagagtcactccgttctaaggttgatgaagctgtagctgtacta caagcccaccaagctaaagaggctgcccagaaagcagttaacagtgccaccggtgttccaactgtttaa | |
| Gene symbol: | PHLDA 1 |
| ntrez Gene ID: | 22822 |
| dna/ac: | BC018929.2 |
| sequence: (SEQ ID NO: 18) | |
| atgctggagagtagcggctgcaaagcgctgaaggagggcgtgctggagaagcgcagcgacgggttgttgcag ctctggaagaaaaagtgttgcatcctcaccgaggaagggctgctgcttatcccgcccaagcagctgcaacaccagcagcag cagcaacagcagcagcagcagcagcaacaacagcccgggcaggggccggccgagccgtcccaacccagtggccccgc tgtcgccagcctcgagccgccggtcaagctcaaggaactgcacttctccaacatgaagaccgtggactgtgtggagcgcaa gggcaagtacatgtacttcactgtggtgatggcagagggcaaggagatcgactttcggtgcccgcaagaccagggctggaa cgccgagatcacgctgcagatggtgcagtacaagaatcgtcaggccatcctggcggtcaaatccacgcggcagaagcagc agcacctggtccagcagcagccccctcgcagccgcagccgcagccgcagctccagccccaaccccagcctcagcctca gccgcaaccccagccccaatcacaaccccagcctcagccccaacccaagcctcagccccagcagctccacccgtatccgc atccacatccacatccacactctcatcctcactcgcacccacaccctcacccgcacccgcatccgcaccaaataccgcaccc acacccacagccgcactcgcagccgcacgggcaccggcttctccgcagcacctccaactctgcctga | |
| Gene symbol: | PIAS2 |
| Entrez Gene ID: | 9063 |
| dna/ac: | BC015190. 1 |
| sequence: (SEQ ID NO: 19) | |
| atggcggatttcgaagagttgaggaatatggtttctagttttagggtttctgaactacaagtattactaggctttgctgg acggaataaaagtggacgcaagcatgacctcctgatgagggcgctgcatttattgaagagcggctgcagccctgcggttcag attaaaatccgagaattgtatagacgccgatatccacgaactcttgaaggactttctgatttatccacaatcaaatcatcggttttc agtttggatggtggctcatcacctgtagaacctgacttggccgtggctggaatccactcgttgccttccacttcagttacacctca ctcaccatcctctcctgttggttctgtgctgcttcaagatactaagcccacatttgagatgcagcagccatctccccccaattcctcc tgtccatcctgatgtgcagttaaaaaatctgcccttttatgatgtccttgatgttctcatcaagcccacgagtttagttcaaagcagt attcagcgatttcaagagaagtttttattttgctttgacacctcaacaagttagagagatatgcatatccagggatttttgccagg | |
| tggtaggagagattatacagtccaagttcagttgagactttgcctggcagagacaagttgccctcaagaagataactatccaaa tagtctatgtataaaagtaaatggaagctatttcctttgcctggctatgcaccaccgcctaaaaatgggattgaacagaagcgc cctggacgccccttgaatattacatctttagttaggttatcttcagctgtgccaaaccaaatttccatttcttgggcatcagaaattg ggaagaattactctatgtctgtatatcttgtacggcagcttacatcagccatgttattacagagattaaaaatgaaaggtattagaa accctgatcattccagagcactaattaaagaaaaacttactgcagatcctgatagtgaaattgctacaactagccttcgggtatc cttgatgtgccctttaggaaaaatgaggctgacaatcccatgccgtgcagtgacttgtacacatctgcagtgttttgatgctgccc tctatctacaaatgaatgagaaaaagcccacctggatttgtcctgtgtgtgacaaaaaagctgcctatgaaagtctaatattagat gggctttttatggaaattctcaatgactgttctgatgtagatgagatcaaattccaagaagatggttcttggtgtccaatgagaccg aagaaagaagctatgaaagtatccagccaaccgtgtacaaaaatagaaagttcaagcgtcctcagtaagccttgttcagtgac tgtagccagtgaggcaagcaagaagaaagtagatgttattgatcttacaatagaaagctcttctgacgaagaggaagaccctc ctgccaaaaggaaatgcatctttatgtcagaaacacaaagcagcccaaccaaaggggttctcatgtatcagccatcttctgtaa gggtgcccagtgtgacttcggttgatcctgctgctattccgccttcattaacagactactcagtaccattccaccatacgccaata tcaagcatgtcatcagatttgccaggagaacaaagaagaaatgatattaataatgaactgaagcttggaacatcttctgatactg tgcaacagtga | |
| Gene symbol: | PRKCBP1 |
| Entrez Gene ID: | 23613 |
| dna/ac: | BC030721.1 |
| sequence: (SEQ ID NO: 20) | |
| atgacaatgctcaccattgaacagttatcctacctgctcaagtttgccattcagaaaatgaaacagccagggacaga tgcattccagaagcccgttccattggaacagcaccctgactatgcggaatacatcttccatccaatggacctttgtacattggaa aagaatgcgaaaaagaaaatgtatggctgcacagaagccttcctggctgatgcaaagtggattttgcacaactgcatcatttat aatggggggaaatcacaaattgacgcaaatagcgaaagtagtcatcaaaatctgtgaacatgagatgaatgaaatcgaagtatg tccagaatgttatctagctgcttgccaaaaacgagataactggttttgtgagccttgtagcaatccacatcctttggtctgggcca aactgaaggggtttccattctggcctgcaaaagctctaagggataaagacgggcaggtcgatgcccgattctttggacaacat gacagggcctgggttccaataaataattgctacctcatgtctaaagaaattcctttttctgtgaaaaagactaagagcatcttcaa cagtgccatgcaagagatggaggtttacgtggagaacatccgcaggaagtttggggtttttaattactctccatttaggacaccc tacacacccaacagccagtatcaaatgctgctcgatcccaccaaccccagcgccggcactgccaagatagacaagcagga gaaggtcaagctcaactttgacatgacggcatcccccaagatcctgatgagcaagcctgtgctgagtgggggcacaggccg ccggatttccttgtcggatatgccgcgctcccccatgagcacaaactcttctgtgcacacgggctccgacgtggagcaggatg ctgagaagaaggccacgtcgagccacttcagtgcgagcgaggagtccatggacttcctggataagagcacagcttcaccag cctccaccaagacgggacaagcagggagtttatccggcagcccaaagcccttctctcctcaactgtcagctcctatcacgac gaaaacggacaaaacctccaccaccggcagcatcctgaatcttaacctggatcgaagcaaagctgagatggatttgaagga gctgagcgagtcggtccagcaacagtccacccctgttcctctcatctctcccaagcgccagattcgtagcaggttccagctga atcttgacaagaccatagagagttgcaaagcacaattaggcataaatgaaatctcggaagatgtctatacggccgtagagcac agcgattcggaggattctgagaagtcagatagtagcgatagtgagtatatcagtgatgatgagcagaagtctaagaacgagc cagaagacacagaggacaaagaaggttgtcagatggacaaagagccatctgctgttaaaaaaaagcccaagcctacaaac ccagtggagattaaagaggagctgaaaagcacgtcaccagccagcgagaaggcagaccctggagcagtcaaggacaag gccagccctgagcctgagaaggacttttccgaaaaggcaaaaccttcacctcaccccataaaggataaactgaagggaaaa gatgagacggattccccaacagtccatttgggcctggactctgattcagagagcgaacttgtcatagatttaggagaagaccat tctgggcgggagggtcgaaaaaataagaaggaacccaaagaaccatctcccaaacaggatgttgtaggtaaaactccacca tccacgacggtgggcagccattctcccccggaaacaccggtgctcacccgctcttccgcccaaacttccgcggctggcgcc acagccaccaccagcacgtcctccacggtcaccgtcacggccccggcccccgccgccacaggaagcccagtgaaaaag cagaggccgcttttaccgaaggagactgccccggccgtgcagcgggtcgtgtggaactcatcaactgtccagcagaagga gatcacacagagcccatccacgtccaccatcaccctggtgaccagcacacagtcatcgcccctggtcaccagctcggggtc catgagcacccttgtgtcctcagtcaacgctgacctgcccatcgccactgcctcagctgatgtcgccgctgatattgccaagta | |
| cactagcaaaatgatggatgcaataaaaggaacaatgacagaaatatacaacgatctttctaaaaacactactggaagcacaa tagctgagattcgcaggctgaggatcgagatagagaagctccagtggctgcaccagcaagagctctccgaaatgaaacaca acttagagctgaccatggcggagatgcggcagagcctggagcaggagcgggaccggctcatcgccgaggtgaagaagc agctggagttggagaagcagcaggcggtggatgagaccaagaagaagcagtggtgcgccaactgcaagaaggaggcca tcttttactgctgttggaacaccagctactgtgactacccctgccagcaagcccactggcctgagcacatgaagtcctgcaccc agtcagctactgctcctcagcaggaagcggatgctgaggtgaacacagaaacactaaataagtcctcccaggggagctcct cgagcacacaatcagcaccttcagaaacggccagcgcctccaaagagaaggagacgtcagctgagaaaagcaaggaga gtggctcgacccttgacctttctggctccagagagacgccctcctccattctcttaggctccaaccaaggctctgttagcaaaa ggtgtgacaagcaacctgcctatgccccaaccaccacagaccaccagccgcaccccaactaccccgcccagaagtaccatt cccggagtaataaatccagttggagcagcagtgatgagaagaggggatcgacacgttccgatcacaacaccagtaccagca cgaagagcctcctcccgaaagagtctcggctggacaccttctgggactag | |
| Gene symbol: | PRKRA |
| Entrez Gene ID: | 8575 |
| dna/ac: | BC009470.1 |
| sequence: (SEQ ID NO: 21) | |
| atgtcccagagcaggcaccgcgccgaggccccgccgctggagcgcgaggacagtgggaccttcagtttgggg aagatgataacagctaagccagggaaaacaccgattcaggtattacacgaatacggcatgaagaccaagaacatcccagttt atgaatgtgaaagatctgatgtgcaaatacacgtgcccactttcaccttcagagtaaccgttggtgacataacctgcacaggtg aaggtacaagtaagaagctggcgaaacatagagctgcagaggctgccataaacattttgaaagccaatgcaagtatttgcttt gcagttcctgaccccttaatgcctgacccttccaagcaaccaaagaaccagcttaatcctattggttcattacaggaattggctat tcatcatggctggagacttcctgaatatacccttcccaggagggaggacctgctcataagagagaatatactacaatttgcag gctagagtcatttatggaaactggaaagggggcatcaaaaaagcaagccaaaaggaatgctgctgagaaatttcttgccaaat ttagtaatatttctccagagaaccacatttctttaacaaatgtagtaggacattctttaggatgtacttggcattccttgaggaattct cctggtgaaaagatcaacttactgaaaagaagcctccttagtattccaaatacagattacatccagctgcttagtgaaattgcca aggaacaaggttttaatataacatatttggatatagatgaactgagcgccaatggacaatatcaatgtcttgctgaactgtccacc agccccatcacagtctgtcatggctccggtatctcctgtggcaatgcacaaagtgatgcagctcacaatgctttgcagtatttaa agataatagcagaaagaaagtaa | |
| Gene symbol: | PSME3 |
| Entrez Gene ID: | 10197 |
| dna/ac: | BC001423.2 |
| sequence: (SEQ ID NO: 22) | |
| atggcctcgttgctgaaggtggatcaggaagtgaagctcaaggttgattctttcagggagcggatcacaagtgag gcagaagacttggtggcaaatttttcccaaagaagttattagaacttgatagttttctgaaggaaccaatcttaaacatccatgac ctaactcagatccactctgacatgaatctcccagtccctgaccccattcttctcaccaatagccatgatggactggatggtccca cttataagaagcgaaggttggatgagtgtgaagaagccttccaaggaaccaaggtgtttgtgatgcccaatgggatgctgaaa agcaaccagcagctggtggacattattgagaaagtgaaacctgagatccggctgttgattgagaaatgtaacacggtcaaaat gtgggtacagctcctgattcccaggatagaagatggaaacaactttggggtgtccattcaggaggaaacagttgcagagcta agaactgttgagagtgaagctgcatcttatctggaccagatttctagatattatattacaagagccaaattggtttctaaaatagct aaatatccccatgtggaggactatcgccgcaccgtgacagagattgatgagaaagaatatatcagccttcggctcatcatatca gagctgaggaatcaatatgtcactctacatgacatgatcctgaaaaatatcgagaagatcaaacggccccggagcagcaatg cagagactctgtactga | |
| Gene symbol: | RAB11FIP3 |
| Entrez Gene ID: | 9727 |
| dna/ac: | BC051360.1 |
| sequence: (SEQ ID NO: 23) | |
| atggcgtcggccccgccggcctcgcccccgggctcggagccgccggggcccgacccggagccgggcgggc cggacgggccgggggcggcgcaactggctccgggccctgcggagctacgcctcggagcgcccgtcggcggccccgac ccgcagtccccgggcctggatgagcctgcgcccggggccgctgcagatggcggggcgcgttggagcgccgggccggc cccggggctggagggaggcccgcgagaccccgggccgtccgccccgccgccgcgctccggcccgcgggggcagcttg cgagccccgacgccccgggcccagggccgcgctccgaagcgccgcttccagaactcgacccgttgttctcctggactgag gagcccgaggagtgtggccccgcgagctgcccggagagcgcgcctttccgcttgcaggggtccagcagcagccaccga gcgcggggcgaggtcgacgtcttctctcccttccccgcgcccacggcgggcgagctggcgctggagcaaggtcccgggt ccccgccgcagccctcggacctcagccagacccacccccttccgagcgagcccgtggggagtcaggaggacggccccc gcctccgagccgtgttcgatgccctggacggggatggggacggtttcgtccgcatcgaggacttcatccagtttgctacggtc tacggggcagagcaggtgaaggacttaactaagtacttggatcccagtgggctcggcgtgatcagctttgaagacttctacca agggatcacagccatcagaaacggagatcctgatggccagtgctacggtggtgtcgcttctgcccaagatgaggagcccct ggcctgcccggacgagttcgatgacttcgtcacctatgaggccaacgaggtgacggacagcgcgtacatgggctccgaga gcacctacagtgagtgtgagaccttcacggacgaggacaccagcaccctggtgcaccctgagctgcaacctgaaggggac gcagacagtgccggcggctcggccgtgccctctgagtgcctggacgccatggaggagcccgaccatggtgccctgctgct gctcccaggcaggcctcacccccatggccagtctgtcatcacggtgatcgggggcgaggagcactttgaggactacggtga aggcagtgaggcggagctgtccccagagaccctatgcaacgggcagctgggctgcagtgaccccgctttcctcacgccca gtccgacaaagcggctctccagcaagaaggtggcaaggtacctgcaccagtcaggggccctgaccatggaggccctgga ggacccttcccccgagctcatggagggcccagaggaggacattgctgacaaggttgtcttcctggaaaggcgtgtgctgga gctggaaaaggacacggcagccaccggtgagcaacacagccgcctgaggcaggagaacctgcagctggtgcacagagc aaacgccctggaggagcagctgaaggagcaggagctgagagcctgcgagatggtcctggaagagacccggcgtcagaa ggagctcctgtgcaagatggagagggagaagagcattgagatcgagaacctgcagaccaggctacagcaactggacgag gagaacagtgaactccggtcctgcacgccctgtctgaaggccaacattgagcgtctggaggaggagaagcagaagctgttg gatgagatagagtcgctgacgctgcggctcagtgaagagcaggagaacaagaggagaatgggggacaggctgagtcacg agaggcaccagttccagagggacaaggaggccacccaggagctgatcgaggacctccgaaagcagctggagcacctgc agctcctcaagctggaggccgagcagcggcggggccgcagcagcagcatgggcctgcaggagtaccacagccgcgcc cgggagagcgagctggagcaggaggtccgcaggctgaagcaggacaaccgcaacctgaaggagcagaacgaggagct gaacgggcagatcattaccctcagcatccagggcgccaagagcctcttctccacagccttctctgagtccctggctgcagag atcagctccgtctcccgagatgagctcatggaggcgattcagaagcaggaggagatcaacttccgcctgcaggactacatcg acaggatcatcgtggccatcatggagaccaacccgtccatcctggaggtcaagtag | |
| Gene symbol: | RALBP1 |
| Entrez Gene ID: | 10928 |
| dna/ac: | BC013126.1 |
| sequence: (SEQ ID NO: 24) | |
| atgactgagtgcttcctgccccccaccagcagccccagtgaacaccgcagggtggagcatggcagcgggctta cccggacccccagctctgaagagatcagccctactaagtttcctggattgtaccgcactggcgagccctcacctccccatgac atcctccatgagcctcctgatgtagtgtctgatgatgagaaagatcatgggaagaaaaaagggaaatttaagaaaaaggaaaa gaggactgaaggctatgcagcctttcaggaagatagctctggagatgaggcagaaagtccttctaaaatgaagaggtccaag | |
| ggaatccatgttttcaagaagcccagcttttctaaaaagaaggaaaaggattttaaaataaaagagaaacccaaagaagaaaa gcataaagaagaaaagcacaaagaagaaaaacataaagagaagaagtcaaaagacttgacagcagctgatgttgttaaaca gtggaaggaaaagaagaaaaagaaaaagccaattcaggagccagaggtgcctcagattgatgttccaaatctcaaacccatt tttggaattcctttggctgatgcagtagagaggaccatgatgtatgatggcattcggctgccagccgttttccgtgaatgtataga ttacgtagagaagtatggcatgaagtgtgaaggcatctacagagtatcaggaattaaatcaaaggtggatgagctaaaagcag cctatgaccgggaggagtctacaaacttggaagactatgagcctaacactgtagccagtttgctgaagcagtatttgcgagac cttccagagaatttgcttaccaaagagcttatgcccagatttgaagaggcttgtgggaggaccacggagactgagaaagtgca ggaattccagcgtttactcaaagaactgccagaatgtaactatcttctgatttcttggctcattgtgcacatggaccatgtcattgc aaaggaactggaaacaaaaatgaatatacagaacatttctatagtgctcagcccaactgtgcagatcagcaatcgagtcctgta tgtgtttttcacacatgtgcaagaactctttggaaatgtggtactaaagcaagtgatgaaacctctgcgatggtctaacatggcca cgatgcccacgctgccagagacccaggcgggcatcaaggaggagatcaggagacaggagtttcttttgaattgtttacatcg agatctgcagggtgggataaaggatttgtctaaagaagaaagattatgggaagtacaaagaattttgacagccctcaaaagaa aactgagagaagctaaaagacaggagtgtgaaaccaagattgcacaagagatagccagtctttcaaaagaggatgtttccaa agaagagatgaatgaaaatgaagaagttataaatattctccttgctcaggagaatgagatcctgactgaacaggaggagctcc tggccatggagcagtttctgcgccggcagattgcctcagaaaaagaagagattgaacgcctcagagctgagattgctgaaatt cagagtcgccagcagcacggccgaagtgagactgaggagtactcctccgagagcgagagcgagagtgaggatgaggag gagctgcagatcattctggaagacttacagagacagaacgaagagctggaaataaagaacaatcatttgaatcaagcaattca tgaggagcgcgaggccatcatcgagctgcgcgtgcagctgcggctgctccagatgcagcgagccaaggccgagcagca ggcgcaggaggacgaggagcctgagtggcgcgggggtgccgtccagccgcccagagacggcgtccttgagccaaaag cagctaaagagcagccaaaggcaggcaaggagccggcaaagccatcgcccagcagggataggaaggagacgtccatct ga | |
| Gene symbol: | RAN |
| Entrez Gene ID: | 5901 |
| dna/ac: | BC014518.2 |
| sequence: (SEQ ID NO: 25) | |
| atggctgcgcagggagagccccaggtccagttcaaacttgtattggttggtgatggtggtactggaaaaacgacct tcgtgaaacgtcatttgactggtgaatttgagaagaagtatgtagccaccttgggtgttgaggttcatcccctagtgttccacacc aacagaggacctattaagttcaatgtatgggacacagccggccaggagaaattcggtggactgagagatggctattatatcca agcccagtgtgccatcataatgtttgatgtaacatcgagagttacttacaagaatgtgcctaactggcatagagatctggtacga gtgtgtgaaaacatccccattgtgttgtgtggcaacaaagtggatattaaggacaggaaagtgaaggcgaaatccattgtcttc caccgaaagaagaatcttcagtactacgacatttctgccaaaagtaactacaactttgaaaagcccttcctctggcttgctagga agctcattggagaccctaacttggaatttgttgccatgcctgctctcgccccaccagaagttgtcatggacccagctttggcagc acagtatgagcacgacttagaggttgctcagacaactgctctcccggatgaggatgatgacctgtga | |
| Gene symbol: | RARA |
| Entrez Gene ID: | 5914 |
| dna/ac: | BC008727.2 |
| sequence: (SEQ ID NO: 26) | |
| atggccagcaacagcagctcctgcccgacacctgggggcgggcacctcaatgggtacccggtgcctccctacg ccttcttcttccccccctatgctgggtggactctccccgccaggcgctctgaccactctccagcaccagcttccagttagtggata tagcacaccatccccagccaccattgagacccagagcagcagttctgaagagatagtgcccagccctccctcgccaccccc tctaccccgcatctacaagccttgctttgtctgtcaggacaagtcctcaggctaccactatggggtcagcgcctgtgagggctg caagggcttcttccgccgcagcatccagaagaacatggtgtacacgtgtcaccgggacaagaactgcatcatcaacaaggt | |
| gacccggaaccgctgccagtactgccgactgcagaagtgctttgaagtgggcatgtccaaggagtctgtgagaaacgaccg aaacaagaagaagaaggaggtgcccaagcccgagtgctctgagagctacacgctgacgccggaggtgggggagctcatt gagaaggtgcgcaaagcgcaccaggaaaccttccctgccctctgccagctgggcaaatacactacgaacaacagctcaga acaacgtgtctctctggacattgacctctgggacaagttcagtgaactctccaccaagtgcatcattaagactgtggagttcgcc aagcagctgcccggcttcaccaccctcaccatcgccgaccagatcaccctcctcaaggctgcctgcctggacatcctgatcct gcggatctgcacgcggtacacgcccgagcaggacaccatgaccttctcggacgggctgaccctgaaccggacccagatgc acaacgctggcttcggccccctcaccgacctggtctttgccttcgccaaccagctgctgcccctggagatggatgatgcgga gacggggctgctcagcgccatctgcctcatctgcggagaccgccaggacctggagcagccggaccgggtggacatgctg caggagccgctgctggaggcgctaaaggtctacgtgcggaagcggaggcccagccgcccccacatgttccccaagatgct aatgaagattactgacctgcgaagcatcagcgccaagggggctgagcgggtgatcacgctgaagatggagatcccgggct ccatgccgcctctcatccaggaaatgttggagaactcagagggcctggacactctgagcggacagccgggggggtggggg gcgggacgggggtggcctggcccccccgccaggcagctgtagccccagcctcagccccagctccaacagaagcagccc ggccacccactccccgtga | |
| Gene symbol: | RBMS 1 |
| Entrez Gene ID: | 5937 |
| dna/ac: | BC012992.2 |
| sequence: (SEQ ID NO: 27) | |
| atgggcaaagtgtggaaacagcagatgtaccctcagtacgccacctactattacccccagtatctgcaagccaag cagtctctggtcccagcccaccccatggcccctcccagtcccagcaccaccagcagtaataacaacagtagcagcagtagc aactcaggatgggatcagctcagcaaaacgaacctctatatccgaggactgcctccccacaccaccgaccaggacctggtg aagctctgtcaaccatatgggaaaatagtctccacaaaggcaattttggataagacaacaaacaaatgcaaaggttatggttttg tcgactttgacagccctgcagcagctcaaaaagctgtgtctgccctgaaggccagtggggttcaagctcaaatggcaaagca acaggaacaagatcctaccaacctctacatttctaatttgccactctccatggatgagcaagaactagaaaatatgctcaaacca tttggacaagttatttctacaaggatactacgtgattccagtggtacaagtcgtggtgttggctttgctaggatggaatcaacaga aaaatgtgaagctgttattggtcattttaatggaaaatttattaagacaccaccaggagtttctgcccccacagaacctttattgtgt aagtttgctgatggaggacagaaaaagagacagaacccaaacaaatacatccctaatggaagaccatggcatagagaagg agaggctggaatgacacttacttacgacccaactacagctgctatacagaacggattttatccttcaccatacagtattgctaca aaccgaatgatcactcaaacttctattacaccctatattgcatctcctgtatctgcctaccaggtgcaaagtccttcgtggatgcaa cctcaaccatatattctacagcaccctggtgccgtgttaactccctcaatggagcacaccatgtcactacagcccgcatcaatg atcagccctctggcccagcagatgagtcatctgtcactaggcagcaccggaacatacatgcctgcaacgtcagctatgcaag gagcctacttgccacagtatgcacatatgcagacgacagcggttcctgttgaggaggcaagtggtcaacagcaggtggctgt cgagacgtctaatgaccattctccatatacctttcaacctaataagtaa | |
| Gene symbol: | RDBP |
| Entrez Gene ID: | 7936 |
| dna/ac: | BC050617.2 |
| sequence: (SEQ ID NO: 28) | |
| atgttggtgataccccccggactgagcgaggaagaggaggctctgcagaagaaattcaacaagctcaagaaaaa gaaaaaggcattgctggctctgaagaagcaaagtagcagcagcacaaccagccaaggtggtgtcaaacgctcactatcaga gcagcctgtcatggacacagccacagcaacagagcaggcaaagcagctggtgaagtcaggagccatcagtgccatcaag | |
| gctgagaccaagaactcaggcttcaagcgttctcgaacccttgaggggaagttaaaggaccccgagaaggaccagtccc cactttccagccgttccagaggagcatatctgctgatgatgacctgcaagagtcatccagacgtccccagaggaaatctctgta tgagagctttgtgtcttctagtgatcgacttcgagaactaggaccagatggagaagaggcagagggcccaggggctggtgat ggtccccctcgaagctttgactggggctatgaagaacgcagtggtgcccactcctcagcctcccctccccgaagccgcagc cgggaccgcagccatgagaggaaccgggacagagaccgagatcgggagcgggatcgagaccgggatcgagacagag acagagagcgggacagggatcgggatcgggatcgagatcgagaccgggaacgggacagggatcgggagcgggatcga gaccgagaccgagagggtcctttccgcaggtcggattcattccctgaacggcgagcccctaggaaaggaatactctctatg tatatggagaagacatgacacccacccttctccgtgggccttctctccttttggaaacatcattgacctctccatggacccacc cagaaactgtgccttcgtcacctatgaaaagatggagtcagcagatcaggccgttgctgagctcaacgggacccaggtgga gtctgtacagctcaaagtcaacatagcccgaaaacagcccatgctggatgccgctactggcaagtctgtctggggctccctcg ctgtccagaacagccctaagggttgccaccgggacaagaggacccagattgtctacagtgatgacgtctacaaggaaaacc ttgtggatggcttctag | |
| Gene symbol: | RNF 12 |
| Entrez Gene ID: | 51132 |
| dna/ac: | BC013357.2 |
| sequence: (SEO ID NO: 29) | |
| atggaaaactcagattccaatgacaaaggaagtggtgatcagtctgcagcacagcgcagaagtcagatggaccg attggatcgagaagaagctttctatcaatttgtaaataacctgagtgaagaagattataggcttatgagagataacaatttgctag gcaccccaggtgaaagtactgaggaagagttgctgagacgactacagcaaattaaagaaggcccaccaccgcaaaactca gatgaaaatagaggaggagactcttcagatgatgtgtctaatggtgactctataatagactggcttaactctgtcagacaaactg gaaatacaacaagaagtgggcaaagaggaaaccaatcttggagagcagtgagtcggactaatccaaacagtggtgatttca gattcagtttagagataaatgttaaccgtaataatgggagccaaaattcagagaatgaaaatgagccatctgcaagacgttcta gtggagaaaatgtggaaaacaacagccaaaggcaagtggaaaacccacgatctgaatcaacatctgcaaggccatctagat cagaacgaaattcaactgaagcgttaacagaggtcccacctaccagaggtcagaggagggcaagaagcaggagcccaga ccatcggagaaccagagcaagagctgaaagaagtaggtcacctctgcatccaatgagtgaaattccacgaagatctcatcat agtatctcatctcagacttttgaacatcctttggtaaatgagacggagggaagttctagaacccggcaccatgtgacattgagg cagcaaatatctgggcctgagttgctaagtagaggtctttttgcagcttctggaacaagaaatgcttctcaaggagcaggttctt cagacacagctgccagtggtgaatctacaggatcaggacagagacctccaaccatagtccttgatcttcaagtaagaagagtt cgtcctggagaatatcggcagagagatagcatagccagcagaactcggtctaggtctcagacaccaaacaacactgtcacct atgaaagtgaacgaggaggttttaggcgtacattttcacgttctgagcgggcaggtgtgagaacctatgtcagtaccatcagaa ttcccattcgtagaatcttaaatactggtttaagtgagactacatctgttgcaattcagaccatgttaaggcagataatgacaggttt tggtgagttaagctattttatgtacagtgatagcgactcagagcctactggctcagtctcaaatcgaaatatggaaagggcaga gtcacggagtggaagaggaggttctggtggtggtagtagttctggttccagttcgagttccagttccagttcgagttccagttcc agttcaagttccagttccagtcctagttccagttccggtggtgaaagttcagaaactagctcagatttatttgaaggcagtaatga aggaagctcatcatcaggctcatcaggtgccaggcgagagggtcgacatagggccccagtcacatttgatgaaagtggctct ttgcccttccttagcctggctcagtttttcctcttaaatgaggatgatgatgaccaacctagaggactcaccaaagaacagattga caacttggcaatgagaagttttggtgaaaatgatgcattaaaaacctgtagtgtttgcattacagaatatacagaaggcaacaaa cttcgtaaactaccttgttcccatgagtaccatgtccactgcatcgatcgctggttatctgagaattctacctgtcctatttgtcgca gagcagtcttagcttctggtaacagagaaagtgttgtgtaa | |
| Gene symbol: | RPL30 |
| Entrez Gene ID: | 6156 |
| dna/ac: | BC032700.2 |
| sequence: (SEQ ID NO: 30) | |
| atggtggccgcaaagaagacgaaaaagtcgctggagtcgatcaactctaggctccaactcgttatgaaaagtggg aagtacgtcctggggtacaagcagactctgaagatgatcagacaaggcaaagcgaaattggtcattctcgctaacaactgcc cagctttgaggaaatctgaaatagagtactatgctatgttggctaaaactggtgtccatcactacagtggcaataatattgaactg ggcacagcatgcggaaaatactacagagtgtgcacactggctatcattgatccaggtgactctgacatcattagaagcatgcc agaacagactggtgaaaagtaa | |
| Gene symbol: | RPL31 |
| Entrez Gene ID: | 6160 |
| dna/ac: | BC017343.2 |
| sequence: (SEQ ID NO: 31) | |
| atggctcccgcaaagaagggtggcgagaagaaaaagggccgttctgccatcaacgaagtggtaacccgagaat acaccatcaacattcacaagcgcatccatggagtgggcttcaagaagcgtgcacctcgggcactcaaagagattcggaaatt tgccatgaaggagatgggaactccagatgtgcgcattgacaccaggctcaacaaagctgtctgggccaaaggaataaggaa tgtgccataccgaatccgtgtgcggctgtccagaaaacgtaatgaggatgaagattcaccaaataagctatatactttggttacc tatgtacctgttaccactttcaaaaatctacagacagtcaatgtggatgagaactaa | |
| Gene symbol: | RUFY1 |
| Entrez Gene ID: | 80230 |
| dna/ac: | BC032571.1 |
| sequence: (SEQ ID NO: 32) | |
| atgatggaggagcgtgccaacctgatgcacatgatgaaactcagcatcaaggtgttgctccagtcggctctgagc ctgggccgcagcctggatgcggaccatgccccttgcagcagttctttgtagtgatggagcactgcctcaaacatgggctga aagttaagaagagttttattggccaaaataaatcattctttggtcctttggagctggtggagaaactttgtccagaagcatcagat atagcgactagtgtcagaaatcttccagaattaaagacagctgtgggaagaggccgagcgtggctttatcttgcactcatgcaa aagaaactggcagattatctgaaagtgcttatagacaataaacatctcttaagcgagttctatgagcctgaggctttaatgatgg aggaagaagggatggtgattgttggtctgctggtgggactcaatgttctcgatgccaatctctgcttgaaaggagaagacttgg attctcaggttggagtaatagatttttccctctaccttaaggatgtgcaggatcttgatggtggcaaggagcatgaaagaattact gatgtccttgatcaaaaaaattatgtggaagaacttaaccggcacttgagctgcacagttggggatcttcaaaccaagatagat ggcttggaaaagactaactcaaagcttcaagaagagctttcagctgcaacagaccgaatttgctcacttcaagaagaacagca gcagttaagagaacaaaatgaattaattcgagaaagaagtgaaaagagtgtagagataacaaaacaggataccaaagttgag ctggagacttacaagcaaactcggcaaggtctggatgaaatgtacagtgatgtgtggaagcagctaaaagaggagaagaaa gtccggttggaactggaaaaagaactggagttacaaattggaatgaaaaccgaaatggaaattgcaatgaagttactggaaa aggacacccacgagaagcaggacacactagttgccctccgccagcagctggaagaagtcaaagcgattaatttacagatgtt tcacaaagctcagaatgcagagagcagtttgcagcagaagaatgaagccatcacatcctttgaaggaaaaaccaaccaagtt atgtccagcatgaaacaaatggaagaaaggttgcagcactcggagcgggcgaggcagggagctgaggagcggagccac aagctgcagcaggagctgggcgggaggatcggcgccctgcagctgcagctctcccagctgcacgagcaatgctcaagcct ggagaaagaattgaaatcagaaaaagagcaaagacaggctcttcagcgcgaattacagcacgagaaagacacttcctctct actcaggatggagctgcaacaagtggaaggactgaaaaaggagttgcgggagcttcaggacgagaaggcagagctgcag aagatctgcgaggagcaggaacaagccctccaggaaatgggcctgcacctcagccagtccaagctgaagatggaagatat aaaagaagtgaaccaggcactgaagggccacgcctggctgaaagatgacgaagcgacacactgtaggcagtgtgagaag | |
| gagttctccatttcccggagaaagcaccactgccggaactgtggccacatcttctgcaacacctgctccagcaacgagctgg ccctgccctcctaccccaagccggtgcgagtgtgcgacagctgccacaccctgctcctgcagcgctgctcctccacggcctc ctga | |
| Gene symbol: | SMN1 |
| Entrez Gene ID: | 6606 |
| dna/ac: | BC000908.2 |
| sequence: (SEQ ID NO: 33) | |
| atggcgatgagcagcggcggcagtggtggcggcgtcccggagcaggaggattccgtgctgttccggcgcggc acaggccagagcgatgattctgacatttgggatgatacagcactgataaaagcatatgataaagctgtggcttcatttaagcat gctctaaagaatggtgacatttgtgaaacttcgggtaaaccaaaaaccacacctaaaagaaaacctgctaagaagaataaaag ccaaaagaagaatactgcagcttccttacaacagtggaaagttggggacaaatgttctgccatttggtcagaagacggttgcat ttacccagctaccattgcttcaattgattttaagagagaaacctgtgttgtggtttacactggatatggaaatagagaggagcaaa atctgtccgatctactttccccaatctgtgaagtagctaataatatagaacagaatgctcaagagaatgaaaatgaaagccaagt ttcaacagatgaaagtgagaactccaggtctcctggaaataaatcagataacatcaagcccaaatctgctccatggaactctttt ctccctccaccacccccccatgccagggccaagactgggaccaggaaagccaggtctaaaattcaatggcccaccaccgcc accgccaccaccaccaccccacttactatcatgctggctgcctccatttccttctggaccaccaataattcccccaccacctccc atatgtccagattctcttgatgatgctgatgctttgggaagtatgttaatttcatggtacatgagtggctatcatactggctattatat ggaaatgctggcatag | |
| Gene symbol: | SNK |
| Entrez Gene ID: | 10769 |
| dna/ac: | BC013879.2 |
| sequence: (SEQ ID NO: 34) | |
| atggagcttttgcggactatcacctaccagccagccgccagcaccaaaatgtgcgagcaggcgctgggcaagg gttgcggagcagactcgaagaagaagcggccgccgcagcccccgaggaatcgcagccacctcagtcccaggcgcaag tgcccccggcggcccctcaccaccatcaccaccattcgcactcggggccggagatctcgcggattatcgtcgaccccacga ctgggaagcgctactgccggggcaaagtgctgggaaagggtggctttgcaaaatgttacgagatgacagatttgacaaataa caaagtctacgccgcaaaaattattcctcacagcagagtagctaaacctcatcaaagggaaaagattgacaaagaaatagag cttcacagaattcttcatcataagcatgtagtgcagttttaccactacttcgaggacaaagaaaacatttacattctcttggaatact gcagtagaaggtcaatggctcatattttgaaagcaagaaaggtgttgacagagccagaagttcgatactacctcaggcagatt gtgtctggactgaaataccttcatgaacaagaaatcttgcacagagatctcaaactagggaactttttattaatgaagccatgga actaaaagttggggacttcggtctggcagccaggctagaacccttggaacacagaaggagaacgatatgtggtaccccaaat tatctctctcctgaagtcctcaacaaacaaggacatggctgtgaatcagacatttgggccctgggctgtgtaatgtatacaatgtt actagggaggcccccatttgaaactacaaatctcaaagaaacttataggtgcataagggaagcaaggtatacaatgccgtcct cattgctggctcctgccaagcacttaattgctagtatgttgtccaaaaacccagaggatcgtcccagtttggatgacatcattcga catgactttttttgcagggcttcactccggacagactgtcttctagctgttgtcatacagttccagatttccacttatcaagcccag ctaagaatttctttaagaaagcagctgctgctctttttggtggcaaaaaagacaaagcaagatatattgacacacataatagagt gtctaaagaagatgaagacatctacaagcttaggcatgatttgaaaaagacttcaataactcagcaacccagcaaacacagga cagatgaggagctccagccacctaccaccacagttgccaggtctggaacacccgcagtagaaaacaagcagcagattggg gatgctattcggatgatagtcagagggactcttggcagctgtagcagcagcagtgaatgccttgaagacagtaccatgggaa gtgttgcagacacagtggcaagggttcttcggggatgtctggaaaacatgccggaagctgattgcattcccaaagagcagct gagcacatcatttcagtgggtcaccaaatgggttgattactctaacaaatatggctttgggtaccagctctcagaccacaccgtc ggtgtccttttcaacaatggtgctcacatgagcctccttccagacaaaaaaacagttcactattacgcagagcttggccaatgct | |
| cagttttcccagcaacagatgctcctgagcaatttattagtcaagtgacggtgctgaaatacttttctcattacatggaggagaac ctcatggatggtggagatctgcctagtgttactgatattcgaagacctcggctctacctccttcagtggctaaaatctgataaggc cctaatgatgctctttaatgatggcacctttcaggtgaatttctaccatgatcatacaaaaatcatcatctgtagccaaaatgaaga ataccttctcacctacatcaatgaggataggatatctacaactttcaggctgacaactctgctgatgtctggctgttcatcagaatt aaaaaatcgaatggaatatgccctgaacatgctcttacaaagatgtaactga | |
| Gene symbol: | SRPK1 |
| Entrez Gene ID: | 6732 |
| dna/ac: | BC038292.1 |
| sequence: (SEO ID NO: 35) | |
| atggagcggaaagtgcttgcgctccaggcccgaaagaaaaggaccaaggccaagaaggacaaagcccaaag gaaatctgaaactcagcaccgaggctctgctccccactctgagagtgatctaccagagcaggaagaggagattctgggatct gatgatgatgagcaagaagatcctaatgattattgtaaaggaggttatcatcttgtgaaaattggagatctattcaatgggagata ccatgtgatccgaaagttaggctggggacacttttcaacagtatggttatcatgggatattcaggggaagaaatttgtggcaatg aaagtagttaaaagtgctgaacattacactgaaacagcactagatgaaatccggttgctgaagtcagttcgcaattcagaccct aatgatccaaatagagaaatggttgttcaactactagatgactttaaaatatcaggagttaatggaacacatatctgcatggtattt gaagttttggggcatcatctgctcaagtggatcatcaaatccaattatcaggggcttccactgccttgtgtcaaaaaaattattca gcaagtgttacagggtcttgattatttacataccaagtgccgtatcacccacactgacattaaaccagagaacatcttattgtcag tgaatgagcagtacattcggaggctggctgcagaagcaacagaatggcagcgatctggagctcctccgccttccggatctgc agtcagtactgctccccagcctaaaccagctgacaaaatgtcaaagaataagaagaagaaattgaagaagaagcagaagcg ccaggcagaattactagagaagcgaatgcaggaaattgaggaaatggagaaagagtcgggccctgggcaaaaaagacca aacaagcaagaagaatcagagagtcctgttgaaagacccttgaaagagaacccacctaataaaatgacccaagaaaaactt gaagagtcaagtaccattggccaggatcaaacgcttatggaacgtgatacagagggtggtgcagcagaaattaattgcaatg gagtgattgaagtcattaattatactcagaacagtaataatgaaacattgagacataaagaggatctacataatgctaatgactgt gatgtccaaaatttgaatcaggaatctagtttcctaagctcccaaaatggagacagcagcacatctcaagaaacagactcttgt acacctataacatctgaggtgtcagacaccatggtgtgccagtcttcctcaactgtaggtcagtcattcagtgaacaacacatta gccaacttcaagaaagcattcgggcagagataccctgtgaagatgaacaagagcaagaacataacggaccactggacaac aaaggaaaatccacggctggaaattttcttgttaatccccttgagccaaaaaatgcagaaaagctcaaggtgaagattgctgac cttggaaatgcttgttgggtgcacaaacatttcactgaagatattcaaacaaggcaatatcgttccttggaagttctaatcggatct ggctataatacccctgctgacatttggagcacggcatgcatggcctttgaactggccacaggtgactatttgtttgaacctcattc aggggaagagtacactcgagatgaagatcacattgcattgatcatagaacttctggggaaggtgcctcgcaagctcattgtgg caggaaaatattccaaggaattttcaccaaaaaaggtgacctgaaacatatcacgaagctgaaaccttggggcctttttgagg ttctagtggagaagtatgagtggtcgcaggaagaggcagctggcttcacagatttcttactgcccatgttggagctgatccctg agaagagagccactgccgccgagtgtctccggcacccttggcttaactcctaa | |
| Gene symbol: | SSA2 |
| Entrez Gene ID: | 6738 |
| dna/ac: | BC036658.2 |
| sequence: (SEQ ID NO: 36) | |
| atggaggaatctgtaaaccaaatgcagccactgaatgagaagcagatagccaattctcaggatggatatgtatggc aagtcactgacatgaatcgactacaccggttcttatgtttcggttctgaaggtgggacttattatatcaaagaacagaagttgggc | |
| cttgaaaatgctgaagctttaattagattgattgaagatggcagaggatgtgaagtgatacaagaaataaagtcatttagtcaag aaggcagaaccacaaagcaagagcctatgctctttgcacttgccatttgttcccagtgctccgacataagcacaaaacaagca gcatttaaagctgtttctgaagtttgtcgcattcctacccatctctttactttatccagtttaagaaagatctgaaggaaagcatgaa atgtggcatgtggggtcgtgccctccggaaggctatagcggactggtacaatgagaaaggtggcatggcccttgctctggca gttacaaaatataaacagagaaatggctggtctcacaaagatctattaagattgtcacatcttaaaccttccagtgaaggacttgc aattgtgaccaaatatattacaaagggctggaaagaagttcatgaattgtataaagaaaaagcactctctgtggagactgaaaa attattaaagtatctggaggctgtagagaaagtgaagcgcacaagagatgagctagaagtcattcatctaatagaagaacata gattagttagagaacatcttttaacaaatcacttaaagtctaaagaggtatggaaggctttgttacaagaaatgccgcttactgca ttactaaggaatctaggaaagatgactgctaattcagtacttgaaccaggaaattcagaagtatctttagtatgtgaaaaactgtg taatgaaaaactattaaaaaaggctcgtatacatccatttcatattttgatcgcattagaaacttacaagacaggtcatggtctcag agggaaactgaagtggcgccctgatgaagaaattttgaaagcattggatgctgcttttataaaacatttaagacagttgaacca actggaaaacgtttcttactagctgttgatgtcagtgcttctatgaaccaaagagttttgggtagtatactcaacgctagtacagtt gctgcagcaatgtgcatggttgtcacacgaacagaaaaagattcttatgtagttgctttttccgatgaaatggtaccatgtccagt gactacagatatgaccttacaacaggttttaatggctatgagtcagatcccagcaggtggaactgattgctctcttccaatgatct gggctcagaagacaaacacacctgctgatgtcttcattgtattcactgataatgagacctttgctggaggtgtccatcctgctatt gctctgagggagtatcgaaagaaaatggatattccagctaaattgattgtttgtggaatgacatcaaatggtttcaccattgcaga cccagatgatagaggcatgttggatatgtgcggctttgatactggagctctggatgtaattcgaaatttcacattagatatgattta a | |
| Gene symbol: | SSNA1 |
| Entrez Gene ID: | 8636 |
| dna/ac: | BC000864.1 |
| sequence: (SEQ ID NO: 37) | |
| atgacccagcagggcgcggcgctgcagaactacaacaacgagctggtcaagtgcatagaggagctgtgccag aagcgggaggagctgtgccggcagatccaggaggaggaggacgagaagcagcggctgcagaatgaggtgaggcagct gacagagaagctggcccgcgtcaacgagaacctggcacgcaagattgcctctcgcaacgagttcgaccggaccatcgcgg agacggaggccgcctacctcaagatcctggagagctcccagactttgctcagcgttctcaagagggaagctgggaacctga ccaaggctacagccccagaccagaaaagtagcggcggcagggacagctga | |
| Gene symbol: | STAU1 |
| Entrez Gene ID: | 6780 |
| dna/ac: | BC050432.1 |
| sequence: (SEO ID NO: 38) | |
| atgtctcaagttcaagtgcaagttcagaacccatctgctgctctctcagggagccaaatactgaacaagaaccagtc tcttctctcacagcctttgatgagtattccttctactactagctctctgccctctgaaaatgcaggtagacccattcaaaactctgctt taccctctgcatctattacatccaccagtgcagctgcagaaagcataacccctactgtagaactaaatgcactgtgcatgaaact tggaaaaaaaccaatgtataagcctgttgacccttactctcggatgcagtccacctataactacaacatgagaggaggtgcttat cccccgaggtacttttacccatttccagttccacctttactttatcaagtggaactttctgtgggaggacagcaatttaatggcaaa ggaaagacaagacaggctgcgaaacacgatgctgctgccaaagcgttgaggatcctgcagaatgagcccctgccagaga ggctggaggtgaatggaagagaatccgaagaagaaaatctcaataaatctgaaataagtcaagtgtttgagattgcacttaaa cggaacttgcctgtgaatttcgaggtggcccgggagagtggcccaccccacatgaagaactttgtgaccaaggtttcggttgg | |
| ggagtttgtgggggaaggtgaagggaaaagcaagaagatttcaaagaaaaatgccgccatagctgttcttgaggagctgaa gaagttaccgcccctgcctgcagttgaacgagtaaagcctagaatcaaaaagaaaacaaaacccatagtcaagccacagac aagcccagaatatggccaggggatcaatccgattagccgactggcccagatccagcaggcaaaaaaggagaaggagcca gagtacacgctcctcacagagcgaggcctcccgcgccgcagggagtttgtgatgcaggtgaaggttggaaaccacactgc agaaggaacgggcaccaacaagaaggtggccaagcgcaatgcagccgagaacatgctggagatccttggtttcaaagtcc cgcaggcgcagcccaccaaacccgcactcaagtcagaggagaagacacccataaagaaaccaggggatggaagaaaag taaccttttttgaacctggctctggggatgaaaatgggactagtaataaagaggatgagttcaggatgccttatctaagtcatca gcagctgcctgctggaattcttcccatggtgcccgaggtcgcccaggctgtaggagttagtcaaggacatcacaccaaagatt ttaccagggcagctccgaatcctgccaaggccacggtaactgccatgatagcccgagagttgttgtatgggggcacctcgcc cacagccgagaccattttaaagaataacatctcttcaggccacgtaccccatggacctctcacgagaccctctgagcaactgg actatctttccagagtccagggattccaggttgaatacaaagacttccccaaaaacaacaagaacgaatttgtatctcttatcaat tgctcctctcagccacctctgatcagccatggtatcggcaaggatgtggagtcctgccatgatatggctgcgctgaacatctta aagttgctgtctgagttggaccaacaaagtacagagatgccaagaacaggaaacggaccaatgtctgtgtgtgggaggtgct ga | |
| Gene symbol: | STK11 |
| Entrez Gene ID: | 6794 |
| dna/ac: | BC007981.2 |
| sequence: (SEQ ID NO: 39) | |
| atggaggtggtggacccgcagcagctgggcatgttcacggagggcgagctgatgtcggtgggtatggacacgtt catccaccgcatcgactccaccgaggtcatctaccagccgcgccgcaagcgggccaagctcatcggcaagtacctgatgg gggacctgctgggggaaggctcttacggcaaggtgaaggaggtgctggactcggagacgctgtgcaggagggccgtcaa gatcctcaagaagaagaagttgcgaaggatccccaacggggaggccaacgtgaagaaggaaattcaactactgaggaggt tacggcacaaaaatgtcatccagctggtggatgtgttatacaacgaagagaagcagaaaatgtatatggtgatggagtactgc gtgtgtggcatgcaggaaatgctggacagcgtgccggagaagcgtttcccagtgtgccaggcccacgggtacttctgtcagc tgattgacggcctggagtacctgcatagccagggcattgtgcacaaggacatcaagccggggaacctgctgctcaccaccg gtggcaccctcaaaatctccgacctgggcgtggccgaggcactgcacccgttcgcggcggacgacacctgccggaccag ccagggctccccggctttccagccgcccgagattgccaacggcctggacaccttctccggcttcaaggtggacatctggtcg gctggggtcaccctctacaacatcaccacgggtctgtaccccttcgaaggggacaacatctacaagttgtttgagaacatcgg gaaggggagctacgccatcccgggcgactgtggcccccgctctctgacctgctgaaaggatgcttgagtacgaaccgg ccaagaggttctccatccggcagatccggcagcacagctggttccggaagaaacatcctccggctgaagcaccagtgccca tcccaccgagcccagacaccaaggaccggtggcgcagcatgactgtggtgccgtacttggaggacctgcacggcgcgga cgaggacgaggacctcttcgacatcgaggatgacatcatctacactcaggacttcacggtgcccggacaggtcccagaaga ggaggccagtcacaatggacagcgccggggcctccccaaggccgtgtgtatgaacggcacagaggcggcgcagctgag caccaaatccagggcggagggccgggcccccaaccctgcccgcaaggcctgctccgccagcagcaagatccgccggct gtcggcctgcaagcagcagtga | |
| Gene symbol: | TOM1 |
| Entrez Gene ID: | 10043 |
| dna/ac: | BC046151.1 |
| sequence: (SEQ ID NO: 40) | |
| atggactttctcctggggaacccgttcagctctccagtgggacagcgcatcgagaaagccacagatggctccctg cagagcgaggactgggccctcaacatggagatctgcgacatcatcaacgagacggaggaaggtcccaaagatgccctccg agcagtaaagaagagaatcgtggggaataagaacttccacgaggtgatgctggctctcacagtcttagaaacctgtgtcaag aactgcgggcaccgcttccacgtgctggtggccagccaggacttcgtggagagtgtgctggtgaggaccatcctgcccaag aacaacccacccaccatcgtgcatgacaaagtgctcaacctcatccagtcctgggctgacgcgttccgcagctcgcccgatc tgacaggtgtggtcaccatctatgaggacctgcggaggaaaggcctggagttccccatgactgacctggacatgctgtcacc catccacacaccccagaggaccgtgttcaactcagagacacaatcaggacaggattctgtgggcactgactccagccagca agaggactctggccagcatgctgcccctctgcccgccccgcccatactctccggtgacacgcccatagcaccaaccccgga acagattggaagctgcgcagtgagctggagatggtgagtggaacgtgagggtgatgtcggagatgctgacggagctgg tgcccacccaggccgagcccgcagacctggagctgctgcaggagctcaaccgcacgtgccgagccatgcagcagcgggt cctggagctcatccctcagatcgccaatgagcagctgacagaggagctgctcatcgtcaatgacaatctcaacaatgtgttcct gcgccatgaacggtttgaacggttccgaacaggccagaccaccaaggccccaagtgaggccgagccggcagctgacctg atcgacatgggccctgacccagcagccaccggcaacctctcatcccagctggcaggaatgaacctgggctccagcagtgtg agagctggcctgcagtctctggaggcctctggtcgactggaagatgagtttgacatgtttgcgctgacacggggcagctcact ggctgaccaacggaaagaggtaaaatacgaagcccccaagcaacagacggcctggctggagccctggacgcccggca gcagagcactggcgcgatcccagtcacccaggcctgcctcatggaggacatcgagcagtggctgtccactgacgtgggta atgatgcggaagagcctaaggggtcaccagcgaaggtaaatttgacaaattcctggaagaacggccaaagccgcggac cgattgcccaacctctccagcccctcagctgaggggcccccgggtcccccatctggcccagcgccccggaagaagaccca ggagaaagatgatgacatgctgtttgccttatga | |
| Gene symbol: | TXNL2 |
| Entrez Gene ID: | 10539 |
| dna/ac: | BC005289 |
| sequence: (SEQ ID NO: 41) | |
| atggcggcgggggcggctgaggcagctgtagcggccgtggaggaggtcggctcagccgggcagtttgagga gctgctgcgcctcaaagccaagtccctccttgtggtccatttctgggcaccatgggctccacagtgtgcacagatgaacgaag ttatggcagagttagctaaagaactccctcaagtttcatttgtgaagttggaagctgaaggtgttcctgaagtatctgaaaaatat gaaattagctctgttcccacttttctgtttttcaagaattctcagaaaatcgaccgattagatggtgcacatgccccagagttgacc aaaaaagttcagcgacatgcatctagtggctccttcctacccagcgctaatgaacatcttaaagaagatctcaaccttcgcttga agaaattgactcatgctgccccctgcatgctgtttatgaaaggaactcctcaagaaccacgctgtggtttcagcaagcagatgg tggaaattcttcacaaacataatattcagtttagcagttttgatatcttctcagatgaagaggttcgacagggactcaaagcctatt ccagttggcctacctatcctcagctctatgtttctggagagctcataggaggacttgatataattaaggagctagaagcatctga agaactagatacaatttgtcccaaagctcccaaattagaggaaaggctcaaagtgctgacaaataaagcttctgtgatgctcttt atgaaaggaaacaaacaggaagcaaaatgtggattcagcaaacaaattctggaaatactaaatagtactggtgttgaatatga aacattcgatatattggaggatgaagaagttcggcaaggattaaaagcttactcaaattggccaacataccctcagctgtatgtg aaaggggagctggtgggaggattggatattgtgaaggaactgaaagaaaatggtgaattgctgcctatactgagaggagaa aattaa | |
| Gene symbol: | TXNRD 1 |
| Entrez Gene ID: | 7296 |
| dna/ac: | BC018122.1 |
| sequence: (SEQ ID NO: 42) | |
| atgaacggccctgaagatcttcccaagtcctatgactatgaccttatcatcattggaggtggctcaggaggtctggc agctgctaaggaggcagcccaatatggcaagaaggtgatggtcctggactttgtcactcccacccctcttggaactagatggg gtcttggaggaacatgtgtgaatgtgggttgcatacctaaaaaactgatgcatcaagcagctttgttaggacaagccctgcaag actctcgaaattatggatggaaagtcgaggagacagttaagcatgattgggacagaatgatagaagctgtacagaatcacatt ggctctttgaattggggctaccgagtagctctgcgggagaaaaaagtcgtctatgagaatgcttatgggcaatttattggtcctc acaggattaaggcaacaaataataaaggcaaagaaaaaatttattcagcagagagatttctcattgccactggtgaaagacca cgttacttgggcatccctggtgacaaagaatactgcatcagcagtgatgatcttttctccttgccttactgcccgggtaagaccct ggttgttggagcatcctatgtcgctttggagtgcgctggatttcttgctggtattggtttagacgtcactgttatggttaggtccattc ttcttagaggatttgaccaggacatggccaacaaaattggtgaacacatggaagaacatggcatcaagtttataagacagttcg taccaattaaagttgaacaaattgaagcagggacaccaggccgactcagagtagtagctcagtccaccaatagtgaggaaat cattgaaggagaatataatacggtgatgctggcaataggaagagatgcttgcacaagaaaaattggcttagaaaccgtaggg gtgaagataaatgaaaagactggaaaaatacctgtcacagatgaagaacagaccaatgtgccttacatctatgccattggcga tatattggaggataaggtggagctcaccccagttgcaatccaggcaggaagattgctggctcagaggctctatgcaggttcca ctgtcaagtgtgactatgaaaatgttccaaccactgtatttactcctttggaatatggtgcttgtggcctttctgaggagaaagctg tggagaagtttggggaagaaaatattgaggtttaccatagttacttttggccattggaatggacgattccgtcaagagataacaa caaatgttatgcaaaaataatctgtaatactaaagacaatgaacgtgttgtgggctttcacgtactgggtccaaatgctggagaa gttacacaaggctttgcagctgcgctcaaatgtggactgaccaaaaagcagctggacagcacaattggaatccaccctgtctg tgcagaggtattcacaacattgtctgtgaccaagcgctctggggcaagcatcctccaggctggctgctgaggttaa | |
| Gene symbol: | VCL |
| Entrez Gene ID: | 7414 |
| dna/ac: | BC039174.1 |
| sequence: (SEQ ID NO: 43) | |
| atgccagtgtttcatacgcgcacgatcgagagcatcctggagccggtggcacagcagatctcccacctggtgata atgcacgaggagggcgaggtggacggcaaagccattcctgacctcaccgcgcccgtggccgccgtgcaggcggccgtca gcaacctcgtccgggttggaaaagagactgttcaaaccactgaggatcagattttgaagagagatatgccaccagcatttatta aggttgagaatgcttgcaccaagcttgtccaggcagctcagatgcttcagtcagacccttactcagtgcctgctcgagattatct aattgatgggtcaaggggcatcctctctggaacatcagacctgctccttaccttcgatgaggctgaggtccgtaaaattattaga gtttgcaaaggaattttggaatatcttacagtggcagaggtggtggagactatggaagatttggtcacttacacaaagaatcttg ggccaggaatgactaagatggccaagatgattgacgagagacagcaggagctcactcaccaggagcaccgagtgatgttg gtgaactcgatgaacaccgtgaaagagttgctgccagttctcatttcagctatgaagatttttgtaacaactaaaaactcaaaaaa ccaaggcatagaggaagctttaaaaaatcgcaattttactctagaaaaaatgagtgctgaaattaatgagataattcgtgtgttac aactcacctcttgggatgaagatgcctgggccagcaaggacactgaagccatgaagagagcattggcctccatagactcca aactgaaccaggccaaaggttggctccgtgaccctagtgcctccccaggggatgctggtgagcaggccatcagacagatct tagatgaagctggaaaagttggtgaactctgtgcaggcaaagaacgcagggagattctgggaacttgcaaaatgctagggc agatgactgatcaagtggctgacctccgtgccagaggacaaggatcctcaccggtggccatgcagaaagctcagcaggtat ctcagggtctggatgtgctcacagcaaaagtggaaaatgcagctcgcaagctggaagccatgaccaactcaaagcagagca ttgcaaagaagatcgatgctgctcagaactggcttgcagatccaaatggtggaccggaaggagaagagcagattcgaggtg ctttggctgaagctcggaaaatagcagaattatgtgatgatcctaaagaaagagatgacattctacgttcccttggggaaatatc tgctctgacttctaaattagcagatctacgaagacaggggaaaggagattctccagaggctcgagccttggccaaacaggtg gccacggccctgcagaacctgcagaccaaaaccaaccgggctgtggccaacagcagaccggccaaagcagctgtacacc | |
| ttgagggcaagattgagcaagcacagcggtggattgataatcccacagtggatgaccgtggagtcggtcaggctgccatcc gggggcttgtggccgaagggcatcgtctggctaatgttatgatggggccttatcggcaagatcttctcgccaagtgtgaccga gtggaccagctgacagcccagctggctgacctggctgccagagggaaggggagagtcctcaggcacgagcacttgcat ctcagctccaagactccttaaaggatctaaaagctcggatgcaggaggccatgactcaggaagtgtcagatgttttcagcgat accacaactcccatcaagctgttggcagtggcagccacggcgcctcctgatgcgcctaacaggaagaggtatttgatgaga gggcagctaactttgaaaaccattcaggaaagcttggtgctacggccgagaaggcggctgcggttggtactgctaataaatc aacagtggaaggcattcaggcctcagtgaagacggcccgagaactcacaccccaggtggtctcggctgctcgtatcttactt aggaaccctggaaatcaagctgcttatgaacattttgagaccatgaagaaccagtggatcgataatgttgaaaaaatgacagg gctggtggacgaagccattgataccaaatctctgttggatgcttcagaagaagcaattaaaaagacctggacaagtgcaag gtagctatggccaacattcagcctcagatgctggttgctggggcaaccagtattgctcgtcggccaaccggatcctgctggt ggctaagagggaggtggagaattccgaggatcccaagttccgtgaggctgtgaaagctgcctctgatgaattgagcaaaac catctccccgatggtgatggatgcaaaagctgtggctggaaacatttccgaccctggactgcaaaagagcttcctggactcag gatatcggatcctgggagctgtggccaaggtcagagaagccttccaacctcaggagcctgacttcccgccgcctccaccag accttgaacaactccgactaacagatgagcttgctcctcccaaaccacctctgcctgaaggtgaggtccctccacctaggcct ccaccaccagaggaaaaggatgaagagttccctgagcagaaggccggggaggtgattaaccagccaatgatgatggctgc cagacagctccatgatgaagctcgcaaatggtccagcaagggcaatgacatcattgcagcagccaagcgcatggctctgct gatggctgagatgtctcggctggtaagagggggcagtggtaccaagcgggcactcattcagtgtgccaaggacatcgccaa ggcctcagatgaggtgactcggttggccaaggaggttgccaagcagtgcacagataaacggattagaaccaacctcttaca ggtatgtgagcgaatcccaaccataagcacccagctcaaaatcctgtccacagtgaaggccaccatgctgggccggaccaa catcagtgatgaggagtctgagcaggccacagagatgctggttcacaatgcccagaacctcatgcagtctgtgaaggagact gtgcgggaagctgaagctgcttcaatcaaaattcgaacagatgctggatttacactgcgctgggttagaaagactccctggta ccagtag | |
| Gene symbol: | ZMAT2 |
| Entrez Gene ID: | 153527 |
| dna/ac: | BC056668.1 |
| sequence: (SEQ ID NO: 44) | |
| atggcgtcgggcagcgggacaaaaaacttggactttcgccgaaagtgggacaaagatgaatatgagaaactcgc cgagaagaggctcacggaagagagagaaaagaaagatggaaaaccagtgcagcctgtcaagcgagagcttttacggcat agggactacaaggtggacttggaatccaagcttgggaagacaattgtcattaccaagacaacccctcaatctgagatgggag gatattactgcaatgtctgtgactgtgtggtgaaggactccatcaactttctggatcacattaatggaaagaaacatcagagaaa cctgggcatgtctatgcgtgtggaacgttccaccctggatcaggtgaagaaacgttttgaggtcaacaagaagaagatggaa gagaagcagaaggattatgattttgaggaaaggatgaaggagctcagagaagaggaggaaaaggccaaagcgtacaaga aagagaaacagaaggagaagaaaaggagggctgaggaggacttgacatttgaggaggacgatgagatggcagctgtgat gggcttctctggctttggttccaccaagaagagttactga | |
| Gene symbol: | ZNF38 |
| Entrez Gene ID: | 7589 |
| dna/ac: | BC047309.1 |
| sequence: (SEQ ID NO: 45) | |
| atgatgaccaaggtactaggcatggccccagttctgggccctaggcctccacaggagcaggtggggcctctgat ggtaaaagtcgaggagaaagaagagaaaggcaagtaccttcctagcctggagatgttccgccagcgcttcaggcagtttgg gtaccatgataccccctggaccccgagaggccctgagccaactccgggtgctctgctgtgagtggctgaggcccgagatcca caccaaggagcagatcctggagctactggtgctggagcagttcctgaccatcctgccccaggagctccaggcctgggtgca ggagcattgcccggagagcgctgaagaggctgtcactctcctcgaagatctggagcgggaactggatgagccaggacacc aggtctcaactcctccaaacgaacagaaaccggtgtgggagaagatatcctcctcaggaactgcaaaggaatccccgagca gcatgcagccacagcccttggagaccagtcacaaatacgagtcttgggggcccctgtacatccaagagtctggtgaggagc aggagttcgctcaagatccaagaaaggtccgagattgcagattgagtacccagcacgaggaatcagcagatgagcagaaa ggttctgaagcagaggggctcaaagggatataatttctgtgattatcgccaataaacctgaggccagcttagagaggcagtg cgtaaaccttgaaaatgaaaaaggaacaaaacccccctcttcaagaggcaggctccaagaaaggtagagaatcagttcctact aaacctaccccaggagagagacgttatatatgtgctgaatgtggcaaagcctttagtaatagctcaaatctcaccaaacacag gagaacacacactggggagaaaccttacgtgtgcaccaagtgtgggaaagctttcagccacagctcaaacctcaccctcca ctacagaacacacttggtggaccggccctatgactgtaagtgtggaaaagcttttgggcagagctcagaccttcttaaacatca gagaatgcacacagaagaggcgccatatcagtgcaaagattgtggcaaggctttcagcgggaaaggcagcctcattcgtca ctatcggatccacactggggagaagccttatcagtgtaacgaatgtgggaagagcttcagtcagcatgcgggcctcagctcc caccagagactccacaccggagagaagccatataagtgtaaggagtgtgggaaagccttcaaccacagctccaacttcaat aaacaccacagaatccacaccggggaaaagccctactggtgtcatcactgtggaaagaccttctgtagcaagtccaatctttc caaacatcagcgagtccacactggagagggagaagcaccgtaa | |

## Claims

1. A method useful in diagnosing a patient as having or being predisposed to developing lupus, comprising testing a sample from the patient against a panel of auto-antigens to detect the presence, absence or a modulated level of auto-antibodies in the sample, wherein the panel of auto-antigens comprises:
(a) the other 31 auto-antigens listed in Table 2,
(b) the auto-antigens listed in Table 1,
(c) the auto-antigens listed in Table 4,
(d) the auto-antigens listed in Table 3,
(e) the panel of (a) or (b) or (c) or (d) in which up to 5 auto-antigens are deleted or replaced, or
(f) the panel of (a) in which up to 6, 7, 8, 9 or 10 or more auto-antigens are deleted or replaced, or
(g) the panel of (d) in which up to 10, 15, 20 or 25 or more auto-antigens are deleted or replaced, or
(h) at least 10 auto-antigens selected from Table 3

2. A method of diagnosing a patient as having or being predisposed to developing lupus, comprising detecting in a sample taken from said patient one or more auto-antibodies that bind an auto-antigen selected from the group consisting of the auto-antigens listed in Table 3, wherein the presence of said one or more auto-antibodies, or an increase in the concentration of said one or more auto-antibodies, indicates that the patient suffers from or is predisposed to develop lupus.

3. A method according to claim 1(h), wherein: (i) the at least 10 auto-antigens are selected from Table 1, or (ii) the at least 10 auto-antigens are selected from Table 2.

4. A method according to claim 1(h) or to claim 3, wherein the panel comprises at least 20 auto-antigens and/or wherein the panel comprises ZMAT2.

5. A method according to claim 1(c), wherein the panel comprises one or more additional auto-antigens selected from the group consisting of the auto-antigens of Table 5.

6. The method of any one of the preceding claims wherein detecting the presence or an increased level of at least two auto-antibodies provides information useful for diagnosis; for example, wherein detecting the presence of an increased level of at least three, preferably at least four and more preferably at least five auto-antibodies provides information useful for diagnosis.

7. A method of:
(A) monitoring the progression or regression of lupus, or monitoring the progression to a flare of the disease or transition from a flare into remission, comprising:
(a) performing the method of any one of claims 1 to 7,
(b) repeating step (a) using a second sample obtained from the patient at a time later than the first sample;
(c) comparing the auto-antibodies detected in said first and second samples; wherein a change in the level or presence or absence of the auto-antibodies detected between the first and second samples indicates the progression or regression of lupus, or the progression to a flare, or transition from a flare into remission;
(B) monitoring the efficacy in a patient of a therapeutic agent to lupus, comprising:
(a) performing the method of any one of claims 1 to 15,
(b) administering the therapeutic agent to the patient;
(c) performing the method of any one of claims 1 to 15 using a second sample from the patient taken after the administration of the therapeutic agent;
(d) comparing the auto-antibodies detected in said first and second samples; wherein a change in the level or presence or absence of the auto-antibodies detected in the first and second sample is indicative of the efficacy of the therapeutic agent to lupus.
(C) diagnosing a patient as having or being predisposed to developing lupus, comprising detecting in a sample taken from said patient one or more auto-antibodies that bind an auto-antigen selected from:
a) the group consisting of ZMAT2 (Zinc finger, matrin type 2); BPY21P1 (MAPIS protein); CEBPG (CCAAT/enhancer binding protein (C/EBP), gamma); E1B-AP5 (E1B-55kDa-associated protein 5); FUS (Fusion (involved in t(12;16) in malignant liposarcoma)); HAGH (Hydroxyacylglutathione hydrolase); HMG20B (High-mobility group 20B); HOXB6 (Homeo box B6, transcript variant 2); KRT8 (Keratin 8); LIN28 (Lin-28 homolog (C. elegans)); NDUFV3 (NADH dehydrogenase (ubiquinone) flavoprotein 3, 10kDa); PABPC1 (Poly(A) binding protein, cytoplasmic 1); PHLDA1 (Pleckstrin homology-like domain, family A, member 1); PIAS2 (Msx-interacting-zinc finger, transcript variant alpha); RAB11FIP3 (RAB11 family interacting protein 3 (class II)); RAN (RAN, member RAS oncogene family); RARA (Retinoic acid receptor alpha); RBMS1 (RNA binding motif, single stranded interacting protein 1, transcript variant); RDBP (RD RNA binding protein); RNF12 (Ring finger protein 12, transcript variant 1); RUFY1 (RUN and FYVE domain containing 1); SMN1 (Survival of motor neuron 1, telomeric); SRPK1 (SFRS protein kinase 1); SSNA1 (Sjogren's syndrome nuclear autoantigen 1); STAU (Staufen, RNA binding protein (Drosophila), transcript variant T3); STK11 (Serine/threonine kinase 11 (Peutz-Jeghers syndrome)); TOM1 (Target of myb1 (chicken)); TXNL2 (Thioredoxin-like, clone MGC:12349); TXNRD1 (Thioredoxin reductase 1, transcript variant 5); and ZNF38 (Zinc finger protein 38), or
b) the group consisting of ZMAT2 (Zinc finger, matrin type 2); ASPSCR1 (alveolar soft part sarcoma chromosome region, candidate 1); CEBPG (CCAAT/enhancer binding protein (C/EBP), gamma); DDX55 (DEAD (Asp-Glu-Ala-Asp) box polypeptide 55); DOM3Z (dom-3 homolog Z (C. elegans)); E1B-AP5 (E1B-55kDa-associated protein 5); FUS (Fusion (involved in t(12;16) in malignant liposarcoma)); HMG20B (High-mobility group 20B); KRT8 (Keratin 8); LIN28 (Lin-28 homolog (C. elegans)); LNX (ligand of numb-protein X); MAP1S (MAP1S protein); PABPC1 (Poly(A) binding protein, cytoplasmic 1); PHLDA1 (Pleckstrin homology-like domain, family A, member 1); PIAS2 (Msx-interacting-zinc finger, transcript variant alpha); PRKCBP 1 (protein kinase C binding protein 1); PRKRA (protein kinase, interferon-inducible double stranded RNA dependent activator); RARA (Retinoic acid receptor, alpha); RDBP (RD RNA binding protein); RPL30 (ribosomal protein L30); RPL31 (ribosomal protein L31); SNK (serum-inducible kinase); SRPK1 (SFRS protein kinase 1); STAU1 (Staufen, RNA binding protein (Drosophila), transcript variant T3); TXNL2 (Thioredoxin-like, clone MGC:12349); VCL (vinculin); and ZNF38 (Zinc finger protein 38),
wherein the presence of said one or more auto-antibodies, or an increase in the concentration of said one or more auto-antibodies, indicates that the patient suffers from or is predisposed to develop lupus.
*and optionally* wherein the method further comprises detecting in a sample taken from said patient one or more auto-antibodies that bind an auto-antigen selected from the group consisting of: BANK1 (B-cell scaffold protein with ankyrin repeats 1); IFI16 (Interferon, gamma-inducible protein 16); PSME3 (Proteasome (prosome, macropain) activator subunit 3 (PA28 gamma; Ki), transc); RALBP1 (Ra1A-binding protein 1); and SSA2 (Sjogren syndrome antigen A2 (60kDa, ribonucleoprotein autoantigen SS-A/Ro));
(D) diagnosing a patient as having or being predisposed to developing lupus, comprising detecting in a sample from said patient auto-antibodies to a panel comprising 5 or more auto-antigens selected from:
a) the group consisting of ZMAT2 (Zinc finger, matrin type 2); BPY2IP1 (MAP1S protein); CEBPG (CCAAT/enhancer binding protein (C/EBP), gamma); E1B-AP5 (E1B-SSkDa-associated protein 5); FUS (Fusion (involved in t(12;16) in malignant liposarcoma)); HAGH (Hydroxyacylglutathione hydrolase); HMG20B (High-mobility group 20B); HOXB6 (Homeo box B6, transcript variant 2); KRT8 (Keratin 8); LIN28 (Lin-28 homolog (C. elegans)); NDUFV3 (NADH dehydrogenase (ubiquinone) flavoprotein 3, 10kDa); PABPC1 (Poly(A) binding protein, cytoplasmic 1); PHLDA1 (Pleckstrin homology-like domain, family A, member 1); PIAS2 (Msx-interacting-zinc finger, transcript variant alpha); RAB11FIP3 (RAB11 family interacting protein 3 (class II)); RAN (RAN, member RAS oncogene family); RARA (Retinoic acid receptor alpha); RBMS1 (RNA binding motif, single stranded interacting protein 1, transcript variant); RDBP (RD RNA binding protein); RNF12 (Ring finger protein 12, transcript variant 1); RUFY1 (RUN and FYVE domain containing 1); SMN1 (Survival of motor neuron 1, telomeric); SRPK1 (SFRS protein kinase 1); SSNA1 (Sjogren's syndrome nuclear autoantigen 1); STAU (Staufen, RNA binding protein (Drosophila), transcript variant T3); STK11 (Serine/threonine kinase 11 (Peutz-Jeghers syndrome)); TOM1 (Target of myb1 (chicken)); TXNL2 (Thioredoxin-like, clone MGC:12349); TXNRD1 (Thioredoxin reductase 1, transcript variant 5); and ZNF38 (Zinc finger protein 38), or
b) the group consisting of ZMAT2 (Zinc finger, matrin type 2); ASPSCR1 (alveolar soft part sarcoma chromosome region, candidate 1); CEBPG (CCAAT/enhancer binding protein (C/EBP), gamma); DDX55 (DEAD (Asp-Glu-Ala-Asp) box polypeptide 55); DOM3Z (dom-3 homolog Z (C. elegans)); E1B-AP5 (E1B-55kDa-associated protein 5); FUS (Fusion (involved in t(12;16) in malignant liposarcoma)); HMG20B (High-mobility group 20B); KRT8 (Keratin 8); LIN28 (Lin-28 homolog (C. elegans)); LNX (ligand of numb-protein X); MAP1S (MAP1S protein); PABPC1 (Poly(A) binding protein, cytoplasmic 1); PHLDA1 (Pleckstrin homology-like domain, family A, member 1); PIAS2 (Msx-interacting-zinc finger, transcript variant alpha); PRKCBP1 (protein kinase C binding protein 1); PRKRA (protein kinase, interferon-inducible double stranded RNA dependent activator); RARA (Retinoic acid receptor, alpha); RDBP (RD RNA binding protein); RPL30 (ribosomal protein L30); RPL31 (ribosomal protein L31); SNK (serum-inducible kinase); SRPK1 (SFRS protein kinase 1); STAU1 (Staufen, RNA binding protein (Drosophila), transcript variant T3); TXNL2 (Thioredoxin-like, clone MGC:12349); VCL (vinculin); and ZNF38 (Zinc finger protein 38),
wherein the presence or separately a significant increase in the concentration of said auto-antibodies in the sample to substantially all members of the panel indicates that the patient suffers from or is predisposed to develop lupus.
(E) monitoring the progression or regression of lupus, or monitoring the progression to a flare of the disease or transition from a flare into remission, comprising
(a) detecting in a first sample from a patient the presence of one or more auto-antibodies that bind an auto-antigen selected from:
(i) the group consisting of ZMAT2 (Zinc finger, matrin type 2); BPY2IP1 (MAP1S protein); CEBPG (CCAAT/enhancer binding protein (C/EBP), gamma); EIB-AP5 (E1B-55kDaassociated protein 5); FUS (Fusion (involved in t(12;16) in malignant liposarcoma)); HAGH (Hydroxyacylglutathione hydrolase); HMG20B (High-mobility group 20B); HOXB6 (Homeo box B6, transcript variant 2); KRT8 (Keratin 8); LIN28 (Lin-28 homolog (C. elegans)); NDUFV3 (NADH dehydrogenase (ubiquinone) flavoprotein 3, 10kDa); PABPC1 (Poly(A) binding protein, cytoplasmic 1); PHLDA1 (Pleckstrin homology-like domain, family A, member 1); PIAS2 (Msx-interacting-zinc finger, transcript variant alpha); RAB11FIP3 (RAB11 family interacting protein 3 (class II)); RAN (RAN, member RAS oncogene family); RARA (Retinoic acid receptor alpha); RBMS1 (RNA binding motif, single stranded interacting protein 1, transcript variant); RDBP (RD RNA binding protein); RNF12 (Ring finger protein 12, transcript variant 1); RUFY1 (RUN and FYVE domain containing 1); SMN1 (Survival of motor neuron 1, telomeric); SRPK1 (SFRS protein kinase 1); SSNA1 (Sjogren's syndrome nuclear autoantigen 1); STAU (Staufen, RNA binding protein (Drosophila), transcript variant T3); STK11 (Serine/threonine kinase 11 (Peutz-Jeghers syndrome)); TOM1 (Target of myb1 (chicken)); TXNL2 (Thioredoxin-like, clone MGC:12349); TXNRD1 (Thioredoxin reductase 1, transcript variant 5); and ZNF38 (Zinc finger protein 38), or
(ii) the group of auto-antigens consisting of ZMAT2 (Zinc finger, matrin type 2); ASPSCR1 (alveolar soft part sarcoma chromosome region, candidate 1); CEBPG (CCAAT/enhancer binding protein (C/EBP), gamma); DDX55 (DEAD (Asp-Glu-Ala-Asp) box polypeptide 55); DOM3Z (dom-3 homolog Z (C. elegans)); E1B-AP5 (E1B-55kDa-associated protein 5); FUS (Fusion (involved in t(12;16) in malignant liposarcoma)); HMG20B (High-mobility group 20B); KRT8 (Keratin 8); LIN28 (Lin-28 homolog (C. elegans)); LNX (ligand of numb-protein X); MAP1S (MAP1S protein); PABPC1 (Poly(A) binding protein, cytoplasmic 1); PHLDA1 (Pleckstrin homology-like domain, family A, member 1); PIAS2 (Msx-interacting-zinc finger, transcript variant alpha); PRKCBP 1 (protein kinase C binding protein 1); PRKRA (protein kinase, interferon-inducible double stranded RNA dependent activator); RARA (Retinoic acid receptor, alpha); RDBP (RD RNA binding protein); RPL30 (ribosomal protein L30); RPL31 1 (ribosomal protein L31); SNK (serum-inducible kinase); SRPK1 (SFRS protein kinase 1); STAU1 (Staufen, RNA binding protein (Drosophila), transcript variant T3); TXNL2 (Thioredoxin-like, clone MGC:12349); VCL (vinculin); and ZNF38 (Zinc finger protein 38),
(b) detecting said auto-antibodies in a second sample obtained from the patient at a time later than the first sample;
(c) comparing the auto-antibodies in said first and second samples;
wherein a change in the level or presence or absence of the auto-antibodies in the first and second sample indicates the progression or regression of lupus, or the progression to a flare, or transition from a flare into remission.
(F) monitoring the efficacy of a therapeutic agent to lupus, comprising:
(a) detecting in a first sample from a patient the presence of one or more auto-antibodies that bind an auto-antigen selected from:
(i) the group consisting of ZMAT2 (Zinc finger, matrin type 2); BPY2IP1 (MAPIS protein); CEBPG (CCAAT/enhancer binding protein (C/EBP), gamma); E1B-AP5 (EIB-55kDaassociated protein 5); FUS (Fusion (involved in t(12;16) in malignant liposarcoma)); HAGH (Hydroxyacylglutathione hydrolase); HMG20B (High-mobility group 20B); HOXB6 (Homeo box B6, transcript variant 2); KRT8 (Keratin 8); LIN28 (Lin-28 homolog (C. elegans)); NDUFV3 (NADH dehydrogenase (ubiquinone) flavoprotein 3, 10kDa); PABPC1 (Poly(A) binding protein, cytoplasmic 1); PHLDA1 (Pleckstrin homology-like domain, family A, member 1); PIAS2 (Msx-interacting-zinc finger, transcript variant alpha); RAB11FIP3 (RAB11 family interacting protein 3 (class II)); RAN (RAN, member RAS oncogene family); RARA (Retinoic acid receptor alpha); RBMS1 (RNA binding motif, single stranded interacting protein 1, transcript variant); RDBP (RD RNA binding protein); RNF12 (Ring finger protein 12, transcript variant 1); RUFY1 (RUN and FYVE domain containing 1); SMN1 (Survival of motor neuron 1, telomeric); SRPK1 (SFRS protein kinase 1); SSNA1 (Sjogren's syndrome nuclear autoantigen 1); STAU (Staufen, RNA binding protein (Drosophila), transcript variant T3); STK11 (Serine/threonine kinase 11 (Peutz-Jeghers syndrome)); TOM1 (Target of myb1 (chicken)); TXNL2 (Thioredoxin-like, clone MGC:12349); TXNRD1 (Thioredoxin reductase 1, transcript variant 5); and ZNF38 (Zinc finger protein 38), or
(ii) the group consisting of the auto-antigens of ZMAT2 (Zinc finger, matrin type 2); ASPSCR1 (alveolar soft part sarcoma chromosome region, candidate 1); CEBPG (CCAAT/enhancer binding protein (C/EBP), gamma); DDX55 (DEAD (Asp-Glu-Ala-Asp) box polypeptide 55); DOM3Z (dom-3 homolog Z (C. elegans)); E1B-AP5 (E1B-55kDa-associated protein 5); FUS (Fusion (involved in t(12;16) in malignant liposarcoma)); HMG20B (High-mobility group 20B); KRT8 (Keratin 8), LIN28 (Lin-28 homolog C. elegans)); LNX (ligand of numb-protein X); MAPIS (MAPIS protein); PABPC1 (Poly(A) binding protein, cytoplasmic 1); PHLDA1 (Pleckstrin homology-like domain, family A, member 1); PIAS2 (Msx-interacting-zinc finger, transcript variant alpha); PRKCBP1 (protein kinase C binding protein 1); PRKRA (protein kinase, interferon-inducible double stranded RNA dependent activator); RARA (Retinoic acid receptor, alpha); RDBP (RD RNA binding protein); RPL30 (ribosomal protein L30); RPL31 (ribosomal protein L31); SNK (serum-inducible kinase); SRPK1 (SFRS protein kinase 1); STAU1 (Staufen, RNA binding protein (Drosophila), transcript variant T3); TXNL2 (Thioredoxin-like, clone MGC:12349); VCL (vinculin); and ZNF38 (Zinc finger protein 38),
(b) administering the therapeutic agent to the patient;
(c) detecting said one or more auto-antibodies in a second sample from the patient taken after the administration of the therapeutic agent;
(d) comparing the one or more auto-antibodies in said first and second samples;
wherein a change in the level or presence or absence of the auto-antibodies in the first and second sample is indicative of the efficacy of the therapeutic agent to lupus.

8. The method of any of (A), (B), (E) or (F) in claim 7, wherein the change is:
an increase in the number of auto-antigens to which auto-antibodies are detected in the second sample as compared to the first sample, and said change indicates that lupus has become more severe;
a decrease in the number of auto-antigens to which auto-antibodies are detected in the second sample as compared to the first sample, and said change indicates that lupus has become less severe;
a change in the concentration of one or more of the auto-antibodies in the second sample as compared to the first sample;
an increase in the concentration of one or more of the auto-antibodies in the second sample as compared to the first sample, and wherein the increase indicates that lupus has become more severe;
a decrease in the concentration of one or more of the auto-antibodies in the second sample as compared to the first sample, and wherein the decrease indicates that lupus has become less severe;
an increase in the concentration of one or more of the auto-antibodies in the second sample as compared to the first sample, wherein the increase indicates that lupus has become less severe;
a decrease in the concentration of one or more of the auto-antibodies in the second sample as compared to the first sample, wherein the decrease indicates that lupus has become more severe; a change in the one or more auto-antibodies which is a decrease in the number of auto-antigens to which auto-antibodies are detected, or the concentration of auto-antibodies, in the second sample as compared to the first sample, and wherein the decrease indicates that the therapeutic agent is efficacious;
a change in the one or more auto-antibodies which is a increase in the number of auto-antigens to which auto-antibodies are detected in the second sample as compared to the first sample, and wherein the increase indicates that the therapeutic agent is efficacious;
a decrease in the concentration of the auto-antibody;
an increase in the concentration of the auto-antibody;
for example:
o wherein the decrease in concentration is by at least 5%, 10%, 15% or 20
o wherein the increase in concentration is by at least 5%, 10%, 15% or 20%.
and/or wherein the absence of said auto-antibodies in the sample indicates that the patient does not have or is not predisposed to developing lupus thereby providing a negative diagnosis.

9. The method of any one of claims 1 to 8, wherein the panel of auto-antigens may further comprise one or more of: BANK1 (B-cell scaffold protein with ankyrin repeats 1); IFI16 (Interferon, gamma-inducible protein 16); PSME3 (Proteasome (prosome, macropain) activator subunit 3 (PA28 gamma; Ki), transc); RALBP1 (RaIA-binding protein 1); and SSA2 (Sjogren syndrome antigen A2 (60kDa, ribonucleoprotein autoantigen SS-A/Ro)).

10. The method of claim 7, wherein: the presence of auto-antibodies to at least two of the auto-antigens are detected; wherein the panel comprises 10 or more of said auto-antigens; or wherein a panel comprising 3 or more of said auto-antigens is used for the detection of auto-antibodies.

11. The method of any previous claim, wherein: (i) the patient is pre-symptomatic; and/or (ii) the sample is a body fluid; for example, a body fluid selected from blood, serum, plasma, saliva, lymphatic fluid, wound secretion, urine, faeces, mucus or cerebrospinal fluid (CSF); and, in particular example, wherein the sample is serum or plasma; and/or (iii) the method specifies a panel which comprises ZMAT2.

12. A diagnostic kit for use in diagnosing a patient as having or being predisposed to developing lupus, said kit comprising:
(A) one or more auto-antigens selected from:
(a) the group consisting of ZMAT2 (Zinc finger, matrin type 2); BPY2IP1 (MAP 1 S protein); CEBPG (CCAAT/enhancer binding protein (C/EBP), gamma); E1B-AP5 (E1B-55kDa-associated protein 5); FUS (Fusion (involved in t(12;16) in malignant liposarcoma)); HAGH (Hydroxyacylglutathione hydrolase); HMG20B (High-mobility group 20B); HOXB6 (Homeo box B6, transcript variant 2); KRT8 (Keratin 8); LIN28 (Lin-28 homolog (C. elegans)); NDUFV3 (NADH dehydrogenase (ubiquinone) flavoprotein 3, 10kDa); PABPC1 (Poly(A) binding protein, cytoplasmic 1); PHLDA1 (Pleckstrin homology-like domain, family A, member 1); PIAS2 (Msx-interacting-zinc finger, transcript variant alpha); RAB11FIP3 (RAB11 family interacting protein 3 (class II)); RAN (RAN, member RAS oncogene family); RARA (Retinoic acid receptor alpha); RBMS1 (RNA binding motif, single stranded interacting protein 1, transcript variant); RDBP (RD RNA binding protein); RNF12 (Ring finger protein 12, transcript variant 1); RUFY1 (RUN and FYVE domain containing 1); SMN1 (Survival of motor neuron 1, telomeric); SRPK1 (SFRS protein kinase 1); SSNA1 (Sjogren's syndrome nuclear autoantigen 1); STAU (Staufen, RNA binding protein (Drosophila), transcript variant T3); STK11 (Serine/threonine kinase 11 (Peutz-Jeghers syndrome)); TOM1 (Target of myb1 (chicken)); TXNL2 (Thioredoxin-like, clone MGC:12349); TXNRD1 (Thioredoxin reductase 1, transcript variant 5); and ZNF38 (Zinc finger protein 38), or
(b) the group consisting of auto-antigens of ZMAT2 (Zinc finger, matrin type 2); ASPSCR1 (alveolar soft part sarcoma chromosome region, candidate 1); CEBPG (CCAAT/enhancer binding protein (C/EBP), gamma); DDX55 (DEAD (Asp-Glu-Ala-Asp) box polypeptide 55); DOM3Z (dom-3 homolog Z (C. elegans)); E1B-AP5 (E1B-55kDa-associated protein 5); FUS (Fusion (involved in t(12;16) in malignant liposarcoma)); HMG20B (High-mobility group 20B); KRT8 (Keratin 8); LIN28 (Lin-28 homolog (C. elegans)); LNX (ligand of numb-protein X); MAP1S (MAP1S protein); PABPC1 (Poly(A) binding protein, cytoplasmic 1); PHLDA1 (Pleckstrin homology-like domain, family A, member 1); PIAS2 (Msx-interacting-zinc finger, transcript variant alpha); PRKCBP1 (protein kinase C binding protein 1); PRKRA (protein kinase, interferon-inducible double stranded RNA dependent activator); RARA (Retinoic acid receptor, alpha); RDBP (RD RNA binding protein); RPL30 (ribosomal protein L30); RPL31 (ribosomal protein L31); SNK (serum-inducible kinase); SRPK1 (SFRS protein kinase 1); STAU1 (Staufen, RNA binding protein (Drosophila), transcript variant T3); TXNL2 (Thioredoxin-like, clone MGC:12349); VCL (vinculin); and ZNF38 (Zinc finger protein 38), or
(c) the group consisting of the auto-antigens of Table 3;
which one or more auto-antigens are immobilised on a substrate. or
(B) a panel comprising 5 or more auto-antigens selected from:
(a) the group consisting of ZMAT2 (Zinc finger, matrin type 2); BPY2IP1 (MAP1S protein); CEBPG (CCAAT/enhancer binding protein (C/EBP), gamma); E1B-AP5 (E1B-SSkDa-associated protein 5); FUS (Fusion (involved in t(12;16) in malignant liposarcoma)); HAGH (Hydroxyacylglutathione hydrolase); HMG20B (High-mobility group 20B); HOXB6 (Homeo box B6, transcript variant 2); KRT8 (Keratin 8); LIN28 (Lin-28 homolog (C. elegans)); NDUFV3 (NADH dehydrogenase (ubiquinone) flavoprotein 3, 10kDa); PABPC1 (Poly(A) binding protein, cytoplasmic 1); PHLDA1 (Pleckstrin homology-like domain, family A, member 1); PIAS2 (Msx-interacting-zinc finger, transcript variant alpha); RAB11FIP3 (RAB11 family interacting protein 3 (class II)); RAN (RAN, member RAS oncogene family); RARA (Retinoic acid receptor alpha); RBMS1 (RNA binding motif, single stranded interacting protein 1, transcript variant); RDBP (RD RNA binding protein); RNF12 (Ring finger protein 12, transcript variant 1); RUFY1 (RUN and FYVE domain containing 1); SMN1 (Survival of motor neuron 1, telomeric); SRPK1 (SFRS protein kinase 1); SSNA1 (Sjogren's syndrome nuclear autoantigen 1); STAU (Staufen, RNA binding protein (Drosophila), transcript variant T3); STK11 (Serine/threonine kinase 11 (Peutz-Jeghers syndrome)); TOM1 (Target of myb1 (chicken)); TXNL2 (Thioredoxin-like, clone MGC:12349); TXNRD1 (Thioredoxin reductase 1, transcript variant 5); and ZNF38 (Zinc finger protein 38), or
(b) the group consisting of auto-antigens of ZMAT2 (Zinc finger, matrin type 2); ASPSCR1 (alveolar soft part sarcoma chromosome region, candidate 1); CEBPG (CCAAT/enhancer binding protein (C/EBP), gamma); DDX55 (DEAD (Asp-Glu-Ala-Asp) box polypeptide 55); DOM3Z (dom-3 homolog Z (C. elegans)); E1B-AP5 (E1B-55kDa-associated protein 5); FUS (Fusion (involved in t(12;16) in malignant liposarcoma)); HMG20B (High-mobility group 20B); KRT8 (Keratin 8); LIN28 (Lin-28 homolog (C. elegans)); LNX (ligand of numb-protein X); MAP1S (MAP1S protein); PABPC1 (Poly(A) binding protein, cytoplasmic 1); PHLDA1 (Pleckstrin homology-like domain, family A, member 1); PIAS2 (Msx-interacting-zinc finger, transcript variant alpha); PRKCBP1 (protein kinase C binding protein 1); PRKRA (protein kinase, interferon-inducible double stranded RNA dependent activator); RARA (Retinoic acid receptor, alpha); RDBP (RD RNA binding protein); RPL30 (ribosomal protein L30); RPL31 (ribosomal protein L31); SNK (serum-inducible kinase); SRPK1 (SFRS protein kinase 1); STAU1 (Staufen, RNA binding protein (Drosophila), transcript variant T3); TXNL2 (Thioredoxin-like, clone MGC:12349); VCL (vinculin); and ZNF38 (Zinc finger protein 38), or
(c) the group consisting of the auto-antigens of Table 3;
wherein the auto-antigens are immobilised on a substrate,
*for example* wherein said substrate comprises a strip, a slide, beads, SPR or planar waveguide chip or a well of a microtitre plate.

13. A kit according to claim 12, wherein the panel (i) comprises 10 or more auto-antigens, or (ii) comprises 20 or more auto-antigens; and, optionally, further comprising an anti-human immunoglobulin antibody and means for detecting said anti-human immunoglobulin antibody (for example, wherein the means for detecting comprise a colorimetric or a fluorescent assay or a label free detection method selected from mass spectrometry, chromatography, SPR or planar waveguide technology)

14. The kit of claim 12 or claim 13, wherein the panel of auto-antigens is carried on said substrate in the form of an array; for example, wherein the auto-antigens constitute at least 5%, 10%, 15%, 20%, 25%, 40% 50%, 70%, 80%, 85%, 90% or 95% of the total number of proteins on the array; and, optionally, wherein the array (A) includes one or more other proteins or polypeptides, and means for automatically analysing the results of tests performed using said array, said analysing means including computer-readable instructions in which results from said one or more other proteins or polypeptides are blinded during the analysis, (B) further comprises one or more control proteins or polypeptides; (C) further comprises one or more control assays for anti-RNA or anti-DNA antibodies; (D) further comprises one or more control assays for metabolites; or (E) comprises one or more replicates of the auto-antigens or controls.

15. Use of an auto-antigen selected from:
(a) the group consisting of ZMAT2 (Zinc finger, matrin type 2); BPY2IP1 (MAPIS protein); CEBPG (CCAAT/enhancer binding protein (C/EBP), gamma); E1B-AP5 (E1B-55kDa-associated protein 5); FUS (Fusion (involved in t(12;16) in malignant liposarcoma)); HAGH (Hydroxyacylglutathione hydrolase); HMG20B (High-mobility group 20B); HOXB6 (Homeo box B6, transcript variant 2); KRT8 (Keratin 8); LIN28 (Lin-28 homolog (C. elegans)); NDUFV3 (NADH dehydrogenase (ubiquinone) flavoprotein 3, 10kDa); PABPC1 (Poly(A) binding protein, cytoplasmic 1); PHLDA1 (Pleckstrin homology-like domain, family A, member 1); PIAS2 (Msx-interacting-zinc finger, transcript variant alpha); RAB11FIP3 (RAB11 family interacting protein 3 (class II)); RAN (RAN, member RAS oncogene family); RARA (Retinoic acid receptor alpha); RBMS1 (RNA binding motif, single stranded interacting protein 1, transcript variant); RDBP (RD RNA binding protein); RNF12 (Ring finger protein 12, transcript variant 1); RUFY1 (RUN and FYVE domain containing 1); SMN1 (Survival of motor neuron 1, telomeric); SRPK1 (SFRS protein kinase 1); SSNA1 (Sjogren's syndrome nuclear autoantigen 1); STAU (Staufen, RNA binding protein (Drosophila), transcript variant T3); STK11 (Serine/threonine kinase 11 (Peutz-Jeghers syndrome)); TOM1 (Target of myb1 (chicken)); TXNL2 (Thioredoxin-like, clone MGC:12349); TXNRD1 (Thioredoxin reductase 1, transcript variant 5); and ZNF38 (Zinc finger protein 38);
(b) the group consisting of auto-antigens of ZMAT2 (Zinc finger, matrin type 2); ASPSCR1 (alveolar soft part sarcoma chromosome region, candidate 1); CEBPG (CCAAT/enhancer binding protein (C/EBP), gamma); DDX55 (DEAD (Asp-Glu-Ala-Asp) box polypeptide 55); DOM3Z (dom-3 homolog Z (C. elegans)); E1B-AP5 (E1B-55kDa-associated protein 5); FUS (Fusion (involved in t(12;16) in malignant liposarcoma)); HMG20B (High-mobility group 20B); KRT8 (Keratin 8); LIN28 (Lin-28 homolog (C. elegans)); LNX (ligand of numb-protein X); MAP1S (MAP1S protein); PABPC1 (Poly(A) binding protein, cytoplasmic 1); PHLDA1 (Pleckstrin homology-like domain, family A, member 1); PIAS2 (Msx-interacting-zinc finger, transcript variant alpha); PRKCBP1 (protein kinase C binding protein 1); PRKRA (protein kinase, interferon-inducible double stranded RNA dependent activator); RARA (Retinoic acid receptor, alpha); RDBP (RD RNA binding protein); RPL30 (ribosomal protein L30); RPL31 (ribosomal protein L31); SNK (serum-inducible kinase); SRPK1 (SFRS protein kinase 1); STAU1 (Staufen, RNA binding protein (Drosophila), transcript variant T3); TXNL2 (Thioredoxin-like, clone MGC:12349); VCL (vinculin); and ZNF38 (Zinc finger protein 38), or
(c) the group consisting of the auto-antigens of Table 3,
in the manufacture of a vaccine for the treatment or prevention of lupus; for example, wherein the vaccine comprises two or more of the antigens; and wherein, optionally, the vaccine additionally comprises an adjuvant.

16. A panel of auto-antigens, wherein said panel comprises or consists of:
(i)
(a) the auto-antigens listed in Table 2,
(b) the panel of (a) in which up to 5 auto-antigens are deleted or replaced, or
(c) the panel of (a) in which up to 6, 7, 8, 9 or 10 ore more auto-antigens are deleted or replaced;
(ii)
(a) the auto-antigens listed in Table 1,
(b) the panel of (a) in which up to 5 auto-antigens are deleted or replaced, or
(c) the panel of (a) in which up to 6, 7, 8, 9 or 10 or more auto-antigens are deleted or replaced;
(iii)
(a) the auto-antigens listed in Table 4,
(b) the panel of (a) in which up to 5 auto-antigens are deleted or replaced, or
(c) the panel of (a) in which up to 6, 7, 8, 9 or 10 or more auto-antigens are deleted or replaced; or
(iv)
(a) the auto-antigens listed in Table 3,
(b) the panel of (a) in which up to 5 auto-antigens are deleted or replaced, or
(c) the panel of (a) in which up to 10, 15, 20 or 25 or more auto-antigens are deleted or replaced;
*for example:*
according to (i)(b), (i)(c), (ii)(b), (ii)(c), (iii)(b), (iii)(c), (iv)(b) or (iv)(c) wherein the panel comprises ZMAT2,
*and optionally*
wherein the auto-antigens are variants.
